(19) Europäisches Patentamt
European Patent Office
Office européen des brevets

(11) EP 1 673 625 B1

(12) **EUROPEAN PATENT SPECIFICATION**

(45) Date of publication and mention of the grant of the patent:
**09.12.2009 Bulletin 2009/50**

(21) Application number: **04788755.9**

(22) Date of filing: **15.09.2004**

(51) Int Cl.:
***G01N 33/48*** (2006.01) ***G01N 33/543*** (2006.01)
***C12P 21/06*** (2006.01) ***C12Q 1/68*** (2006.01)
***C12N 15/90*** (2006.01) ***G06F 19/00*** (2006.01)

(86) International application number:
**PCT/US2004/030085**

(87) International publication number:
**WO 2005/040344 (06.05.2005 Gazette 2005/18)**

(54) **GENERATION OF STABILIZED PROTEINS BY COMBINATORIAL CONSENSUS MUTAGENESIS**

ERZEUGUNG STABILER PROTEINE DURCH KOMBINATORISCHE KONSENSUS-MUTAGENESE

GENERATION DE PROTEINES STABILISEES PAR MUTAGENESE DE CONSENSUS COMBINATOIRE

(84) Designated Contracting States:
**AT BE BG CH CY CZ DE DK EE ES FI FR GB GR HU IE IT LI LU MC NL PL PT RO SE SI SK TR**

(30) Priority: **16.10.2003 US 688255**

(43) Date of publication of application:
**28.06.2006 Bulletin 2006/26**

(73) Proprietor: **GENENCOR INTERNATIONAL, INC.**
**Palo Alto, California 94304 (US)**

(72) Inventors:
- **AEHLE, Wolfgang**
  **NL-2645 GB Delfgauw (NL)**
- **RAMER, Sandra, W.**
  **Sunnyvale, CA 94089 (US)**
- **SCHELLENBERGER, Volker**
  **Palo Alto, CA 94303 (US)**

(74) Representative: **Kiddle, Simon John et al**
**Mewburn Ellis LLP**
**33 Gutter Lane**
**London**
**EC2V 8AS (GB)**

(56) References cited:
**US-B1- 6 582 914 US-B1- 6 713 279**
**US-B2- 6 706 503**

- **AMIN N ET AL: "Construction of stabilized proteins by combinatorial consensus mutagenesis" PROTEIN ENGINEERING, DESIGN AND SELECTION, OXFORD JOURNAL, LONDON, GB, vol. 17, no. 11, November 2004 (2004-11), pages 787-793, XP002404110 ISSN: 1741-0126**
- **OSTERMEIER M: "Synthetic gene libraries: in search of the optimal diversity" TRENDS IN BIOTECHNOLOGY, ELSEVIER PUBLICATIONS, CAMBRIDGE, GB, vol. 21, no. 6, June 2003 (2003-06), pages 244-247, XP004428425 ISSN: 0167-7799**
- **SANDGREN M ET AL: "COMPARISON OF FAMILY 12 GLYCOSIDE HYDROLASES AND RECRUITED SUBSTITUTIONS IMPORTANT FOR THERMAL STABILITY" RCSB PDB, 1 April 2003 (2003-04-01), XP002432449**
- **LEHMANN M. ET AL: 'From DNA to Improved Functionality: Using Protein Sequence Comparisons to Rapidly Design a Thermostable Consensus Phytase' PROTEIN ENGINEERING vol. 13, no. 1, January 2000, pages 49 - 57, XP002186780**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 673 625 B1

SEQUENCE LISTING

<110> Genencor International, Inc.

<120> Generation of Stabilized Proteins by Combinatorial Consensus Mutagenesis

<130> SJK/FP6368443

<140> EP 04788755.9
<141> 2003-10-16

<150> PCT/US2004/030085
<151> 2004-09-15

<150> US 10/688,255
<151> 2003-10-16

<160> 86

<170> PatentIn version 3.2

<210> 1
<211> 5069

<212> DNA
<213> Artificial Sequence

<220>
<223> plasmid pCB04

<400> 1

```
gagtcgtatt acaattcact ggccgtcgtt ttacaacgtc gtgactggga aaaccctggc     60
gttacccaac ttaatcgcct tgcagcacat ccccctttcg ccagctggcg taatagcgaa    120
gaggcccgca ccgatcgccc ttcccaacag ttgcgcagcc tgaatggcga atggacgcgc    180
cctgtagcgg cgcattaagc gcggcgggtg tggtggttac gcgcagcgtg accgctacac    240
ttgccagcgc cctagcgccc gctcctttcg ctttcttccc ttcctttctc gccacgttcg    300
ccggctttcc ccgtcaagct ctaaatcggg ggctcccttt agggttccga tttagtgctt    360
tacggcacct cgaccccaaa aaacttgatt agggtgatgg ttcacgtagt gggccatcgc    420
cctgatagac ggtttttcgc cctttgacgt tggagtccac gttctttaat agtggactct    480
tgttccaaac tggaacaaca ctcaacccta tctcggtcta ttcttttgat ttataaggga    540
ttttgccgat ttcggcctat tggttaaaaa atgagctgat ttaacaaaaa tttaacgcga    600
attttaacaa aatattaacg cttacaattt cctgatgcgg tattttctcc ttacgcatct    660
gtgcggtatt tcacaccgca tatggtgcac tctcagtaca atctgctctg atgccgcata    720
gttaagccag ccccgacacc cgccaacacc cgctgacgcg ccctgacggg cttgtctgct    780
cccggcatcc gcttacagac aagctgtgac cgtctccggg agctgcatgt gtcagaggtt    840
ttcaccgtca tcaccgaaac gcgcgagacg aaagggcctc gtgatacgcc tatttttata    900
ggttaatgtc atgataataa tggtttctta gacgtcaggt ggcacttttc ggggaaatgt    960
gcgcggaacc cctatttgtt tatttttcta aatacattca aatatgtatc cgctcatgag   1020
acaataaccc tgtggcagca tcacccgacg cactttgcgc cgaataaata cctgtgacgg   1080
aagatcactt cgcagaataa ataaatcctg gtgtccctgt tgataccggg aagccctggg   1140
ccaacttttg gcgaaaatga gacgttgatc ggcacgtaag aggttccaac tttcaccata   1200
atgaaataag atcactaccg ggcgtatttt ttgagttatc gagattttca ggagctaagg   1260
aagctaaaat ggagaaaaaa atcactggat ataccaccgt tgatatatcc caatggcatc   1320
gtaaagaaca ttttgaggca tttcagtcag ttgctcaatg tacctataac cagaccgttc   1380
agctggatat tacggccttt ttaaagaccg taaagaaaaa taagcacaag ttttatccgg   1440
cctttattca cattcttgcc cgcctgatga atgctcatcc ggaattccgt atggcaatga   1500
aagacggtga gctggtgata tgggatagtg ttcacccttg ttacaccgtt ttccatgagc   1560
aaactgaaac gttttcatcg ctctggagtg aataccacga cgatttccgg cagtttctac   1620
acatatattc gcaagatgtg gcgtgttacg gtgaaaacct ggcctatttc cctaaagggt   1680
ttattgagaa tatgtttttc gtctcagcca atccctgggt gagtttcacc agttttgatt   1740
taaacgtggc caatatggac aacttcttcg cccccgtttt caccatgggc aaatattata   1800
```

```
cgcaaggcga caaggtgctg atgccgctgg cgattcaggt tcatcatgcc gtctgtgatg   1860
gcttccatgt cggcagaatg cttaatgaat tacaacagta ctgcgatgag tggcagggcg   1920
gggcgtaaag acagatcgct gagataggtg cctcactgat taagcattgg taactgtcag   1980
accaagttta ctcatatata ctttagattg atttaaaact tcatttttaa tttaaaagga   2040
tctaggtgaa gatccttttt gataatctca tgaccaaaat cccttaacgt gagttttcgt   2100
tccactgagc gtcagacccc gtagaaaaga tcaaaggatc ttcttgagat cctttttttc   2160
tgcgcgtaat ctgctgcttg caaacaaaaa aaccaccgct accagcggtg gtttgtttgc   2220
cggatcaaga gctaccaact ctttttccga aggtaactgg cttcagcaga gcgcagatac   2280
caaatactgt tcttctagtg tagccgtagt taggccacca cttcaagaac tctgtagcac   2340
cgcctacata cctcgctctg ctaatcctgt taccagtggc tgctgccagt ggcgataagt   2400
cgtgtcttac cgggttggac tcaagacgat agttaccgga taaggcgcag cggtcgggct   2460
gaacgggggg ttcgtgcaca cagcccagct tggagcgaac gacctacacc gaactgagat   2520
acctacagcg tgagctatga gaaagcgcca cgcttcccga agggagaaag gcggacaggt   2580
atccggtaag cggcagggtc ggaacaggag agcgcacgag ggagcttcca gggggaaacg   2640
cctggtatct ttatagtcct gtcgggtttc gccacctctg acttgagcgt cgatttttgt   2700
gatgctcgtc aggggggcgg agcctatgga aaaacgccag caacgcggcc tttttacggt   2760
tcctggcctt ttgctggcct tttgctcaca tgttctttcc tgcgttatcc cctgattctg   2820
tggataaccg tattaccgcc tttgagtgag ctgataccgc tcgccgcagc cgaacgaccg   2880
agcgcagcga gtcagtgagc gaggaagcgg aagagcgccc aatacgcaaa ccgcctctcc   2940
ccgcgcgttg gccgattcat taatgcagct ggcacgacag gtttcccgac tggaaagcgg   3000
gcagtgagcg caacgcaatt aatgtgagtt agctcactca ttaggcaccc caggctttac   3060
actttatgct tccggctcgt atgttgtgtg gaattgtgag cggataacaa tttcacacag   3120
gaaacagcta tgaccatgat tacgccaagc tatttaggtg acactataga atactcaagc   3180
ttaagaagga gatatacata tgaaaaagtt actattcgca attccactag tcgttccttt   3240
ctattctcac tctacgccag tgtcagaaaa acagctggcg gaggtggtcg cgaatacgat   3300
taccccgctg atgaaagcac agagtgttcc aggcatggcg gtggccgtta tttatcaggg   3360
aaaaccgcac tattacacat ttggcaaggc cgatatcgcg gcgaataaac ccgttacgcc   3420
tcagaccctg ttcgagctgg gttctataag taaaaccttc accggcgttt taggtgggga   3480
tgccattgct cgcggtgaaa tttcgctgga cgatgcggtg accagatact ggccacagct   3540
gacgggcaag cagtggcagg gtattcgtat gctggatctc gccacctaca ccgctggcgg   3600
cctgccgcta caggtaccgg atgaggtcac ggataacgcc tccctgctgc gcttttatca   3660
aaactggcag ccgcagtgga agcctggcac aacgcgtctt tacgccaacg ccagcatcgg   3720
tctttttggt gcgctggcgg tcaaaccttc tggcatgccc tatgagcagg ccatgacgac   3780
gcgggtcctt aagccgctca agctggacca tacctggatt aacgtgccga aagcggaaga   3840
ggcgcattac gcctggggct atcgtgacgg taaagcggtg cgcgtttcgc cgggtatgct   3900
ggatgcacaa gcctatggcg tgaaaaccaa cgtgcaggat atggcgaact gggtcatggc   3960
caacatggcc ccggagaacg ttgctgatgc ctcacttaag cagggcatcg cgctggcgca   4020
gtcgcgctac tggcgtatcg ggtcaatgta tcagggtctc ggctgggaga tgctcaactg   4080
gcccgtggag gcaaacacgg tggtcgaggg cagcgacagt aaggtagcgc tagcgccgtt   4140
gcccgtggca gaagtgaatc caccggctcc cccggtcaaa gcgtcctggg tccataaaac   4200
tggctctact ggcgggtttg gatcctacgt ggcctttatt cctgaaaagc agatcggtat   4260
tgtgatgctc gcgaatacaa gctatccgaa cccggctcga gttgaggcgg cataccatat   4320
cctagaggcg ctacagggtg gcggatcggc cgaaactgtt gaacaccacc atcatcacca   4380
ttagcccggg gctgctcact acactctagg tggaggttca ccattcgttt gtgaatatca   4440
aggccaatcg tctgacctgc ctcaacctcc tgtcaatgct ggcggcggct ctggtggtgg   4500
ttctggtggc ggctctgagg gtggtggctc tgagggtggc ggttctgagg gtggcggctc   4560
tgagggaggc ggttccggtg gtggctctgg ttccggtgat tttgattatg aaaagatggc   4620
aaacgctaat aagggggcta tgaccgaaaa tgccgatgaa aacgcgctac agtctgacgc   4680
taaaggcaaa cttgattctg tcgctactga ttacggtgct gctatcgatg gtttcattgg   4740
tgacgtttcc ggccttgcta atggtaatgg tgctactggt gattttgctg gctctaattc   4800
ccaaatggct caagtcggtg acggtgataa ttcaccttta atgaataatt tccgtcaata   4860
tttaccttcc ctccctcaat cggttgaatg tcgccctttt gtctttagcg ctggtaaacc   4920
atatgaattt tctattgatt gtgacaaaat aaacttattc cgtggtgtct ttgcgtttct   4980
tttatatgtt gccaccttta tgtatgtatt ttctacgttt gctaacatac tgcgtaataa   5040
ggagtcttaa taagaattcg ccctatagt                                     5069
```

<210> 2
<211> 120

<212> PRT
<213> Artificial Sequence

<220>
<223> CAB1 heavy chain

<400> 2

```
Gln Val Lys Leu Gln Gln Ser Gly Ala Glu Leu Val Arg Ser Gly Thr
1               5                   10                  15
Ser Val Lys Leu Ser Cys Thr Ala Ser Gly Phe Asn Ile Lys Asp Ser
            20                  25                  30
Tyr Met His Trp Leu Arg Gln Gly Pro Glu Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Thr Val Thr Val Ser Ser
        115                 120
```

<210> 3
<211> 15
<212> PRT
<213> Artificial Sequence

<220>
<223> CAB1 linker

<400> 3

```
Gly Gly Gly Gly Ser Gly Gly Gly Gly Ser Gly Gly Gly Gly Ser
1               5                   10                  15
```

<210> 4
<211> 110
<212> PRT
<213> Artificial Sequence

<220>
<223> CAB1 light chain

<400> 4

```
Glu Asn Val Leu Thr Gln Ser Pro Ala Ile Met Ser Ala Ser Pro Gly
1               5                   10                  15
Glu Lys Val Thr Ile Thr Cys Ser Ala Ser Ser Ser Val Ser Tyr Met
            20                  25                  30
His Trp Phe Gln Gln Lys Pro Gly Thr Ser Pro Lys Leu Trp Ile Tyr
        35                  40                  45
Ser Thr Ser Asn Leu Ala Ser Gly Val Pro Ala Arg Phe Ser Gly Ser
    50                  55                  60
Gly Ser Gly Thr Ser Tyr Ser Leu Thr Ile Ser Arg Met Glu Ala Glu
65                  70                  75                  80
Asp Ala Ala Thr Tyr Tyr Cys Gln Gln Arg Ser Ser Tyr Pro Leu Thr
                85                  90                  95
Phe Gly Ala Gly Thr Lys Leu Glu Leu Lys Arg Ala Ala Thr



            100                 105                 110
```

<210> 5
<211> 360
<212> PRT
<213> Artificial Sequence

<220>
<223> BLA

<400> 5

```
Leu Val Ser Glu Lys Gln Leu Ala Glu Val Val Ala Asn Thr Ile Thr
1               5                   10                  15
Pro Leu Met Lys Ala Gln Ser Val Pro Gly Met Ala Val Ala Val Ile
            20                  25                  30
Tyr Gln Gly Lys Pro His Tyr Tyr Thr Phe Gly Lys Ala Asp Ile Ala
        35                  40                  45
Ala Asn Lys Pro Val Thr Pro Gln Thr Leu Phe Glu Leu Gly Ser Ile
    50                  55                  60
Ser Lys Thr Phe Thr Gly Val Leu Gly Gly Asp Ala Ile Ala Arg Gly
65                  70                  75                  80
Glu Ile Ser Leu Asp Asp Ala Val Thr Arg Tyr Trp Pro Gln Leu Thr
                85                  90                  95
Gly Lys Gln Trp Gln Gly Ile Arg Met Leu Asp Leu Ala Thr Tyr Thr
            100                 105                 110
Ala Gly Gly Leu Pro Leu Gln Val Pro Asp Glu Val Thr Asp Asn Ala
        115                 120                 125
Ser Leu Leu Arg Phe Tyr Gln Asn Trp Gln Pro Gln Trp Lys Pro Gly
    130                 135                 140
Thr Thr Arg Leu Tyr Ala Asn Ala Ser Ile Gly Leu Phe Gly Ala Leu
145                 150                 155                 160
Ala Val Lys Pro Ser Gly Met Pro Tyr Glu Gln Ala Met Thr Thr Arg
                165                 170                 175
Val Leu Lys Pro Leu Lys Leu Asp His Thr Trp Ile Asn Val Pro Lys
            180                 185                 190
Ala Glu Glu Ala His Tyr Ala Trp Gly Tyr Arg Asp Gly Lys Ala Val
        195                 200                 205
Arg Val Ser Pro Gly Met Leu Asp Ala Gln Ala Tyr Gly Val Lys Thr
    210                 215                 220
Asn Val Gln Asp Met Ala Asn Trp Val Met Ala Asn Met Ala Pro Glu
225                 230                 235                 240
Asn Val Ala Asp Ala Ser Leu Lys Gln Gly Ile Ala Leu Ala Gln Ser
                245                 250                 255
Arg Tyr Trp Arg Ile Gly Ser Met Tyr Gln Gly Leu Gly Trp Glu Met
            260                 265                 270
Leu Asn Trp Pro Val Glu Ala Asn Thr Val Val Glu Thr Ser Phe Gly
        275                 280                 285
Asn Val Ala Leu Ala Pro Leu Pro Val Ala Glu Val Asn Pro Pro Ala
    290                 295                 300
Pro Pro Val Lys Ala Ser Trp Val His Lys Thr Gly Ser Thr Gly Gly
305                 310                 315                 320
Phe Gly Ser Tyr Val Ala Phe Ile Pro Glu Lys Gln Ile Gly Ile Val
                325                 330                 335
Met Leu Ala Asn Thr Ser Tyr Pro Asn Pro Ala Arg Val Glu Ala Ala
            340                 345                 350
Tyr His Ile Leu Glu Ala Leu Gln
        355                 360
```

<210> 6
<211> 5178
<212> DNA
<213> Artificial Sequence

<220>
<223> pME27.1 plasmid

<400> 6

```
aggaattatc atatgaaata cctgctgccg accgctgctg ctggtctgct gctcctcgct     60
gcccagccgg ccatggccca ggtgaaactg cagcagtctg gggcagaact tgtgaggtca    120
gggacctcag tcaagttgtc ctgcacagct tctggcttca acattaaaga ctcctatatg    180
cactggttga ggcaggggcc tgaacagggc ctggagtgga ttggatggat tgatcctgag    240
aatggtgata ctgaatatgc cccgaagttc cagggcaagg ccactttac tacagacaca     300
tcctccaaca cagcctacct gcagctcagc agcctgacat ctgaggacac tgccgtctat    360
tattgtaatg aggggactcc gactgggccg tactactttg actactgggg ccaagggacc    420
acggtcaccg tctcctcagg tggaggcggt tcaggcggag gtggctctgg cggtggcgga    480
tcagaaaatg tgctcaccca gtctccagca atcatgtctg catctccagg ggagaaggtc    540
accataacct gcagtgccag ctcaagtgta agttacatgc actggttcca gcagaagcca    600
ggcacttctc ccaaactctg gatttatagc acatccaacc tggcttctgg agtccctgct    660
cgcttcagtg gcagtggatc tgggacctct tactctctca caatcagccg aatggaggct    720
gaagatgctg ccacttatta ctgccagcaa agatctagtt acccactcac gttcggtgct    780
ggcaccaagc tggagctgaa acgggcggcc acaccggtgt cagaaaaaca gctggcggag    840
gtggtcgcga atacgattac cccgctgatg aaagcccagt ctgttccagg catggcggtg    900
gccgttattt atcagggaaa accgcactat tacacatttg gcaaggccga tatcgcggcg    960
aataaacccg ttacgcctca gaccctgttc gagctgggtt ctataagtaa aaccttcacc   1020
ggcgttttag gtggggatgc cattgctcgc ggtgaaattt cgctggacga tgcggtgacc   1080
agatactggc cacagctgac gggcaagcag tggcagggta ttcgtatgct ggatctcgcc   1140
acctacaccg ctggcggcct gccgctacag gtaccggatg aggtcacgga taacgcctcc   1200
ctgctgcgct tttatcaaaa ctggcagccg cagtggaagc ctggcacaac gcgtctttac   1260
gccaacgcca gcatcggtct tttggtgcg ctggcggtca aaccttctgg catgccctat    1320
gagcaggcca tgacgacgcg ggtccttaag ccgctcaagc tggaccatac ctggattaac   1380
gtgccgaaag cggaagaggc gcattacgcc tggggctatc gtgacggtaa agcggtgcgc   1440
gtttcgccgg gtatgctgga tgcacaagcc tatggcgtga aaaccaacgt gcaggatatg   1500
gcgaactggg tcatggcaaa catggcgccg gagaacgttg ctgatgcctc acttaagcag   1560
ggcatcgcgc tggcgcagtc gcgctactgg cgtatcgggt caatgtatca gggtctgggc   1620
tgggagatgc tcaactggcc cgtggaggcc aacacggtgg tcgagacgag ttttggtaat   1680
gtagcactgg cgccgttgcc cgtggcagaa gtgaatccac cggctccccc ggtcaaagcg   1740
tcctgggtcc ataaaacggg ctctactggc gggtttggca gctacgtggc ctttattcct   1800
gaaaagcaga tcggtattgt gatgctcgcg aatacaagct atccgaaccc ggcacgcgtt   1860
gaggcggcat accatatcct cgaggcgcta cagtaggaat tcgagctccg tcgacaagct   1920
tgcggccgca ctcgagatca aacgggctag ccagccagaa ctcgccccgg aagaccccga   1980
ggatgtcgag caccaccacc accaccactg agatccggct gctaacaaag cccgaaagga   2040
agctgagttg gctgctgcca ccgctgagca ataactagca taaccccttg gggcctctaa   2100
acgggtcttg aggggttttt tgctgaaagg aggaactata tccggattgg cgaatgggac   2160
gcgccctgta gcggcgcatt aagcgcggcg ggtgtggtgg ttacgcgcag cgtgaccgct   2220
acacttgcca gcgccctagc gcccgctcct ttcgctttct tcccttcctt tctcgccacg   2280
ttcgccggct ttccccgtca agctctaaat cgggggctcc ctttagggtt ccgatttagt   2340
gctttacggc acctcgaccc caaaaaactt gattagggtg atggttcacg tagtgggcca   2400
tcgccctgat agacggtttt tcgccctttg acgttggagt ccacgttctt taatagtgga   2460
ctcttgttcc aaactggaac aacactcaac cctatctcgg tctattcttt tgatttataa   2520
gggattttgc cgatttcggc ctattggtta aaaaatgagc tgatttaaca aaaatttaac   2580
gcgaatttta acaaaatatt aacgcttaca atttcctgat gcggtatttt ctccttacgc   2640
atctgtgcgg tatttcacac cgcatatggt gcactctcag tacaatctgc tctgatgccg   2700
catagttaag ccagccccga cacccgccaa cacccgctga cgcgccctga cgggcttgtc   2760
tgctcccggc atccgcttac agacaagctg tgaccgtctc cgggagctgc atgtgtcaga   2820
ggttttcacc gtcatcaccg aaacgcgcga gacgaaaggg cctcgtgata cgcctatttt   2880
tataggttaa tgtcatgata ataatggttt cttagacgtc aggtggcact tttcggggaa   2940
atgtgcgcgg aacccctatt tgtttatttt tctaaataca ttcaaatatg tatccgctca   3000
tgagacaata accctgtggc agcatcaccc gacgcacttt gcgccgaata aatacctgtg   3060
acggaagatc acttcgcaga ataaataaat cctggtgtcc ctgttgatac cgggaagccc   3120
```

```
tgggccaact tttggcgaaa atgagacgtt gatcggcacg taagaggttc caactttcac   3180
cataatgaaa taagatcact accgggcgta tttttgagt tatcgagatt ttcaggagct   3240
aaggaagcta aaatggagaa aaaatcact ggatatacca ccgttgatat atcccaatgg   3300
catcgtaaag aacattttga ggcatttcag tcagttgctc aatgtaccta taaccagacc   3360
gttcagctgg atattacggc ctttttaaag accgtaaaga aaaataagca caagttttat   3420
ccggccttta ttcacattct tgcccgcctg atgaatgctc atccggaatt ccgtatggca   3480
atgaaagacg gtgagctggt gatatgggat agtgttcacc cttgttacac cgttttccat   3540
gagcaaactg aaacgttttc atcgctctgg agtgaatacc acgacgattt ccggcagttt   3600
ctacacatat attcgcaaga tgtggcgtgt tacggtgaaa acctggccta tttccctaaa   3660
gggtttattg agaatatgtt tttcgtctca gccaatccct gggtgagttt caccagtttt   3720
gatttaaacg tggccaatat ggacaacttc ttcgcccccg ttttcacgat gggcaaatat   3780
tatacgcaag gcgacaaggt gctgatgccg ctggcgattc aggttcatca tgccgtctgt   3840
gatggcttcc atgtcggcag aatgcttaat gaattacaac agtactgcga tgagtggcag   3900
ggcggggcgt aaagacagat cgctgagata ggtgcctcac tgattaagca ttggtaactg   3960
tcagaccaag tttactcata tatactttag attgatttaa aacttcattt ttaatttaaa   4020
aggatctagg tgaagatcct ttttgataat ctcatgacca aaatccctta acgtgagttt   4080
tcgttccact gagcgtcaga ccccgtagaa aagatcaaag gatcttcttg agatcctttt   4140
tttctgcgcg taatctgctg cttgcaaaca aaaaaaccac cgctaccagc ggtggtttgt   4200
ttgccggatc aagagctacc aactcttttt ccgaaggtaa ctggcttcag cagagcgcag   4260
ataccaaata ctgttcttct agtgtagccg tagttaggcc accacttcaa gaactctgta   4320
gcaccgccta catacctcgc tctgctaatc ctgttaccag tggctgctgc cagtggcgat   4380
aagtcgtgtc ttaccgggtt ggactcaaga cgatagttac cggataaggc gcagcggtcg   4440
ggctgaacgg ggggttcgtg cacacagccc agcttggagc gaacgaccta caccgaactg   4500
agatacctac agcgtgagct atgagaaagc gccacgcttc ccgaagggag aaaggcggac   4560
aggtatccgg taagcggcag ggtcggaaca ggagagcgca cgagggagct tccaggggga   4620
aacgcctggt atctttatag tcctgtcggg tttcgccacc tctgacttga gcgtcgattt   4680
ttgtgatgct cgtcaggggg gcggagccta tggaaaaacg ccagcaacgc ggccttttta   4740
cggttcctgg ccttttgctg gccttttgct cacatgttct ttcctgcgtt atcccctgat   4800
tctgtggata accgtattac cgcctttgag tgagctgata ccgctcgccg cagccgaacg   4860
accgagcgca gcgagtcagt gagcgaggaa gcggaagagc gcccaatacg caaaccgcct   4920
ctccccgcgc gttggccgat tcattaatgc agctggcacg acaggtttcc cgactggaaa   4980
gcgggcagtg agcgcaacgc aattaatgtg agttagctca ctcattaggc accccaggct   5040
ttacacttta tgcttccggc tcgtatgttg tgtggaattg tgagcggata acaatttcac   5100
acaggaaaca gctatgacca tgattacgcc aagctattta ggtgacacta tagaatactc   5160
aagctttcta gattaagg                                                   5178
```

<210> 7
<211> 80
<212> PRT
<213> Artificial Sequence

<220>
<223> consensus mutation

<400> 7

```
Gln Val Lys Leu Gln Gln Ser Gly Ala Glu Leu Val Arg Ser Gly Thr
1               5                   10                  15
Ser Val Lys Leu Ser Cys Thr Ala Ser Gly Phe Asn Ile Lys Asp Ser
            20                  25                  30
Tyr Met His Trp Leu Arg Gln Gly Pro Glu Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ser Asn Thr Ala Tyr
65                  70                  75                  80
```

<210> 8
<211> 80
<212> PRT
<213> Artificial Sequence

<220>
<223> consensus mutation

<400> 8

```
Leu Gln Leu Ser Ser Leu Thr Ser Glu Asp Thr Ala Val Tyr Tyr Cys
1               5                   10                  15
Asn Glu Gly Thr Pro Thr Gly Pro Tyr Tyr Phe Asp Tyr Trp Gly Gln
            20                  25                  30
Gly Thr Thr Val Thr Val Ser Ser Gly Gly Gly Gly Ser Gly Gly Gly
        35                  40                  45
Gly Ser Gly Gly Gly Gly Ser Glu Asn Val Leu Thr Gln Ser Pro Ala
    50                  55                  60
Ile Met Ser Ala Ser Pro Gly Glu Lys Val Thr Ile Thr Cys Ser Ala
65                  70                  75                  80
```

<210> 9
<211> 84
<212> PRT
<213> Artificial Sequence

<220>
<223> consensus mutation

<400> 9

```
Ser Ser Ser Val Ser Tyr Met His Trp Phe Gln Gln Lys Pro Gly Thr
1               5                   10                  15
Ser Pro Lys Leu Trp Ile Tyr Ser Thr Ser Asn Leu Ala Ser Gly Val
            20                  25                  30
Pro Ala Arg Phe Ser Gly Ser Gly Ser Gly Thr Ser Tyr Ser Leu Thr
        35                  40                  45
Ile Ser Arg Met Glu Ala Glu Asp Ala Ala Thr Tyr Tyr Cys Gln Gln
    50                  55                  60
Arg Ser Ser Tyr Pro Leu Thr Phe Gly Ala Gly Thr Lys Leu Glu Leu
65                  70                  75                  80
Lys Arg Ala Ala
```

<210> 10
<211> 36
<212> DNA
<213> Artificial Sequence

<220>
<223> primer

<400> 10
cgcgtcttta cgccaactcc agcatcggtc tttttg 36

<210> 11

<211> 35
<212> DNA
<213> Artificial Sequence

<220>
<223> primer

<400> 11
ggattaacgt gccgaaatcg gaagaggcgc attac 35

<210> 12
<211> 35
<212> DNA
<213> Artificial Sequence

<220>
<223> primer

<400> 12
gctatcgtga cggtaaaccg gtgcgcgttt cgccg 35

<210> 13
<211> 36
<212> DNA
<213> Artificial Sequence

<220>
<223> primer

<400> 13
gctggcggag gtggtcgaca atacgattac cccgct 36

<210> 14
<211> 36
<212> DNA
<213> Artificial Sequence

<220>
<223> primer

<400> 14
accgcactat tacacatatg gcaaggccga tatcgc 36

<210> 15
<211> 35
<212> DNA
<213> Artificial Sequence

<220>
<223> primer

<400> 15
agtcgcgcta ctggcgtgtc gggtcaatgt atcag 35

<210> 16
<211> 40
<212> DNA
<213> Artificial Sequence

<220>
<223> primer

<400> 16
ctttattcct gaaaagcagc tcggtattgt gatgctcgcg 40

<210> 17
<211> 37
<212> DNA
<213> Artificial Sequence

<220>
<223> primer

<400> 17
ctgttcgagc tgggttctgt aagtaaaacc ttcaccg 37

<210> 18
<211> 35
<212> DNA
<213> Artificial Sequence

<220>
<223> primer

<400> 18
agtggcaggg tattcgtctg ctggatctcg ccacc 35

<210> 19
<211> 35
<212> DNA
<213> Artificial Sequence

<220>
<223> primer

<400> 19
ctatggcgtg aaaaccaccg tgcaggatat ggcga 35

<210> 20

<400> 20
000

<210> 21

<400> 21
000

<210> 22
<211> 36
<212> DNA
<213> Artificial Sequence

<220>
<223> primer

<400> 22

acgtgcagga tatggcgcgc tgggtcatgg ccaaca 36

<210> 23
<211> 36
<212> DNA
<213> Artificial Sequence

<220>
<223> primer

<400> 23
gtaaggtagc gctagcggcg ttgcccgtgg cagaag 36

<210> 24
<211> 35
<212> DNA
<213> Artificial Sequence

<220>
<223> primer

<400> 24
tgaccagata ctggccagag ctgacgggca agcag 35

<210> 25
<211> 37
<212> DNA
<213> Artificial Sequence

<220>
<223> primer

<400> 25
cgggtatgct ggatgcagaa gcctatggcg tgaaaac 37

<210> 26
<211> 35
<212> DNA
<213> Artificial Sequence

<220>
<223> primer

<400> 26
ggacgatgcg gtgaccaaat actggccaca gctga 35

<210> 27
<211> 36
<212> DNA
<213> Artificial Sequence

<220>
<223> primer

<400> 27
agcagtggca gggtattact atgctggatc tcgcca 36

<210> 28

<211> 36
<212> DNA
<213> Artificial Sequence

<220>
<223> primer

<400> 28
aggtcacgga taacgccgcc ctgctgcgct tttatc 36

<210> 29
<211> 35
<212> DNA
<213> Artificial Sequence

<220>
<223> primer

<400> 29
tctcgccacg ccagtgacag aaaaacagct ggcgg 35

<210> 30
<211> 37
<212> DNA
<213> Artificial Sequence

<220>
<223> primer

<400> 30
gagaacgttg ctgatgccac acttaagcag ggcatcg 37

<210> 31
<211> 36
<212> DNA
<213> Artificial Sequence

<220>
<223> primer

<400> 31
cttgctctgc tctcgccgcg ccagtgtcag aaaaac 36

<210> 32
<211> 37
<212> DNA
<213> Artificial Sequence

<220>
<223> primer

<400> 32
caagcctatg gcgtgaaatc caacgtgcag gatatgg 37

<210> 33
<211> 35
<212> DNA
<213> Artificial Sequence

<220>
<223> primer

<400> 33
tgtgatgctc gcgaataaaa gctatccgaa cccgg 35

<210> 34
<211> 35
<212> DNA
<213> Artificial Sequence

<220>
<223> primer

<400> 34
tggcgtgaaa accaacgcgc aggatatggc gaact 35

<210> 35
<211> 35
<212> DNA
<213> Artificial Sequence

<220>
<223> primer

<400> 35
ccgtggaggc aaacacgctg gtcgagggca gcgac 35

<210> 36
<211> 35
<212> DNA
<213> Artificial Sequence

<220>
<223> primer

<400> 36
tggaggcaaa cacggtgatc gagggcagcg acagt 35

<210> 37
<211> 39
<212> DNA
<213> Artificial Sequence

<220>
<223> primer

<400> 37
gaaaaacagc tggcggagat cgtcgcgaat acgattacc 39

<210> 38
<211> 35
<212> DNA
<213> Artificial Sequence

<220>
<223> primer

<400> 38
tgatgaaagc acagagtatt ccaggcatgg cggtg 35

<210> 39
<211> 35
<212> DNA
<213> Artificial Sequence

<220>
<223> primer

<400> 39
accttctggc atgccctttg agcaggccat gacga 35

<210> 40
<211> 35
<212> DNA
<213> Artificial Sequence

<220>
<223> primer

<400> 40
gggaaaaccg cactatttca catttggcaa ggccg 35

<210> 41
<211> 36
<212> DNA
<213> Artificial Sequence

<220>
<223> primer

<400> 41
cttgctctgc tctcgccgcg ccagtgtcag aaaaac 36

<210> 42
<211> 17
<212> DNA
<213> Artificial Sequence

<220>
<223> primer

<400> 42
caggaaacag ctatgac 17

<210> 43
<211> 20
<212> DNA
<213> Artificial Sequence

<220>
<223> primer

<400> 43
gccgctcaag ctggaccata 20

<210> 44
<211> 39
<212> DNA
<213> Artificial Sequence

<220>
<223> primer

<400> 44
gaaaaacagc tggcggagat cgtcgcgaat acgattacc 39

<210> 45
<211> 35
<212> DNA
<213> Artificial Sequence

<220>
<223> primer

<400> 45
tgatgaaagc acagagtatt ccaggcatgg cggtg 35

<210> 46
<211> 35
<212> DNA
<213> Artificial Sequence

<220>
<223> primer

<400> 46
ggacgatgcg gtgaccaaat actggccaca gctga 35

<210> 47
<211> 36
<212> DNA
<213> Artificial Sequence

<220>
<223> primer

<400> 47
acgtgcagga tatggcgcgc tgggtcatgg ccaaca 36

<210> 48
<211> 37
<212> DNA
<213> Artificial Sequence

<220>
<223> primer

<400> 48
gagaacgttg ctgatgccac acttaagcag ggcatcg 37

<210> 49
<211> 35
<212> DNA

<213> Artificial Sequence

<220>
<223> primer

<400> 49
agtcgcgcta ctggcgtgtc gggtcaatgt atcag 35

<210> 50
<211> 35
<212> DNA
<213> Artificial Sequence

<220>
<223> primer

<400> 50
ccgtggaggc aaacacgctg gtcgagggca gcgac 35

<210> 51
<211> 35
<212> DNA
<213> Artificial Sequence

<220>
<223> primer

<400> 51
tggaggcaaa cacggtgatc gagggcagcg acagt 35

<210> 52
<211> 35
<212> DNA
<213> Artificial Sequence

<220>
<223> primer

<400> 52
tgtgatgctc gcgaataaaa gctatccgaa cccgg 35

<210> 53
<211> 41
<212> DNA
<213> Artificial Sequence

<220>
<223> primer

<400> 53
ccgtggaggc aaacacgctg atcgagggca gcgacagtaa g 41

<210> 54
<211> 37
<212> DNA
<213> Artificial Sequence

<220>

<223> primer

<400> 54
cggccatggc ccaggtgcag ctgcagcagt ctggggc 37

<210> 55
<211> 37
<212> DNA
<213> Artificial Sequence

<220>
<223> primer

<400> 55
ctggggcaga acttgtgaaa tcagggacct cagtcaa 37

<210> 56
<211> 37
<212> DNA
<213> Artificial Sequence

<220>
<223> primer

<400> 56
gggcagaact tgtgaggccg gggacctcag tcaagtt 37

<210> 57
<211> 37
<212> DNA
<213> Artificial Sequence

<220>
<223> primer

<400> 57
aacttgtgag gtcagggggc tcagtcaagt tgtcctg 37

<210> 58
<211> 37
<212> DNA
<213> Artificial Sequence

<220>
<223> primer

<400> 58
gcacagcttc tggcttcacc attaaagact cctatat 37

<210> 59
<211> 37
<212> DNA
<213> Artificial Sequence

<220>
<223> primer

<400> 59

cagcttctgg cttcaacttt aaagactcct atatgca 37

<210> 60
<211> 37
<212> DNA
<213> Artificial Sequence

<220>
<223> primer

<400> 60
cttctggctt caacattagc gactcctata tgcactg 37

<210> 61
<211> 37
<212> DNA
<213> Artificial Sequence

<220>
<223> primer

<400> 61
actcctatat gcactgggtg aggcaggggc ctgaaca 37

<210> 62
<211> 37
<212> DNA
<213> Artificial Sequence

<220>
<223> primer

<400> 62
tgcactggtt gaggcaggcg cctgaacagg gcctgga 37

<210> 63
<211> 37
<212> DNA
<213> Artificial Sequence

<220>
<223> primer

<400> 63
ggttgaggca ggggcctggc cagggcctgg agtggat 37

<210> 64
<211> 37
<212> DNA
<213> Artificial Sequence

<220>
<223> primer

<400> 64
ccccgaagtt ccagggccgt gccactttta ctacaga 37

<210> 65

<211> 37
<212> DNA
<213> Artificial Sequence

<220>
<223> primer

<400> 65
cgaagttcca gggcaagttc acttttacta cagacac 37

<210> 66
<211> 37
<212> DNA
<213> Artificial Sequence

<220>
<223> primer

<400> 66
tccagggcaa ggccactatt actacagaca catcctc 37

<210> 67
<211> 37
<212> DNA
<213> Artificial Sequence

<220>
<223> primer

<400> 67
gcaaggccac ttttactcgc gacacatcct ccaacac 37

<210> 68
<211> 37
<212> DNA
<213> Artificial Sequence

<220>
<223> primer

<400> 68
ttactacaga cacatccaaa aacacagcct acctgca 37

<210> 69
<211> 37
<212> DNA
<213> Artificial Sequence

<220>
<223> primer

<400> 69
ctgccgtcta ttattgtgcg gaggggactc cgactgg 37

<210> 70
<211> 37
<212> DNA
<213> Artificial Sequence

<220>
<223> primer

<400> 70
ccgtctatta ttgtaatcgc gggactccga ctgggcc 37

<210> 71
<211> 37
<212> DNA
<213> Artificial Sequence

<220>
<223> primer

<400> 71
ctggcggtgg cggatcacag aatgtgctca cccagtc 37

<210> 72
<211> 37
<212> DNA
<213> Artificial Sequence

<220>
<223> primer

<400> 72
gcggtggcgg atcagaaagc gtgctcaccc agtctcc 37

<210> 73
<211> 38
<212> DNA
<213> Artificial Sequence

<220>
<223> primer

<400> 73
gaaaatgtgc tcacccagcc gccagcaatc atgtctgc 38

<210> 74
<211> 37
<212> DNA
<213> Artificial Sequence

<220>
<223> primer

<400> 74
tgctcaccca gtctccaagc atcatgtctg catctcc 37

<210> 75
<211> 37
<212> DNA
<213> Artificial Sequence

<220>
<223> primer

<400> 75
cccagtctcc agcaatcgtg tctgcatctc cagggga 37

<210> 76
<211> 37
<212> DNA
<213> Artificial Sequence

<220>
<223> primer

<400> 76
tgtctgcatc tccagggcag aaggtcacca taacctg 37

<210> 77
<211> 37
<212> DNA
<213> Artificial Sequence

<220>
<223> primer

<400> 77
ctgcatctcc aggggagacc gtcaccataa cctgcag 37

<210> 78
<211> 37
<212> DNA
<213> Artificial Sequence

<220>
<223> primer

<400> 78
taagttacat gcactggtac cagcagaagc caggcac 37

<210> 79
<211> 37
<212> DNA
<213> Artificial Sequence

<220>
<223> primer

<400> 79
gcacttctcc caaactcgtg atttatagca catccaa 37

<210> 80
<211> 37
<212> DNA
<213> Artificial Sequence

<220>
<223> primer

<400> 80
tggcttctgg agtccctgat cgcttcagtg gcagtgg 37

<210> 81
<211> 37
<212> DNA
<213> Artificial Sequence

<220>
<223> primer

<400> 81
ctcgcttcag tggcagtaaa tctgggacct cttactc 37

<210> 82
<211> 37
<212> DNA
<213> Artificial Sequence

<220>
<223> primer

<400> 82
gtggatctgg gacctctgcg tctctcacaa tcagccg 37

<210> 83
<211> 37
<212> DNA
<213> Artificial Sequence

<220>
<223> primer

<400> 83
ctctcacaat cagccgactg gaggctgaag atgctgc 37

<210> 84
<211> 37
<212> DNA
<213> Artificial Sequence

<220>
<223> primer

<400> 84
gaatggaggc tgaagatgaa gccacttatt actgcca 37

<210> 85
<211> 37
<212> DNA
<213> Artificial Sequence

<220>
<223> primer

<400> 85
aggctgaaga tgctgccgat tattactgcc agcaaag 37

<210> 86
<211> 37
<212> DNA

<213> Artificial Sequence

<220>
<223> primer

<400> 86
acccactcac gttcggtggc ggcaccaagc tggagct 37

## Description

### FIELD OF THE INVENTION

The present invention provides methods and compositions for the production of stabilized proteins. In particular, the present invention provides methods and compositions for the generation of combinatorial libraries of consensus mutations and screening for improved protein variants.

### BACKGROUND OF THE INVENTION

Developing libraries of nucleic acids that comprise various combinations of several or many mutant or derivative sequences is recognized as a powerful method of discovering novel products having improved or more desirable characteristics. A number of powerful methods for mutagenesis have been developed that when used iteratively with focused screening to enrich the useful mutants is known by the general term "directed evolution."

For example, a variety of *in vitro* DNA recombination methods have been developed for the purpose of recombining more or less homologous nucleic acid sequences to obtain novel nucleic acids. For example, recombination methods have been developed comprising mixing a plurality of homologous, but different, nucleic acids, fragmenting the nucleic acids and recombining them using PCR to form chimeric molecules. For example, U.S. Patent No. 5,605,793 describes methods that generally comprise fragmentation of double stranded DNA molecules by DNase I, while U.S. Patent No. 5,965,408 provides methods that generally rely on the annealing of relatively short random primers to target genes and extending them with DNA polymerase. Each of these disclosures relies on polymerase chain reaction (PCR)-like thermocycling of fragments in the presence of DNA polymerase to recombine the fragments. Additional methods known in the art take advantage of the phenomenon known as template switching (*See e.g.*, Meyerhans, and Wain-Hobson, Nucleic Acids Res., 18: 1687-1891 [1990]). One shortcoming of these PCR-based recombination methods however is that the recombination points tend to be limited to those areas of relatively significant homology. Accordingly, in recombining more diverse nucleic acids, the frequency of recombination is dramatically reduced and limited.

In many contexts, it is desirable to be able to develop libraries of mutant molecules that mix and match mutations which are known to be important or interesting due to functional or structural data. Several strategies toward combinatorial mutagenesis have been developed, including "gene shuffling" methods in combination with a mixture of specifically designed oligonucleotide primers to incorporate desired mutations into the shuffling scheme (*See*, Stemmer et al., Biotechn., 18:194-196 [1995]). In other methods *(See,* Osuna et al., Gene, 106:7-12 [1991]), synthetic DNA fragments comprising 50% wild type codon and 50% of an equimolar mixture of codons for each of the 20 amino acids at positions 144, 145 and 200 of *Eco*RI endonuclease were produced. The mutagenic primers were added to a solution of ssDNA template and the primers for the 144 and 145 mutations used separately from the primers for the 200 site. The separate mixtures from each experiment were hybridized to the template ssDNA and extended for one hour with PolIk polymerase. The fragments were isolated and ligated to produce a full length fragment with mutations at all three sites. The fragment was amplified with PCR and purified and cloned into a vector. While it was predicted that a balanced distribution of each of the 20 mutants would be obtained at each position, the authors were unable to verify whether the predicted distribution was attained. Lehmann et al., 2002, Protein Engineering, 15, pp. 403-411 describes consensus mutation for thermostability engineering of proteins.

In another method (*See*, Tu et al., Biotechn., 20:352-353 [1996]) generation of combination of mutations is accomplished by using multiple mutagenic oligonucleotides which are incorporated into a mutagenic nucleotide by a single round of primer extension followed by ligation. In yet another method (*See*, Merino et al., Biotechn., 12:508-509 [1992]) single or combinatorial directed mutagenesis utilizes a universal set of primers complementary to the areas that flank the cloning region of the pUC/M13 vectors used in the mutagenesis scheme for the purpose of optimizing yield of mutants. In a further method *(See,* PCT Publication No. WO 98/42728) several variations on the theme of recombination of related families of nucleic acids are provided. In particular, this publication describes the use of defined primers in combination with recombination based generation of diversity, the defined primers being used to encourage cross-over recombination at sites not otherwise likely to be cross-over points. Recently, methods have been described that allow the construction of libraries based on gene synthesis where the location and level of diversity in the target gene can be widely controlled (*See e.g.*, Ostermeier, Trends Biotechnol., 21, 244-7 [2003]).

While it is apparent that a number of methods exist to construct libraries, it is desirable to develop more efficient methods to design libraries which contain an increased number of variants with improved traits. Indeed, what is needed are methods that provides means to rapidly and efficiently design proteins with desired improvements (*e.g.*, increased stability).

## SUMMARY OF THE INVENTION

The present invention provides methods and compositions for the production of stabilized proteins as set out in the claims. In particular, the present invention provides methods and compositions for the generation of combinatorial libraries of consensus mutations and screening for improved protein variants.

Accordingly, the present invention provides methods for combinatorial consensus mutagenesis comprising the steps: a) identifying a starting gene of interest; b) identifying at least two homologs of the starting gene of interest; c) generating a multiple sequence alignment of the at least two homologs of the starting gene of interest, and the starting gene of interest; d) using the multiple sequence alignment to identify consensus mutations and produce a combinatorial consensus library containing multiple combinations of consensus mutations; e) screening the combinatorial consensus library to identify initial hits; f) sequencing said initial hits to provide sequenced initial hits; g) identifying improving mutations in the sequenced initial hits, wherein said improving mutations are more common among said initial hits as compared to said combinatorial consensus library; h) using the sequenced initial hits to generate an enhanced combinatorial consensus library; and i) screening the enhanced combinatorial consensus library to identify at least one improved hit, wherein said screening e) and i) comprises determining the stability of the initial hit in at least one assay selected from the group consisting of protease resistance assays, thermostability assays, denaturation assays, and functional assays.

In yet additional embodiments, the methods of the present invention further comprise the step of sequencing improved hits. In alternative embodiments, the improved hits are stabilized variants of the starting gene. In some particularly preferred embodiments, the improved hits comprise performance-enhancing mutations. In yet additional preferred embodiments, the methods comprise the further step of analyzing the correlation between sequence and stability of at least two initial hits. In other preferred embodiments, methods of the present invention further comprise the step of analyzing the correlation between sequence and stability of at least two sequenced improved hits.

In some embodiments, the multiple sequence alignment identifies amino acids that occur frequently in homologs but are not part of a consensus sequence. In yet additional embodiments, the steps of the methods are repeated at least once, as desired.

The present invention also provides sequence improved hits that are produced according to the methods of the present invention. In additional embodiments, the present invention provides combinatorial consensus mutagenesis libraries produced according to the methods of the present invention.

In some preferred embodiments, the present invention provides stabilized variants of beta-lactamase, wherein the stabilized variant comprises at least one amino acid change selected from the group consisting of V11I, V251I, R91K, Q95E, A153S, N232R, S247T, V293L, V294I, T342K, I262V, and V284I.

In some alternative preferred embodiments, the present invention provides stabilized variants of carcinoembryonic antigen binder, wherein the stabilized variant comprises at least one amino acid change selected from the group consisting of K3Q, L37V, E42G, E136Q, M146V, F170Y, A194D, and A234G.

In yet additional preferred embodiments, the present invention provides stabilized single chain fragment variable region (scFV), wherein the stabilized scFV variant comprises at least one amino acid change selected from the group consisting of K3Q, L37V, E42G, E136Q, M146V, F170Y, A194D, and A234G.

## DESCRIPTION OF THE FIGURES

Figure 1 provides a map of the plasmid pCB04.

Figure 2 provides the nucleotide sequence (SEQ ID NO:1) of plasmid pCB04.

Figure 3 provides a graph showing the enrichment of consensus mutations observed during screening of NA04 library.

Figure 4 provides a table showing the calculated parameters for some mutations.

Figure 5 provides a graph showing the relative remaining activity of BLA variants of NA04 in the presence of three proteases.

Figure 6 provides a graph showing the stability distribution of 90 variants from NA01, NA02 and NA03.

Figure 7 provides the amino acid sequence of CAB 1. The sequences of the heavy chain (SEQ ID NO:2), linker (SEQ ID NO:3), light chain (SEQ ID NO:4), and BLA (SEQ ID NO:5) are shown.

Figure 8 provides a map of plasmid pME27.1, encoding CAB1.

Figure 9 provides the nucleotide sequence of plasmid pME27.1 (SEQ ID NO:6).

Figure 10 provides the amino acid sequences of consensus mutations used in constructing library NA 05 (SEQ ID NOS:7-9).

Figure 11 provides a graph showing the binding assay results for variants from the library NA05.

Figure 12 provides a graph showing the binding of various isolates from NA06 to CEA.

Figure 13 provides a brief schematic of the steps of the present invention.

## DESCRIPTION OF THE INVENTION

The present invention provides methods and compositions for the production of stabilized proteins. In particular, the present invention provides methods and compositions for the generation of combinatorial libraries of consensus mutations and screening for improved protein variants.

### Definitions

Unless defined otherwise herein, all technical and scientific terms used herein have the same meaning as commonly understood by one of ordinary skill in the art to which this invention belongs (*See e.g.*, Singleton, et al., DICTIONARY OF MICROBIOLOGY AND MOLECULAR BIOLOGY, 2D ED., John Wiley and Sons, New York [1994]; and Hale & Marham, THE HARPER COLLINS DICTIONARY OF BIOLOGY, Harper Perennial, NY [1991], both of which provide one of skill with a general dictionary of many of the terms used herein). Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to carboxy orientation, respectively. The headings provided herein are not limitations of the various aspects or embodiments of the invention that can be had by reference to the specification as a whole. Accordingly, the terms defined immediately below are more fully defined by reference to the specification as a whole.

As used herein, the term, "combinatorial mutagenesis" refers to the methods of the present invention in which libraries of variants of a starting sequence are generated. In these libraries, the variants contain one or several mutations chosen from a predefined set of mutations. In addition, the methods provide means to introduce random mutations which were not members of the predefined set of mutations. In some embodiments, the methods include those set forth in US 6 582 914. In alternative embodiments, combinatorial mutagenesis methods encompass commercially available kits (*e.g.*, QuikChange Multisite, Stratagene, San Diego, CA).

As used herein, the term "library of mutants" refers to a population of cells which are identical in most of their genome but include different homologues of one or more genes. Such libraries can be used, for example, to identify genes or operons with improved traits.

As used herein, the term "starting gene" refers to a gene of interest that encodes a protein of interest that is to be improved and/or changed using the present invention.

As used herein, the term "multiple sequence alignment" ("MSA") refers to the sequences of multiple homologs of a starting gene that are aligned using an algorithm (*e.g.*, Clustal W).

As used herein, the terms "consensus sequence" and "canonical sequence" refer to an archetypical amino acid sequence against which all variants of a particular protein or sequence of interest are compared. The terms also refer to a sequence that sets forth the nucleotides that are most often present in a DNA sequence of interest. For each position of a gene, the consensus sequence gives the amino acid that is most abundant in that position in the MSA. For example, in the Pribnow box, the canonical sequence is $T_{89}$ $A_{89}$ $T_{50}$ $A_{65}$ and $T_{100}$, wherein the subscript indicates the percent occurrence of the most frequently found base.

As used herein, the term "consensus mutation" refers to a difference in the sequence of a starting gene and a consensus sequence. Consensus mutations are identified by comparing the sequences of the starting gene and the consensus sequence resulting from an MSA. In some embodiments, consensus mutations are introduced into the starting gene such that it becomes more similar to the consensus sequence. Consensus mutations also include amino acid changes that change an amino acid in a starting gene to an amino acid that is more frequently found in an MSA at that position relative to the frequency of that amino acid in the starting gene. Thus, the term consensus mutation comprises all single amino acid changes that replace an amino acid of the starting gene with an amino acid that is more abundant than the amino acid in the MSA.

As used herein, the term "initial hit" refers to a variant that was identified by screening a combinatorial consensus mutagenesis library. In preferred embodiments, initial hits have improved performance characteristics, as compared to the starting gene.

As used herein, the term "improved hit" refers to a variant that was identified by screening an enhanced combinatorial consensus mutagenesis library.

As used herein, the terms "improving mutation" and "performance-enhancing mutation" refer to a mutation that leads to improved performance when it is introduced into the starting gene. In some preferred embodiments, these mutations are identified by sequencing hits that were identified during the screening step of the method. In most embodiments, mutations that are more frequently found in hits are likely to be improving mutations, as compared to an unscreened combinatorial consensus mutagenesis library.

As used herein, the term "enhanced combinatorial consensus mutagenesis library" refers to a CCM library that is designed and constructed based on screening and/or sequencing results from an earlier round of CCM mutagenesis and screening. In some embodiments, the enhanced CCM library is based on the sequence of an initial hit resulting from an earlier round of CCM. In additional embodiments, the enhanced CCM is designed such that mutations that were frequently observed in initial hits from earlier rounds of mutagenesis and screening are favored. In some preferred embodiments, this is accomplished by omitting primers that encode performance-reducing mutations or by increasing the concentration of primers that encode performance-enhancing mutations relative to other primers that were used in earlier CCM libraries.

As used herein, the term "performance-reducing mutations" refer to mutations in the combinatorial consensus mutagenesis library that are less frequently found in hits resulting from screening as compared to an unscreened combinatorial consensus mutagenesis library. In preferred embodiments, the screening process removes and/or reduces the abundance of variants that contain "performance-reducing mutations."

As used herein, the term "functional assay" refers to an assay that provides an indication of a protein's activity. In particularly preferred embodiments, the term refers to assay systems in which a protein is analyzed for its ability to function in its usual capacity. For example, in the case of enzymes, a functional assay involves determining the effectiveness of the enzyme in catalyzing a reaction.

As used herein, the term "target property" refers to the property of the starting gene that is to be altered. It is not intended that the present invention be limited to any particular target property. However, in some preferred embodiments, the target property is the stability of a gene product (*e.g.*, resistance to denaturation, proteolysis or other degradative factors), while in other embodiments, the level of production in a production host is altered. Indeed, it is contemplated that any property of a starting gene will find use in the present invention.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (*e.g.*, promoter strength, promoter recognition, promoter regulation, enhancer function), a property affecting RNA processing (*e.g.*, RNA splicing, RNA stability, RNA conformation, and post-transcriptional modification), a property affecting translation (*e.g.*, level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide, as used herein, refer to any characteristic or attribute of a polypeptide that can be selected or detected. These properties include, but are not limited to oxidative stability, substrate specificity, catalytic activity, thermal stability, alkaline stability, pH activity profile, resistance to proteolytic degradation, Km, kcat, Kcat/km ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, ability to treat disease.

As used herein, the term "screening" has its usual meaning in the art and is, in general a multi-step process. In the first step, a mutant nucleic acid or variant polypeptide therefrom is provided. In the second step, a property of the mutant nucleic acid or variant polypeptide is determined. In the third step, the determined property is compared to a property of the corresponding precursor nucleic acid, to the property of the corresponding naturally occurring polypeptide or to the property of the starting material (*e.g.*, the initial sequence) for the generation of the mutant nucleic acid.

It will be apparent to the skilled artisan that the screening procedure for obtaining a nucleic acid or protein with an altered property depends upon the property of the starting material the modification of which the generation of the mutant nucleic acid is intended to facilitate. The skilled artisan will therefore appreciate that the invention is not limited to any specific property to be screened for and that the following description of properties lists illustrative examples only. Methods for screening for any particular property are generally described in the art. For example, one can measure binding, pH, specificity, etc., before and after mutation, wherein a change indicates an alteration. Preferably, the screens are performed in a high-throughput manner, including multiple samples being screened simultaneously, including, but not limited to assays utilizing chips, phage display, and multiple substrates and/or indicators.

As used herein, in some embodiments, screens encompass selection steps in which variants of interest are enriched from a population of variants. Examples of these embodiments include the selection of variants that confer a growth advantage to the host organism, as well as phage display or any other method of display, where variants can be captured from a population of variants based on their binding or catalytic properties. In a preferred embodiment, a library of variants is exposed to stress (heat, protease, denaturation) and subsequently variants that are still intact are identified in a screen or enriched by selection. It is intended that the term encompass any suitable means for selection. Indeed, it is not intended that the present invention be limited to any particular method of screening.

In one embodiment of the invention, the template nucleic acid encodes all or a portion of an antibody. The term "antibody" or grammatical equivalents, as used herein, refer to antibodies and antibody fragments that retain the ability to bind to the epitope that the intact antibody binds and include polyclonal antibodies, monoclonal antibodies, chimeric antibodies, anti-idiotype (anti-ID) antibodies. Preferably, the antibodies are monoclonal antibodies. Antibody fragments include, but are not limited to the complementarity-determining regions (CDRs), single-chain fragment variable regions (scFv), heavy chain variable region (VH), light chain variable region (VL).

As used herein, "host cell" refers to a cell that has the capacity to act as a host and expression vehicle for an incoming sequence. In one embodiment, the host cell is a microorganism.

As used herein, the terms "DNA construct" and "transforming DNA" are used interchangeably to refer to DNA used to introduce sequences into a host cell or organism. The DNA may be generated *in vitro* by PCR or any other suitable technique(s) known to those in the art. In particularly preferred embodiments, the DNA construct comprises a sequence of interest (*e.g.*, as an incoming sequence). In some embodiments, the sequence is operably linked to additional elements such as control elements (*e.g.*, promoters, etc.). The DNA construct may further comprise a selectable marker. It may further comprise an incoming sequence flanked by homology boxes. In a further embodiment, the transforming DNA comprises other non-homologous sequences, added to the ends (*e.g.*, stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell, and/or 2) mutagenize a region of the host cell chromosome (*i.e.*, replace an endogenous sequence with a heterologous sequence), 3) delete target genes; and/or introduce a replicating plasmid into the host.

As used herein, the term "targeted randomization" refers to a process that produces a plurality of sequences where one or several positions have been randomized. In some embodiments, randomization is complete (*i.e.*, all four nucleotides, A, T, G, and C can occur at a randomized position. In alternative embodiments, randomization of a nucleotide is limited to a subset of the four nucleotides. Targeted randomization can be applied to one or several codons of a sequence, coding for one or several proteins of interest. When expressed, the resulting libraries produce protein populations in which one or more amino acid positions can contain a mixture of all 20 amino acids or a subset of amino acids, as determined by the randomization scheme of the randomized codon. In some embodiments, the individual members of a population resulting from targeted randomization differ in the number of amino acids, due to targeted or random insertion or deletion of codons. In further embodiments, synthetic amino acids are included in the protein populations produced. In some preferred embodiments, the majority of members of a population resulting from targeted randomization show greater sequence homology to the consensus sequence than the starting gene.

In some preferred embodiments, mutant DNA sequences are generated with site saturation mutagenesis in at least one codon. In other preferred embodiments, site saturation mutagenesis is performed for two or more codons. In a further embodiment, mutant DNA sequences have more than 40%, more than 45%, more than 50%, more than 55%, more than 60%, more than 65%, more than 70%, more than 75%, more than 80%, more than 85%, more than 90%, more than 95%, or more than 98% homology with the sequence of the starting gene. Alternatively, mutant DNA may be generated *in vivo* using any known mutagenic procedure (*e.g.*, radiation, nitrosoguanidine, etc.). The DNA construct sequences may be wild-type, mutant or modified. In addition, the sequences may be homologous or heterologous.

The terms "modified sequence" and "modified genes" are used interchangeably herein to refer to a sequence that includes a deletion, insertion or interruption of naturally occurring nucleic acid sequence. In some preferred embodiments, the expression product of the modified sequence is a truncated protein (*e.g.*, if the modification is a deletion or interruption of the sequence). In some particularly preferred embodiments, the truncated protein retains biological activity. In alternative embodiments, the expression product of the modified sequence is an elongated protein (*e.g.*, modifications comprising an insertion into the nucleic acid sequence). In some embodiments, an insertion leads to a truncated protein (*e.g.*, when the insertion results in the formation of a stop codon). Thus, an insertion may result in either a truncated protein or an elongated protein as an expression product.

As used herein, the terms "mutant sequence" and "mutant gene" are used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity (*e.g.*, enhanced enzymatic activity).

The terms "mutagenic primer" or "mutagenic oligonucleotide" (used interchangeably herein) are intended to refer to oligonucleotide compositions which correspond to a portion of the template sequence and which are capable of hybridizing thereto. With respect to mutagenic primers, the primer will not precisely match the template nucleic acid, the mismatch or mismatches in the primer being used to introduce the desired mutation into the nucleic acid library. As used herein, "non-mutagenic primer" or "non-mutagenic oligonucleotide" refers to oligonucleotide compositions which will match precisely to the template nucleic acid. In one embodiment of the invention, only mutagenic primers are used. In another preferred embodiment of the invention, the primers are designed so that for at least one region at which a mutagenic primer has been included, there is also non-mutagenic primer included in the oligonucleotide mixture. By adding a mixture of mutagenic primers and non-mutagenic primers corresponding to at least one of the mutagenic primers, it is possible to produce a resulting nucleic acid library in which a variety of combinatorial mutational patterns are presented. For example, if it is desired that some of the members of the mutant nucleic acid library retain their precursor sequence at certain positions while other members are mutant at such sites, the non-mutagenic primers provide the ability to obtain a specific level of non-mutant members within the nucleic acid library for a given residue. The methods of the invention employ mutagenic and non-mutagenic oligonucleotides which are generally between 10-50 bases in length, more preferably about 15-45 bases in length. However, it may be necessary to use primers that are either shorter than 10 bases or longer than 50 bases to obtain the mutagenesis result desired. With respect to corresponding mutagenic and non-mutagenic primers, it is not necessary that the corresponding oligonucleotides be of identical length, but only that there is overlap in the region corresponding to the mutation to be added.

Primers may be added in a pre-defined ratio according to the present invention. For example, if it is desired that the resulting library have a significant level of a certain specific mutation and a lesser amount of a different mutation at the same or different site, by adjusting the amount of primer added, it is possible to produce the desired biased library. Alternatively, by adding lesser or greater amounts of non-mutagenic primers, it is possible to adjust the frequency with which the corresponding mutation(s) are produced in the mutant nucleic acid library.

"Contiguous mutations" means mutations which are presented within the same oligonucleotide primer. For example, contiguous mutations may be adjacent or nearby each other, however, they will be introduced into the resulting mutant template nucleic acids by the same primer.

"Discontiguous mutations" means mutations which are presented in separate oligonucleotide primers. For example, discontiguous mutations will be introduced into the resulting mutant template nucleic acids by separately prepared oligonucleotide primers.

An "incoming sequence" as used herein means a DNA sequence that is newly introduced into the host cell. In some embodiments, the incoming sequence becomes integrated into the host chromosome or genome. The sequence may encode one or more proteins of interest. Thus, as used herein, the term "sequence of interest" refers to an incoming sequence or a sequence to be generated by the host cell. The terms "gene of interest" and "sequence of interest" are used interchangeably herein.

The incoming sequence may comprise a promoter operably linked to a sequence of interest. An incoming sequence comprises a sequence that may or may not already present in the genome of the cell to be transformed (*i.e.*, homologous and heterologous sequences find use with the present invention).

In one embodiment, the incoming sequence encodes at least one heterologous protein, including, but not limited to hormones, enzymes, and growth factors. In an alternative embodiment, the incoming sequence encodes a functional wild-type gene or operon, a functional mutant gene or operon, or a non-functional gene or operon. In some embodiments, the non-functional sequence is inserted into a target sequence to disrupt function, thereby allowing a determination of function of the disrupted gene.

The terms "wild-type sequence," or "wild-type gene" are used interchangeably herein, to refer to a sequence that is native or naturally occurring in a host cell. In some embodiments, the wild-type sequence refers to a sequence of interest that is the starting point of a protein engineering project. The wild-type sequence may encode either a homologous or heterologous protein. A homologous protein is one the host cell would produce without intervention. A heterologous protein is one that the host cell would not produce but for the intervention.

As used herein, the term "heterologous sequence" refers to a sequence derived from a separate genetic source or species. Heterologous sequences encompass non-host sequences, modified sequences, sequences from a different host cell strain, and homologous sequences from a different chromosomal location of the host cell. In some embodiments, homology boxes flank each side of an incoming sequence

As used herein, the term "selectable marker" refers to genes that provide an indication that a host cell has taken up an incoming DNA of interest or some other reaction has occurred. Typically, selectable markers are genes that confer antibiotic resistance or a metabolic advantage on the host cell to allow cells containing the exogenous DNA to be distinguished from cells that have not received any exogenous sequence during the transformation. A "residing selectable marker" is one that is located on the chromosome of the microorganism to be transformed. A residing selectable marker encodes a gene that is different from the selectable marker on the transforming DNA construct.

## DETAILED DESCRIPTION OF THE INVENTION

The present invention provides methods and compositions for the production of stabilized proteins. In particular, the present invention provides methods and compositions for the generation of combinatorial libraries of consensus mutations and screening for improved protein variants.

Protein sequences of organisms have evolved as a result of random mutagenesis and selection. During this process of evolution, many mutations that de-stabilize or otherwise reduce performance of a protein are removed and performance-enhancing mutations are retained. However, evolution also leads to the accumulation of random mutations that may be performance-reducing but have little impact on the fitness of their host organism. Multiple sequence alignments of homologous proteins allow to identify which amino acid is frequently found in a particular position of a protein. These consensus residues are likely to result in functional mutants if they are introduced into a particular sequence of a family of related proteins and it has been demonstrated that such consensus mutations can lead to variants with improved function (*See e.g.*, Steipe et al., J. Mol. Biol., 240: 188-92 [1994]). Thus, it is possible to improve the performance of a protein by systematically introducing individual consensus mutations into a protein. However, this process is very time consuming, as the number of possible consensus mutations can be large and it may be necessary to incorporate several consensus mutations to achieve the desired performance enhancement. An alternative method involves the direct synthesis of a protein's consensus sequence (Lehmann et al., Protein Eng., 13:49-57 [2000]). Indeed, this approach was used to identify a stabilized phytase variant. However, the authors noted in subsequent studies that not all consensus mutations were stabilizing. Thus, it was necessary to remove a number of consensus mutations, which again is a slow and iterative process (Lehmann et al., Protein Eng., 15:403-11 [2002]).

During the development of the present invention, the assumption was made that consensus mutations can be divided into "improving mutations" and "performance-reducing mutations." Thus, methods were developed that allow for the rapid generation of variants of a starting protein that contain a number of improving mutations and few if any performance-reducing mutations. As part of the process, combinatorial consensus mutagenesis (CCM) libraries are created that contain multiple combinations of consensus mutations. In some particularly preferred embodiments, these CCM libraries are screened to identify "initial hits" which contain one or several improving mutations and few if any performance-reducing mutations. In some cases, the resulting initial hits are sufficiently improved for their intended application. However, the present invention further provides methods that allow further improvement of these initial hits. By sequencing several initial hits from a CCM library, improving mutations which are more common among the hits as compared to the initial CCM library are identified. This information facilitates the construction of a second (*i.e.*, "enhanced") CCM library that is enriched in improving mutations. In some embodiments, the enhanced CCM library is constructed based on the starting gene. In alternative embodiments, the enhanced CCM library is started from one or several of the initial hits which already contain some improving mutations, and add further improving mutations (that were found in other initial hits) to them in the enhanced CCM library. If further enhancement is desired, further rounds of CCM library construction based on already improved hits and/or based on additional sequence information resulting from improved and initial hits are performed. This combinatorial process allows one to rapidly identify variants of the starting gene that contain multiple improving consensus mutations but few if any performance-reducing mutations. An overview of the CCM process is outlined in Figure 13.

In particularly preferred embodiments, it is important to note that the effect of mutations on the performance of a protein is not necessarily additive. Thus, mutations that enhance the performance of the starting gene may not necessarily have the same effect in a variant of that gene. One advantage of the CCM process of the present invention is that it explores many combinations of consensus mutations. Thus, the present invention is very likely to identify combinations of such mutations that lead to large improvements in gene performance.

In preferred embodiments, the present invention provides means to identify homologs of a starting gene through use of database searching and/or homology cloning from a sample of interest (*e.g.*, an environmental sample). Once the homolog(s) are identified, MSA are generated and consensus mutations identified. Depending upon the number of differences between the starting sequence and the consensus sequence, the positions at which the MSA gives a clear consensus that differs from the starting gene can be chosen for further investigation. In alternative embodiments, positions are included in the MSA where many homologs differ from the starting sequence, even when there is no clear consensus in that position. In these alternative embodiments, it is possible to generate larger libraries containing more diverse variants.

Next, mutagenic oligonucleotides are designed that introduce the chosen consensus mutation into the starting gene. Then, combinatorial mutagenesis is performed to produce a library of variants. Once this step is completed, improved variants in the library are identified. It is not intended that the present invention be limited to any particular method of screening variants and identifying those with improved properties. Indeed, those of skill in the art know how to best choose a method, as it will depend upon the starting gene, expression host, and the target property to be improved.

In additional embodiments, the variants in the library are sequenced, in particular those that have been improved. In further embodiments, statistical analyses are conducted to estimate the contribution of each individual mutation to the performance of the individual variants. In yet further embodiments, a second combinatorial library is generated, based on the results of the statistical analyses.

## EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm

(revolutions per minute); $H_2O$ (water); $dH_2O$ (deionized water); HCl (hydrochloric acid); aa (amino acid); bp (base pair); kb (kilobase pair); kD (kilodaltons); gm (grams); μg and ug (micrograms); mg (milligrams); ng (nanograms); μl (microliters); ml (milliliters); mm (millimeters); nm (nanometers); μm and um (micrometer); M (molar); mM (millimolar); μM and uM (micromolar); U (units); V (volts); MW (molecular weight); sec (seconds); min(s) (minute/minutes); hr(s) (hour/hours); $MgCl_2$ (magnesium chloride); NaCl (sodium chloride); SOC (2% Bacto-Tryptone, 0.5% Bacto Yeast Extract, 10 mM NaCl, 2.5 mM KCl); Terrific Broth (TB; 12 g/l Bacto Tryptone, 24 g/l glycerol, 2.31 g/l $KH_2PO_4$, and 12.54 g/l $K_2HPO_4$); $OD_{280}$ (optical density at 280 nm); $OD_{600}$ (optical density at 600 nm); C (constant region or chain); V (variable chain); vH and $V_H$(variable heavy chain); vL and $V_L$ (variable light chain); PAGE (polyacrylamide gel electrophoresis); PBS (phosphate buffered saline [150 mM NaCl, 10 mM sodium phosphate buffer, pH 7.2]); PBST (PBS+0.25% Tween® 20); PEG (polyethylene glycol); PCR (polymerase chain reaction); RT-PCR (reverse transcription PCR); SDS (sodium dodecyl sulfate); Tris (tris(hydroxymethyl)aminomethane); w/v (weight to volume); v/v (volume to volume); CEA (carcinoembryonic antigen); CAB (CEA antigen binder); LA medium (per liter: Difco Tryptone Peptone 20g, Difco Yeast Extract 10g, EM Science NaCl 1g, EM Science Agar 17.5g, dH20 to 1L); NCBI (National Center for Biotechnology Information); ATCC (American Type Culture Collection, Rockville, MD); Applied Biosystems (Applied Biosystems, Foster City, CA); Clontech (CLONTECH Laboratories, Palo Alto, CA); Difco (Difco Laboratories, Detroit, MI); Oxoid (Oxoid Inc., Ogdensburg, NY); GIBCO BRL or Gibco BRL (Life Technologies, Inc., Gaithersburg, MD); Millipore (Millipore, Billerica, MA); Bio-Rad (Bio-Rad, Hercules, CA); Invitrogen (Invitrogen Corp., San Diego, CA); NEB (New England Biolabs, Beverly, MA); Sigma (Sigma Chemical Co., St. Louis, MO); Pierce (Pierce Biotechnology, Rockford, IL); Takara (Takara Bio Inc. Otsu, Japan); Roche (Hoffmann-La Roche, Basel, Switzerland); EM Science (EM Science, Gibbstown, NJ); Qiagen (Qiagen, Inc., Valencia, CA); Biodesign (Biodesign Intl., Saco, Maine); Aptagen (Aptagen, Inc., Herndon, VA); Molecular Devices (Molecular Devices, Corp., Sunnyvale, CA); Stratagene (Stratagene Cloning Systems, La Jolla, CA); and Microsoft (Microsoft, Inc., Redmond, WA).

## EXAMPLE 1

### Combinatorial Consensus Mutagenesis of BLA

In this Example, the use of combinatorial consensus mutagenesis with beta-lactamase (BLA) is described. These experiments were performed using plasmid pCB04 which directs the expression of beta-lactamase (BLA) from *Enterobacter cloacae.* BLA expression is driven by a lac promoter. The protein is secreted into the periplasm of *E. coli,* as it contains a leader peptide from the pIII protein of bacteriophage M13. The BLA gene is fused to a gene coding for the D3 domain of the pIII protein of bacteriophage M13. However, there is a amber stop codon located between both genes and consequently, TOP10 cells (Invitrogen, ) carrying the plasmid express BLA and not a fusion protein. Expression of BLA from plasmid pCB04 confers resistance to the antibiotic cefotaxime to the cells. Figure 1 provides a map of plasmid pCB04, while Figure 2 provides the nucleotide sequence (SEQ ID NO:1) of plasmid pCB04. Plasmid pCB04 contains the following features:

| | |
|---|---|
| **P lac:** | 3008-3129 bp |
| **gIII signal:** | 3200-3253 |
| **BLA:** | 3254-4336 |
| **His Tag:** | 4364-4384 |
| **gIII d3:** | 4421-5053 |
| **F1 origin:** | 175-630 |
| **CAT:** | 3253-3912 |

### Choosing Mutations for Mutagenesis

Forty-three publicly available protein sequences for bacterial beta-lactamases of class C type were identified by a keyword search of protein sequences available at NCBI. Among the available sequences were three of particular note: NCBI accession number PNKBP corresponded to the *Enterobacter cloacae* enzyme that has been used as the backbone for protein engineering; NCBI accession number AMPC_PSYIM corresponded to a lactamase isolated from a psychrophilic organism; and NCBI accession number AAM23514 corresponded to a lactamase isolated from a thermophilic organism.

Table 1 provides the accession numbers and corresponding species for the 38 BLA sequences used in the multiple sequence alignment.

**Table 1. Sequences Used in Multiple Sequence Alignment**

| NCBI Accession # | Organism |
|---|---|
| AAL49969 | *Shewanella algae* |
| AAM23514 | *Thermoanaerobacter tengcongensis* |
| AAM90334 | *Klebsiella pneumoniae* |
| AF411145_1 | *Enterobacter cloacae* |
| AF462690_1 | *Aeromonas punctata* |
| AF492445_2 | *Citrobacter mutliniae* |
| AF492446_2 | *Enterobacter cancerogenus* |
| AF492447_2 | *Citrobacter braakii* |
| AF492448_2 | *Citrobacter werkmanii* |
| AF492449_1 | *Escherichia fergusonii* |
| AMPC_CITFR | *Citrobacter freundii* |
| AMPC_ECOLI | *Escherichia coli* K12 |
| AMPC_LYSLA | *Lysobacter lactamgenus* |
| AMPC_MORMO | *Morganella morganii* |
| AMPC_PROST | *Providencia stuartii* |
| AMPC_PSEAE | *Pseudomonas aeruginosa* |
| AMPC_PSYIM | *Psychrobacter immobilis* |
| AMPC_SERMA | *Serratia marcescens* |
| AMPC_YEREN | *Yersinia enterocolitica* |
| CAA54602 | *Klebsiella pneumoniae* |
| CAA56561 | *Aeromonas sobria* |
| CAA76196 | *Salmonella enteriditis* |
| CAB36900 | *Escherichia coli* |
| CAC04522 | *Ochrobactum anthropi* |
| CAC17149 | *Ochrobactum anthropi* |
| CAC17622 | *Ochrobactum anthropi* |
| CAC85157 | *Enterobacter asburiae* |
| CAC85357 | *Enterobacter hormaechei* |
| CAC85358 | *Enterobacter intermedius* |
| CAC85359 | *Enterobacter dissolvents* |
| CAC94553 | *Buttiauxella sp* BTN01 |
| CAC95129 | *Enterobacter cancerogenus* |
| CAD32298 | *Enterobacter amnigenus* |
| CAD32299 | *Enterobacter nimipressuralis* |
| CAD32304 | *Citrobacter youngae* |
| NP_313158 | *Escherichia coli* 0157:H7 |
| PNKBP | *Enterobacter cloacae* |

(continued)

| NCBI Accession # | Organism |
|---|---|
| S13408 | *Pseudomonas aeruginosa* |

The AlignX program within the Vector NTI version 7.0 software suite (Invitrogen) was used to align the 43 sequences identified. AlignX uses a clustalw algorithm; the alignment parameters used were the default parameters recommended and supplied with the program. The alignment was based on the *E. cloacae* sequence. Preliminary examination of this initial alignment revealed a duplicate sequence and a cluster of 4 sequences representing broad-spectrum inhibitor-resistant proteins which were excluded from the final protein alignment. The remaining 38 sequences were realigned, again basing the alignment on the *E. cloacae* sequence. In this alignment, the most-distantly related protein was the lactamase from the thermophilic bacterium. The AlignX program was allowed to define a consensus residue at each position where it was able to, using its default definition of a consensus residue. At each position where the alignment indicated a consensus residue, that residue was compared to the corresponding residue in the *E. cloacae* sequence. In this analysis, 29 residues were identified where the *cloacae* sequence differed from the consensus sequence. These 29 residues were chosen for the first round of mutagenesis.

Primers were designed to incorporate the desired amino acid changes into the *E. cloacae* backbone. General primer design was done following the recommendations of the manufacturer of the Quikchange® Multi-Site kit (Stratagene). Briefly, the constructed primers were 5' phosphorylated, ranged in length from 35 to 40 nucleotides, and had predicted melting temperatures of >75°C. In most cases, the change to the desired amino acid was accomplished by changing a single nucleotide in the primer, although in a few cases, two changes had to be introduced. The mismatching nucleotide or nucleotides was/were placed in the center of the primer, with generally 15-17 nucleotides on either side of the mismatch. Primers were named corresponding to the amino acid to be changed, its position, and the intended mutation. For example, primer "A214S" corresponds to alanine at position 214 to be changed to serine. The numbering starts with the initial methionine in the signal sequence of the wildtype *E. cloacae* protein. All primers were designed to the sense strand.

Three libraries were prepared using the QuikChange® Multi-Site Mutagenesis kit (QCMS) (Stratagene), with some modifications as described below. The first library, "NA01," was prepared using a final concentration of 4 uM for all primers combined (approximately 35 ng of each primer). The second library, "NA02" was prepared using a concentration of 0.4 uM for all primers combined (approximately 3.5 ng of each primer). The third library, "NA03," was prepared using a concentration of 0.4 uM for all primers combined (as with NA02), but the reaction was heated to 95°C for 2 minutes before transformation, in order to determine whether the wild-type background could be reduced. The QCMS protocol recommends the use of 50-100 ng and up to5 primers. Thus, the reaction components used as described in this Example are a bit different from the standard reaction compositions. It was noted that the experiment with 3.5 ng of each primer worked quite well, whereas the experiment with 35 ng of each primer resulted in fewer mutants.

The QCMS reactions contained 18.5 ul ddH2O, 1.0 ul undiluted (100 uM stock of total primers) or diluted primer mix (10 uM stock of total primers), 1.0 ul dNTPs (provided in kit), 1.0 ul template DNA (pCB04wt; 160 ng), 1.0 ul enzyme blend (provided in kit), and 2.5 ul buffer (provided in kit), for a total of 25 ul. The cycling conditions were 95°C for 1 minute, (once), followed by cycling (30x) at 95°C, 1 minute; 55°C for 1 minute, and 65°C for 10 minutes; the reactions were then held at 4°C. Then, the reactions were digested with *Dpn*I (1 ul) for 2 hours at 37°C, after which 0.5 ul *Dpn*I were added, and digestion continued for two more hours. The reactions mixtures were transformed (0.5 ul) into TOP10 electrocompetent cells (Invitrogen). SOC broth was added to make a total volume of 350 ul. Then, 25 ul or 50 ul suspensions of cells were plated on LA + 5ppm CMP (chloramphenicol) (random clones) or LA-5 ppm CMP + 0.1 ppm CTX (cefotaxime) (active clones). Following incubation for about 20 hours (*i.e.*, overnight) at 37°. The number of random and active colonies were compared and found to be comparable for all of the libraries. In the case of libraries NA02 and NA03, a single QCMS reaction was carried out, and it was split into 2 portions after *Dpn*I digestion. One portion, "NA02," was transformed directly into *E. coli* and the second portion, "NA03," was heated at 95°C for 2 min before transformation into *E. coli.* This was conducted to determine if denaturation of hemimethylated DNA by heating after *Dpn*I digestion would reduce the wild type template background in the libraries. No difference was observed in the wild type background in libraries NA02 and NA03. However, library NA01 had a significantly higher wild type background of 48% compared to NA02 and NA03, which had wild type backgrounds of only 17%.

The following list provides the sequences of 29 mutagenic oligonucleotides that were used to generate the combinatorial libraries (the position of the mutation is given based on the entire gene including a 20 amino acid propeptide). The T21A primer was later found to be incorrectly designed and the corresponding mutation was not observed in any of the isolates.

| | |
|---|---|
| A173S | CGCGTCTTTACGCCAACTCCAGCATCGGTCTTTTTG (SEQ ID NO:10) |

| | |
|---|---|
| A214S NO:11) | GGATTAACGTGCCGAAATCGGAAGAGGCGCATTAC (SEQ ID |
| A228P | GCTATCGTGACGGTAAACCGGTGCGCGTTTCGCCG (SEQ ID NO:12) |
| A33D | GCTGGCGGAGGTGGTCGACAATACGATTACCCCGCT (SEQ ID NO:13) |
| F63Y | ACCGCACTATTACACATATGGCAAGGCCGATATCGC (SEQ ID NO:14) |
| I282V | AGTCGCGCTACTGGCGTGTCGGGTCAATGTATCAG (SEQ ID NO:15) |
| I354L | CTTTATTCCTGAAAAGCAGCTCGGTATTGTGATGCTCGCG (SEQ ID NO:16) |
| I85V | CTGTTCGAGCTGGGTTCTGTAAGTAAAACCTTCACCG (SEQ ID NO:17) |
| M126L | AGTGGCAGGGTATTCGTCTGCTGGATCTCGCCACC (SEQ ID NO:18 |
| N246T NO:19) | CTATGGCGTGAAAACCACCGTGCAGGATATGGCGA (SEQ ID |
| N252R | ACGTGCAGGATATGGCGCGCTGGGTCATGGCCAACA (SEQ ID NO:22) |
| P315A | GTAAGGTAGCGCTAGCGGCGTTGCCCGTGGCAGAAG (SEQ ID NO:23) |
| Q115E NO:24) | TGACCAGATACTGGCCAGAGCTGACGGGCAAGCAG (SEQ ID |
| Q239E | CGGGTATGCTGGATGCAGAAGCCTATGGCGTGAAAAC (SEQ ID NO:25) |
| R111K NO:26) | GGACGATGCGGTGACCAAATACTGGCCACAGCTGA (SEQ ID |
| R125T | AGCAGTGGCAGGGTATTACTATGCTGGATCTCGCCA (SEQ ID NO:27) |
| S150A NO:28) | AGGTCACGGATAACGCCGCCCTGCTGCGCTTTTATC (SEQ ID |
| S24T NO:29) | TCTCGCCACGCCAGTGACAGAAAAACAGCTGGCGG (SEQ ID |
| S267T | GAGAACGTTGCTGATGCCACACTTAAGCAGGGCATCG (SEQ ID NO:30) |
| T21A NO:31) | CTTGCTCTGCTCTCGCCGCGCCAGTGTCAGAAAAAC (SEQ ID |
| T245S | CAAGCCTATGGCGTGAAATCCAACGTGCAGGATATGG (SEQ ID NO:32) |
| T362K | TGTGATGCTCGCGAATAAAAGCTATCCGAACCCGG (SEQ ID NO:33) |
| V247A NO:34) | TGGCGTGAAAACCAACGCGCAGGATATGGCGAACT (SEQ ID |
| V303L NO:35) | CCGTGGAGGCAAACACGCTGGTCGAGGGCAGCGAC (SEQ ID |
| V304I NO:36) | TGGAGGCAAACACGGTGATCGAGGGCAGCGACAGT (SEQ ID |
| V31I | GAAAAACAGCTGGCGGAGATCGTCGCGAATACGATTACC (SEQ ID NO:37) |
| V45I NO:38) | TGATGAAAGCACAGAGTATTCCAGGCATGGCGGTG (SEQ ID |
| Y190F | ACCTTCTGGCATGCCCTTTGAGCAGGCCATGACGA (SEQ ID NO:39) |
| Y61F | GGGAAAACCGCACTATTTCACATTTGGCAAGGCCG (SEQ ID NO:40) |
| T21A NO:41) | CTTGCTCTGCTCTCGCCGCGCCAGTGTCAGAAAAAC (SEQ ID |

## Sequencing

Thirty colonies from each library were sequenced using M13 reverse and Dbseq primers by Qiagen Genomic Services (Valencia, CA). The sequences of the primers used in this sequencing were:

M13 reverse: CAGGAAACAGCTATGAC (SEQ ID NO:42)
Dbseq: GCCGCTCAAGCTGGACCATA (SEQ ID NO:43)

The libraries were then screened and analyzed as described in Example 3. Statistical analysis indicated that 11 mutations appeared to stabilize the BLA protein, while 5 mutations appeared to destabilize it. The best clone, "NA03.8" was found to have 2 stabilizing and 1 neutral mutation.

Following the statistical analysis described below, an additional library "NA04," was constructed in order to introduce 9 stabilizing mutations into NA03.8.

## Screen for Thermostability

Libraries NA01, NA02, and NA03 were plated onto agar plates with LA medium containing 5 mg/l chloramphenicol. Thirty colonies from each library were transferred into a 96-well plate containing 200 ul LB(5 mg/l chloramphenicol). Four additional wells were inoculated with TOP10/pCB04, which served as control during the assay. A master plate was generated by adding glycerol and was stored frozen at - 80°C.

A 96-well plate containing 200 ul LB (5 mg/l chloramphenicol and 0.1 mg/l cefotaxime) was inoculated from the master plate using a replication tool. The plate was incubated for 3 days at 25°C in a humidified incubator at 225 rpm. The following operations were performed with each well of the cultured 96 well plate: 50 ul of culture were transferred into a plate that contained 50 ul B-PER reagent (Pierce). The suspension was incubated at room temperature for 90 min to lyze the cells and liberate BLA from the cells. The lysate was diluted 1000-fold and 10000 fold into 100 mM citrate/phosphate buffer pH 7.0 containing 0.125% octylglucopyranoside (Sigma). The diluted samples were heated to 56°C for 1 h with mixing at 650 rpm. Subsequently, 20 ul of the sample were transferred to 180 ul of nitrocefin assay buffer (0.1 mg/l nitrocefin in 50 mM phosphate buffered saline containing 0.125% octylglucopyranoside) and the BLA activity was determined using a Spectramax plus plate reader (Molecular Devices) at 490 nm. In parallel, a control sample was subjected to the same procedure but the heating step was omitted. Based on both activity readings, the fraction of BLA activity that remained after the heat treatment was calculated for each of the 90 variants and 4 controls on the plate.

Out of these 90 clones, 7 clones had mutations which were not intended and appeared to be PCR mistakes that occurred during the QuikChange® reaction. For 3 clones, less than 67% complete sequence was obtained. All clones with unintended mutations or <67% complete sequence were excluded from further analysis.

Figure 6 shows the remaining BLA activity of the 80 isolates from libraries NA01, NA02, and NA03. Of these isolates, 23 had no mutations. These variants are shown in black. It can be seen, that about 38% of the variants are more stable than wild type BLA. Table 2 provides the mutations that were detected in the 5 most stable BLA variants.

**Table 2. Mutations Detected in Stable BLA Variants**

| Clone | Mutations |
|---|---|
| NA03.8 | Q95E, A153S, I334L |
| NA01.18 | A13D, F43Y, I65V, Q95E, R105T, T225S, I262V, V284I, T342K |
| NA02.29 | S130A, A153S, A208P, T225S, V284I |
| NA03.20 | A13D, O95E, M106L, T225S, I262V, I334L |
| NA02.15 | A13D, V25I,I65V, A153S, Q219E, N232R, I262V |

## Statistical Analysis of the Correlation Between Sequence and Stability

The experiments described herein resulted in the identification of 80 isolates from the library for which stability measurements as well as sequence information were obtained. Of these 80 isolates, 23 contained no mutations, while the remaining 57 isolates contained between one and 11 of the consensus mutations. Seven of the isolates contained random mutations which were ignored in the statistical analysis.

Various statistical methods find use in making the determination of which mutations have a stabilizing effect. The description used herein is but one suitable method for this analysis. Thus, although an adaptation of the Free Wilson method was used here, other statistical methods or graphical analysis could have been used as well.

The contribution of each mutation to BLA stability was calculated based on the remaining activity of the 80 isolates using the Free Wilson method (Free and Wilson, J. Med. Chem., 7:395-399 [1964]). This method has been previously adapted to peptide substrates for proteases (*See e.g.*, Pozsgay et al., Eur. J. Biochem., 115:491-495 [1981]). However, it apparently has not been used to characterize protein variants. During the analysis described herein, it was assumed that individual mutations make additive contributions to the stability of the protein. The analysis included 80 variants for which sufficient sequence information was available. The method assigns a parameter $P_k$ to each of the m mutations in the data set. It also assumes that the remaining activity $R_i$ of each variant can be calculated based on these parameters using equation (1):

$$\log(R_i) = \sum_{k=1}^{m} M_{ki} P_k + C \qquad (1)$$

where $M_{ki}$ equals one if variant i contains mutation k, and zero, if variant i does not contain mutation k and C is a constant that should reflect the remaining activity of the wild type enzyme. The parameters were determined by solving equation (2) using the solver function in Microsoft Excel.

$$\sum_{i=1}^{n} \left\{ \log(R_i) - \sum_{k=1}^{m} M_{ki} P_k - C \right\} = \min \qquad (2)$$

The calculated parameters for some of the mutations are summarized in the Figure 4.

The data illustrate, that not all consensus mutations stabilize BLA. Several mutations, Y41F, I65V, M106L, Q219E, and P295A appear to have significantly destabilizing effect on BLA. The following mutations are of particular interest, as they show significant stabilizing effect on BLA: V11I, V25I, R91K, Q95E, A153S, N232R, S247T, I262V, V293L, V294I, T342K.

The most stable variant, NA03.8, was chosen as the starting template for a further combinatorial library (NA04, described below), in order to introduce several additional stabilizing mutations into variant NA03.8.

### Construction of Library NA04

Library NA04 was constructed using NA03.8 as template and 10 mutagenic primers as indicated below. One primer was designed to contain mutations V303L and V304I because these mutations can not be simultaneously introduced into a variant by individual mutagenic primers due to their proximity in the sequence. The combinatorial library NA04 was made with 10 mutagenic primers at a concentration of 0.04µM (*i.e.*, approximately 11ng of each primer). The other conditions used to construct the library were identical to the conditions indicated above for the construction of NA01 through NA03, above. The mutagenic primers are provided below (the position of the mutation is given based on the entire gene including a 20 amino acid pro-peptide).

| | |
|---|---|
| V31I | GAAAAACAGCTGGCGGAGATCGTCGCGAATACGATTACC (SEQ ID NO:44) |
| V45I NO:45) | TGATGAAAGCACAGAGTATTCCAGGCATGGCGGTG (SEQ ID |
| R111K NO:46) | GGACGATGCGGTGACCAAATACTGGCCACAGCTGA (SEQ ID |
| N252R | ACGTGCAGGATATGGCGCGCTGGGTCATGGCCAACA (SEQ ID NO:47) |
| S267T | GAGAACGTTGCTGATGCCACACTTAAGCAGGGCATCG (SEQ ID NO:48) |
| I282V | AGTCGCGCTACTGGCGTGTCGGGTCAATGTATCAG (SEQ ID NO:49) |
| V303L NO:50) | CCGTGGAGGCAAACACGCTGGTCGAGGGCAGCGAC (SEQ ID |
| V304I NO:51) | TGGAGGCAAACACGGTGATCGAGGGCAGCGACAGT (SEQ ID |
| T362K | TGTGATGCTCGCGAATAAAAGCTATCCGAACCCGG (SEQ ID NO:52) |
| V303, V304 | CCGTGGAGGCAAACACGCTGATCGAGGGCAGCGACAGTAAG (SEQ ID NO:53) |

Once the clones grew up, 616 clones from this library were screened for improved resistance to thermolysin, as described below in Example 2.

## EXAMPLE 2

### Screening of NA04 for Protease Resistance

In this Example, experiments conducted to screen the NA04 library for protease resistance. In particular, in these experiments, library NA04 was screened to identify variants that resist degradation by the protease thermolysin at elevated temperature. Thermolysin is a thermostable protease which has been found to preferentially cleave unfolded proteins *(See,* Arnold and Ulbrich-Hofmann, Biochem., 36:2166-2172 [1997]).

The library NA04 was plated onto LA agar containing 5 mg/l chloramphenicol and 0.1 mg/l cefotaxime and incubated for 30 h at 37°C. Colonies were transferred into eight 96-well plates containing 160 ul per well of LB medium containing 5 mg/l chloramphenicol and 0.1 mg/l cefotaxime using an automated colony picker. For each plate, 8 wells were inoculated, with variant NA03.8 used as control. The plates were incubated for 48 h at 37°C in a humidified incubator shaker. Subsequently, 70 ul of culture was transferred to a 96-well filter plate (Millipore) and 70 ul of B-PER reagent (Pierce) was added. After 30 min of incubation at room temperature to allow cell lysis, the plates were filtered producing clear lysate. Then, 90 ul of 25% glycerol was added to the remainder of the culture plates and they were stored at -80°C. The lysate was diluted 500-fold into destabilization buffer (50 mM imidazole pH 7.0, 10 mM $CaCl_2$, 0.005% Tween®-20, 1 mg/l thermolysin (Sigma)). Then, 40 ul of the samples was immediately transferred into a fresh plate containing 10 ul of 50 mM EDTA to inactivate thermolysin. Then, the samples were incubated for 1 hour in a water bath at 46°C to degrade unstable variants of BLA. Subsequently, a second sample of 40 ul was transferred into a fresh plate containing 10 ul of 50 mM EDTA. The amount of BLA activity was measured in both samples (obtained before and after heat treatment) by addition of 25 ul of sample into 175 ul of assay buffer (0.1 mg/l nitrocefin in 50 mM phosphate buffered saline containing 0.125% octylglucopyranoside), and the BLA activity was determined using a Spectramax plus plate reader (Molecular Devices) at 490 nm. The fraction of remaining BLA activity was calculated for each variant and 22 stabilized variants were chosen for further analysis.

The stability of the 22 variants was confirmed by repeating the same assay but testing 4 wells for each variants. During the confirmation experiment, the 22 stabilized variants had remaining activities of 24-45% whereas the parent, NA03.8, had only 13.5% of its activity remaining after thermolysin treatment. Table 3 provides the remaining activity and mutations for the 6 most stable variants.

**Table 3. Remaining Activity and Mutations for Six Variants**

| Variant | Remaining Activity (%) | Mutations |
|---|---|---|
| NA03.8 (parent) | 13.5 | None |
| NA04.2 | 40 | R91K, S247T, I262V |
| NA04.10 | 39 | V11I, V25I, N232R, I262V, V284I |
| NA04.14 | 40 | V11I, R91K, N232R, I262V, V284I |
| NA04.17 | 45 | V25I, R91K, N232R, I262V, V284I |
| NA04.18 | 39 | V25I, R91K, I262V, |
| NA04.22 | 40 | V11I, V25I, R91K, N232R, S247T, I262V, V284I, T342K |

In addition, 40 random variants were also isolated from library NA04 to assess the sequence variation in the library. All 9 intended mutations were observed at frequencies between 13-50%. Random clones from library NA04 contained an average of 3.15 mutations versus 3.9 mutations for the 22 stabilized variants. It was observed that 3 mutations, R91K, I262V, and V284I, were significantly enriched during the screen, which indicates that these 3 mutations have particularly significant stabilizing effect on BLA. In contrast, mutation V25I was reduced in its frequency during the screen which suggest, that this change is destabilizing BLA *(See,* Figure 3).

## EXAMPLE 3

### Testing the Protease Stability of BLA Variants

In this Example, experiments conducted to test the protease stability of three BLA variants (NA03.8, NA04.2, and NA04.17) produced in Example 1 are described. As a control, the parent BLA (pCB04) was also tested. The host cells expressing these variants and control BLA were inoculated into 1 L Terrific Broth containing 5 mg/l chloramphenicol and incubated at 37°C over night. Cells were harvested by centrifugation (6000 xg for 15 minutes). The pellets were resuspended in 200 ml of phosphate-buffered B-PER solution (Pierce). The suspensions were shaken for about 1 hour at room temperature until the pellets were solubilized. Cell wall debris and insoluble protein were removed by centrifugation (15000xg for 15 minutes). The supernatants were stored at 4°C, until purification.

Proteins were first purified using Ni-IMAC (Applied Biosystems). The purification was done on Bio-Cat (PerSeptive Biosystems, Applied Biosystems). A Waters column of 22mm x 95 mm was used. The column was first loaded with 250mM NiCl, then it was washed with water and equilibrated with 10mM HEPES, 0.5M NaCl, pH 8.4. Samples were loaded onto the column, washed with equilibration buffer, and eluted with 10mM HEPES, 0.5M NaCl and a gradient of 200mM imidazole.

The eluted protein was further purified by affinity chromatography using m-aminophenylboronic acid (PBA) resin (SIGMA). This purification was done by gravity flow. 15 ml PBA resin was packed in a disposable column 15 x 120 mm (Bio-Rad) and equilibrated with 20mM TEA, 0.5M NaCl, pH 7. After loading the sample, the columns were washed with 4 column volumes of equilibration buffer, and subsequently BLA was eluted with 0.5M sodium borate, 0.5M NaCl, pH 7. A purity level of 99% was achieved for these proteins, as determined by SDS-PAGE.

Purified proteins (~1ug) were incubated with different concentrations of each test protease in 100mM Tris-HCl 10mM CaC12 0.005% TWEEN®20 pH, 7.9 for different time periods at 37°C in quadruplicates. Trypsin, chymotrypsin, and thermolysin (SIGMA) were tested in these experiments. The BLA activity was measured for samples with protease and without protease by monitoring the hydrolysis of its chromogenic substrate nitrocefin (Oxoid). The remaining activity of protease-treated sample to untreated sample in percent was calculated for each variant (*i.e.*, relative remaining activity). The data were normalized to the most stable variant. Figure 5 provides a graph showing the relative remaining activity of these variants upon exposure to these proteases. As compared to the parent protein, all three of the stabilized variants of BLA were found to be significantly more resistant to protease cleavage by all of the test proteases.

## EXAMPLE 4

### Stabilization of an scFv

In this Example, experiments conducted to stabilize a single chain variable fragment (scFv) are described. As described below, the methods of the present invention provide means to identify stabilized variants of CAB1-scFv. Indeed, the method allowed for the screening of relatively small libraries, with six changes being accumulated in the best-performing variant. The Example also demonstrates that fusion of the CAB1-scFv greatly facilitates the identification of improved variants of this molecule.

### A. Construction of pME27.1

Plasmid pME27.1 was generated by inserting a *Bgl*I/ *Eco*RV fragment encoding a part of the pelB leader, the CAB1-scFv and a small part of BLA into the expression vector pME25. The amino acid sequence of CAB 1 is provided in Figure 7. Figure 8 provides a map of this plasmid, while Figure 9 provides its nucleotide sequence (SEQ ID NO:6). The insert, encoding for the CAB1-scFv, has been synthesized by Aptagen, based on the sequence of the previously described scFv MFE-23 (*See,* Boehm et al., Biochem. J., 346(Pt 2): 519-28 [2000]). Both the plasmid containing the synthetic gene (pPCR-GME1) and pME25 were digested with *Bgl*I and *Eco*RV, gel purified and ligated together with ligase using the Takara DNA ligation kit (Takara) according to the manufacturer's instructions. The ligated product was transformed into TOP10 (Invitrogen) electrocompetent cells, plated on LA medium containing 5 mg/l chloramphenicol and 0.1 mg/l cefotaxime.

Plasmid pME27.1 contains the following features (bases indicated):

| | |
|---|---|
| **P lac:** | 4992-5113 bp |
| **pel B leader:** | 13-78 |
| **CAB 1 scFv:** | 79-810 |
| **BLA:** | 811-1896 |
| **T7 term.:** | 2076-2122 |
| **CAT:** | 3253-3912 |

The CAB1 sequence, indicating heavy (SEQ ID NO:2) and light (SEQ ID NO:4) chain domains, as well as the linker (SEQ ID NO:3), and BLA (SEQ ID NO:5) is provided in Figure 7.

## B. Choosing Mutations for Mutagenesis

The sequence of the vH and vL sequences of CAB1-scFv were compared with a published frequency analysis of human antibodies (Steipe, Sequenzdatenanalyse. ("Sequence Data Analysis", available in German only) in Zorbas and Lottspeich (eds.), Bioanalytik, Spektrum Akademischer Verlag. S. 233-241 [1998]). The authors aligned sequences of variable segments of human antibodies as found in the Kabat data base and calculated the frequency of occurrence of each amino acid for each position. The frequencies were published by the authors on the internet and are shown in Tables 4 and 5. The Tables also show the sequence of CAB1-scFv, the location of the CDRs, and they show which positions were selected for CCM.

**Table 4. Amino Acid Frequencies in Heavy Chains of Human Antibodies**

| Position (Heavy Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 1 | 291 | E | 0.616 | Q | 0.346 | D | 0.014 | G | 0.014 | A | 0.003 | L | 0.003 | Q | | |
| 2 | 293 | V | 0.887 | M | 0.027 | L | 0.024 | S | 0.020 | I | 0.017 | A | 0.007 | V | | |
| 3 | 291 | Q | 0.852 | H | 0.034 | R | 0.027 | T | 0.027 | E | 0.014 | V | 0.014 | K | | 1 |
| 4 | 282 | L | 0.975 | V | 0.011 | A | 0.007 | D | 0.004 | M | 0.004 | | | L | | |
| 5 | 276 | V | 0.645 | Q | 0.148 | L | 0.120 | R | 0.022 | M | 0.014 | N | 0.014 | Q | | |
| 6 | 267 | E | 0.693 | Q | 0.263 | A | 0.022 | D | 0.011 | G | 0.007 | R | 0.004 | Q | | |
| 7 | 265 | S | 0.951 | W | 0.019 | X | 0.015 | T | 0.008 | A | 0.004 | N | 0.004 | S | | |
| 8 | 266 | G | 0.989 | S | 0.008 | T | 0.004 | | | | | | | G | | |
| 9 | 274 | G | 0.624 | A | 0.193 | P | 0.164 | S | 0.011 | E | 0.004 | H | 0.004 | A | | |
| 10 | 271 | G | 0.638 | E | 0.192 | D | 0.081 | A | 0.070 | T | 0.011 | V | 0.007 | E | | |
| 11 | 270 | L | 0.681 | V | 0.270 | F | 0.030 | S | 0.019 | | | | | L | | |
| 12 | 267 | V | 0.757 | K | 0.154 | I | 0.026 | N | 0.022 | L | 0.015 | A | 0.007 | V | | |
| 13 | 247 | K | 0.474 | Q | 0.428 | R | 0.049 | E | 0.034 | G | 0.004 | H | 0.004 | R | | 1 |
| 14 | 251 | P | 0.968 | A | 0.012 | K | 0.008 | G | 0.004 | L | 0.004 | S | 0.004 | S | | 1 |
| 15 | 244 | G | 0.783 | S | 0.156 | T | 0.033 | P | 0.016 | K | 0.008 | E | 0.004 | G | | |
| 16 | 243 | G | 0.488 | E | 0.131 | Q | 0.107 | A | 0.094 | R | 0.082 | S | 0.066 | T | | 1 |
| 17 | 234 | S | 0.766 | T | 0.204 | A | 0.009 | F | 0.009 | P | 0.004 | R | 0.004 | S | | |
| 18 | 244 | L | 0.812 | V | 0.155 | M | 0.008 | A | 0.004 | E | 0.004 | F | 0.004 | V | | |
| 19 | 242 | R | 0.545 | K | 0.240 | S | 0.161 | T | 0.037 | A | 0.012 | Q | 0.004 | K | | |
| 20 | 246 | L | 0.736 | V | 0.191 | I | 0.061 | E | 0.004 | R | 0.004 | X | 0.004 | L | | |
| 21 | 218 | S | 0.729 | T | 0.234 | G | 0.009 | I | 0.009 | A | 0.005 | D | 0.005 | S | | |
| 22 | 217 | C | 0.991 | R | 0.005 | S | 0.005 | | | | | | | C | | |
| 23 | 231 | A | 0.558 | K | 0.203 | T | 0.117 | E | 0.048 | V | 0.022 | I | 0.013 | T | | |
| 24 | 235 | A | 0.638 | V | 0.174 | G | 0.064 | I | 0.055 | T | 0.030 | F | 0.026 | A | | |
| 25 | 226 | S | 0.951 | Y | 0.027 | F | 0.009 | C | 0.004 | K | 0.004 | T | 0.004 | S | | |
| 26 | 225 | G | 0.956 | E | 0.013 | A | 0.009 | D | 0.009 | S | 0.009 | V | 0.004 | G | | |
| 27 | 213 | F | 0.559 | Y | 0.164 | G | 0.150 | D | 0.080 | S | 0.019 | L | 0.014 | F | | |
| 28 | 203 | T | 0.571 | S | 0.286 | I | 0.049 | N | 0.049 | P | 0.015 | A | 0.005 | N | | 1 |
| 29 | 207 | F | 0.749 | V | 0.111 | I | 0.068 | L | 0.053 | T | 0.010 | A | 0.005 | I | | 1 |
| 30 | 202 | S | 0.762 | T | 0.119 | N | 0.035 | G | 0.020 | R | 0.020 | A | 0.010 | K | | 1 |
| 31 | 199 | S | 0.482 | T | 0.136 | D | 0.104 | N | 0.087 | G | 0.060 | K | 0.040 | D | H1 | |
| 32 | 202 | Y | 0.535 | S | 0.144 | N | 0.083 | A | 0.069 | D | 0.031 | G | 0.030 | S | H1 | |
| 33 | 197 | A | 0.269 | Y | 0.162 | G | 0.147 | W | 0.117 | S | 0.091 | T | 0.066 | Y | H1 | |
| 34 | 200 | M | 0.520 | I | 0.210 | W | 0.070 | A | 0.055 | Y | 0.050 | V | 0.040 | M | H1 | |
| 35 | 196 | S | 0.372 | H | 0.235 | N | 0.077 | A | 0.061 | G | 0.051 | Y | 0.046 | H | H1 | |
| 35a | 33 | - | 0.824 | W | 0.096 | V | 0.043 | G | 0.016 | S | 0.016 | N | 0.005 | | H1 | |
| 35b | 27 | - | 0.856 | N | 0.064 | G | 0.037 | S | 0.032 | A | 0.005 | R | 0.005 | | H1 | |
| 36 | 192 | W | 0.990 | M | 0.005 | T | 0.005 | | | | | | | W | | |
| 37 | 193 | V | 0.741 | I | 0.228 | L | 0.021 | G | 0.005 | Q | 0.005 | | | L | | 1 |
| 38 | 190 | R | 0.989 | P | 0.005 | V | 0.005 | | | | | | | R | | |
| 39 | 190 | Q | 0.979 | T | 0.011 | G | 0.005 | R | 0.005 | | | | | Q | | |

| Position (Heavy Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 40 | 191 | A | 0.634 | P | 0.199 | S | 0.073 | M | 0.052 | G | 0.010 | V | 0.010 | G | | 1 |
| 41 | 187 | P | 0.914 | S | 0.043 | T | 0.021 | A | 0.005 | L | 0.005 | Q | 0.005 | P | | |
| 42 | 187 | G | 0.925 | S | 0.064 | P | 0.005 | R | 0.005 | | | | | E | | 1 |
| 43 | 186 | K | 0.683 | Q | 0.183 | R | 0.124 | E | 0.005 | H | 0.005 | | | Q | | |
| 44 | 186 | G | 0.882 | A | 0.048 | S | 0.043 | R | 0.027 | | | | | G | | |
| 45 | 186 | L | 0.978 | P | 0.022 | | | | | | | | | L | | |
| 46 | 185 | E | 0.956 | Q | 0.039 | V | 0.005 | | | | | | | E | | |
| 47 | 184 | W | 0.989 | S | 0.011 | | | | | | | | | W | | |
| 48 | 185 | V | 0.481 | M | 0.222 | I | 0.173 | L | 0.124 | | | | | I | | |
| 49 | 185 | G | 0.600 | S | 0.216 | A | 0.162 | E | 0.005 | L | 0.005 | T | 0.005 | G | | |
| 50 | 185 | R | 0.146 | W | 0.146 | V | 0.119 | A | 0.114 | G | 0.081 | Y | 0.081 | W | H2 | |
| 51 | 185 | I | 0.822 | T | 0.081 | R | 0.027 | V | 0.022 | K | 0.016 | M | 0.011 | I | H2 | |
| 52 | 184 | S | 0.250 | Y | 0.239 | N | 0.123 | K | 0.060 | I | 0.054 | D | 0.050 | D | H2 | |
| 52a | 141 | - | 0.230 | P | 0.180 | Y | 0.153 | G | 0.126 | N | 0.066 | V | 0.055 | P | H2 | |
| 52b | 34 | - | 0.814 | K | 0.115 | R | 0.060 | G | 0.005 | Y | 0.005 | | | | H2 | |
| 52c | 22 | - | 0.880 | T | 0.044 | V | 0.033 | S | 0.022 | A | 0.011 | G | 0.005 | | H2 | |
| 53 | 184 | S | 0.228 | D | 0.163 | Y | 0.125 | G | 0.109 | N | 0.082 | H | 0.054 | E | H2 | |
| 54 | 183 | G | 0.328 | S | 0.202 | D | 0.129 | N | 0.112 | K | 0.082 | F | 0.055 | N | H2 | |
| 55 | 182 | G | 0.544 | S | 0.181 | D | 0.085 | W | 0.066 | Y | 0.060 | N | 0.020 | G | H2 | |
| 56 | 182 | S | 0.231 | D | 0.182 | N | 0.147 | T | 0.143 | Y | 0.077 | G | 0.060 | D | H2 | |
| 57 | 184 | T | 0.582 | K | 0.120 | N | 0.065 | A | 0.054 | I | 0.054 | P | 0.022 | T | H2 | |
| 58 | 183 | Y | 0.322 | N | 0.216 | D | 0.139 | R | 0.060 | H | 0.055 | T | 0.038 | E | H2 | |
| 59 | 184 | Y | 0.908 | F | 0.043 | N | 0.016 | S | 0.011 | D | 0.005 | G | 0.005 | Y | H2 | |
| 60 | 183 | A | 0.579 | N | 0.153 | S | 0.104 | T | 0.055 | R | 0.044 | G | 0.027 | A | H2 | |
| 61 | 184 | D | 0.277 | P | 0.239 | Q | 0.174 | A | 0.141 | V | 0.076 | T | 0.033 | P | H2 | |
| 62 | 185 | S | 0.686 | K | 0.146 | P | 0.065 | N | 0.038 | G | 0.016 | R | 0.016 | K | H2 | |
| 63 | 186 | V | 0.511 | L | 0.247 | F | 0.215 | S | 0.011 | A | 0.005 | K | 0.005 | F | H2 | |
| 64 | 186 | K | 0.581 | Q | 0.274 | R | 0.054 | N | 0.032 | E | 0.022 | T | 0.022 | Q | H2 | |
| 65 | 186 | G | 0.688 | S | 0.237 | T | 0.032 | A | 0.016 | D | 0.011 | E | 0.011 | G | H2 | |
| 66 | 186 | R | 0.935 | Q | 0.054 | H | 0.005 | I | 0.005 | | | | | K | | 1 |
| 67 | 186 | F | 0.462 | V | 0.409 | I | 0.065 | L | 0.054 | A | 0.005 | S | 0.005 | A | | 1 |
| 68 | 186 | T | 0.914 | I | 0.038 | A | 0.016 | S | 0.011 | K | 0.005 | N | 0.005 | T | | |
| 69 | 187 | I | 0.791 | M | 0.139 | V | 0.032 | D | 0.005 | F | 0.005 | G | 0.005 | F | | 1 |
| 70 | 187 | S | 0.684 | T | 0.214 | N | 0.070 | L | 0.032 | | | | | T | | |
| 71 | 187 | R | 0.529 | V | 0.160 | A | 0.107 | P | 0.064 | T | 0.053 | K | 0.043 | T | | 1 |
| 72 | 186 | D | 0.902 | N | 0.071 | K | 0.016 | E | 0.011 | | | | | D | | |
| 73 | 185 | T | 0.368 | N | 0.266 | D | 0.177 | K | 0.070 | E | 0.059 | A | 0.011 | T | | |
| 74 | 186 | S | 0.946 | A | 0.048 | L | 0.005 | | | | | | | S | | |
| 75 | 187 | K | 0.674 | T | 0.139 | I | 0.070 | R | 0.027 | A | 0.021 | F | 0.021 | S | | 1 |
| 76 | 187 | N | 0.701 | S | 0.251 | K | 0.027 | R | 0.011 | T | 0.005 | Y | 0.005 | N | | |
| 77 | 187 | T | 0.615 | Q | 0.273 | S | 0.048 | M | 0.021 | L | 0.016 | P | 0.011 | T | | |

| Position (Heavy Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 78 | 186 | L | 0.364 | A | 0.273 | F | 0.235 | V | 0.096 | I | 0.005 | M | 0.005 | A | | |
| 79 | 187 | Y | 0.638 | S | 0.239 | F | 0.059 | V | 0.048 | H | 0.005 | M | 0.005 | Y | | |
| 80 | 187 | L | 0.782 | M | 0.207 | N | 0.005 | - | 0.005 | | | | | L | | |
| 81 | 187 | Q | 0.529 | E | 0.205 | K | 0.122 | R | 0.032 | T | 0.032 | N | 0.027 | Q | | |
| 82 | 194 | M | 0.497 | L | 0.421 | W | 0.051 | V | 0.015 | I | 0.010 | - | 0.005 | L | | |
| 82a | 195 | N | 0.442 | S | 0.291 | R | 0.077 | T | 0.066 | D | 0.053 | G | 0.020 | S | | |
| 82b | 194 | S | 0.795 | N | 0.082 | R | 0.051 | G | 0.026 | T | 0.021 | A | 0.010 | S | | |
| 82c | 197 | L | 0.701 | V | 0.234 | M | 0.041 | G | 0.010 | A | 0.005 | D | 0.005 | L | | |
| 83 | 197 | R | 0.528 | T | 0.239 | K | 0.122 | D | 0.041 | E | 0.020 | Q | 0.015 | T | | |
| 84 | 198 | A | 0.495 | P | 0.182 | S | 0.177 | T | 0.051 | I | 0.035 | V | 0.030 | S | | |
| 85 | 198 | E | 0.591 | A | 0.172 | D | 0.126 | S | 0.051 | V | 0.045 | G | 0.015 | E | | |
| 86 | 198 | D | 0.975 | T | 0.010 | V | 0.010 | N | 0.005 | | | | | D | | |
| 87 | 198 | T | 0.929 | S | 0.035 | G | 0.010 | M | 0.010 | A | 0.005 | Q | 0.005 | T | | |
| 88 | 198 | A | 0.939 | G | 0.040 | P | 0.005 | T | 0.005 | V | 0.005 | Y | 0.005 | A | | |
| 89 | 198 | V | 0.768 | L | 0.066 | M | 0.056 | T | 0.045 | I | 0.040 | F | 0.010 | V | | |
| 90 | 199 | Y | 0.980 | F | 0.010 | A | 0.005 | I | 0.005 | | | | | Y | | |
| 91 | 199 | Y | 0.930 | F | 0.045 | C | 0.015 | R | 0.005 | T | 0.005 | | | Y | | |
| 92 | 198 | C | 0.990 | A | 0.005 | M | 0.005 | | | | | | | C | | |
| 93 | 198 | A | 0.838 | T | 0.076 | V | 0.061 | H | 0.005 | K | 0.005 | N | 0.005 | N | | 1 |
| 94 | 198 | R | 0.596 | K | 0.162 | T | 0.051 | G | 0.045 | P | 0.045 | Q | 0.025 | E | | 1 |
| 95 | 161 | G | 0.174 | D | 0.120 | E | 0.099 | A | 0.093 | N | 0.092 | P | 0.068 | G | | |
| 96 | 159 | P | 0.168 | R | 0.130 | G | 0.112 | L | 0.062 | V | 0.062 | Y | 0.062 | T | H3 | |
| 97 | 156 | G | 0.170 | P | 0.094 | V | 0.094 | E | 0.088 | T | 0.069 | S | 0.063 | P | H3 | |
| 98 | 155 | G | 0.152 | Y | 0.101 | L | 0.095 | D | 0.087 | V | 0.076 | S | 0.063 | T | H3 | |
| 99 | 143 | G | 0.172 | Y | 0.108 | T | 0.102 | - | 0.089 | A | 0.076 | E | 0.070 | G | H3 | |
| 100 | 131 | - | 0.171 | S | 0.165 | Y | 0.146 | G | 0.095 | V | 0.070 | R | 0.051 | P | H3 | |
| 100a | 110 | - | 0.304 | G | 0.146 | S | 0.095 | D | 0.046 | A | 0.044 | L | 0.044 | Y | H3 | |
| 100b | 99 | - | 0.369 | G | 0.134 | S | 0.127 | T | 0.076 | Y | 0.045 | V | 0.038 | Y | H3 | |
| 100c | 92 | - | 0.410 | G | 0.122 | Y | 0.103 | D | 0.058 | S | 0.058 | P | 0.045 | | H3 | |
| 100d | 72 | - | 0.538 | Y | 0.058 | G | 0.051 | S | 0.051 | C | 0.045 | L | 0.038 | | H3 | |
| 100e | 62 | - | 0.600 | Y | 0.155 | S | 0.045 | F | 0.032 | G | 0.032 | A | 0.026 | | H3 | |
| 100f | 53 | - | 0.658 | Y | 0.097 | H | 0.039 | R | 0.039 | P | 0.026 | S | 0.026 | | H3 | |
| 100g | 41 | - | 0.735 | Y | 0.084 | G | 0.065 | Q | 0.026 | S | 0.019 | D | 0.013 | | H3 | |
| 100h | 30 | - | 0.806 | Y | 0.058 | D | 0.032 | A | 0.019 | G | 0.019 | S | 0.019 | | H3 | |
| 100i | 24 | - | 0.844 | Y | 0.039 | G | 0.026 | X | 0.019 | L | 0.013 | N | 0.013 | | H3 | |
| 100j | 80 | - | 0.481 | Y | 0.149 | A | 0.117 | W | 0.084 | F | 0.045 | G | 0.039 | | H3 | |
| 100k | 138 | F | 0.503 | M | 0.144 | L | 0.137 | - | 0.098 | D | 0.039 | V | 0.033 | F | H3 | |
| 101 | 149 | D | 0.754 | A | 0.073 | R | 0.066 | N | 0.020 | Q | 0.020 | P | 0.013 | D | H3 | |
| 102 | 151 | Y | 0.368 | V | 0.224 | I | 0.112 | S | 0.086 | P | 0.072 | H | 0.053 | Y | H3 | |
| 103 | 154 | W | 0.955 | E | 0.013 | F | 0.013 | D | 0.006 | R | 0.006 | Y | 0.006 | W | | |
| 104 | 154 | G | 0.974 | Y | 0.013 | D | 0.006 | T | 0.006 | | | | | G | | |

| Position (Heavy Chain) | Number of Observations | Observed Frequencies of 5 Most Abundant Amino Acids in Alignment of Human Sequences | | | | | | | | | | | CAB1 Sequence | CDR | Mutated Residues |
|---|---|---|---|---|---|---|---|---|---|---|---|---|---|---|---|
| 105 | 154 | Q | 0.798 | R | 0.104 | K | 0.045 | E | 0.013 | N | 0.013 | S 0.013 | Q | | |
| 106 | 155 | G | 0.987 | Y | 0.006 | - | 0.006 | | | | | | G | | |
| 107 | 152 | T | 0.908 | S | 0.026 | V | 0.020 | G | 0.013 | I | 0.007 | L 0.007 | T | | |
| 108 | 152 | L | 0.645 | T | 0.178 | M | 0.105 | P | 0.020 | K | 0.013 | R 0.013 | T | | |
| 109 | 151 | V | 0.967 | L | 0.013 | I | 0.007 | M | 0.007 | X | 0.007 | | V | | |
| 110 | 151 | T | 0.940 | S | 0.026 | I | 0.013 | A | 0.007 | H | 0.007 | V 0.007 | T | | |
| 111 | 137 | V | 0.978 | I | 0.015 | T | 0.007 | | | | | | V | | |
| 112 | 138 | S | 0.971 | T | 0.014 | R | 0.007 | V | 0.007 | | | | S | | |
| 113 | 131 | S | 0.962 | P | 0.015 | A | 0.008 | L | 0.008 | T | 0.008 | | S | | |

**Table 5. Amino Acid Frequencies in Human vL Fragments**

| Position (Light Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 1 | 95 | Q | 0.589 | S | 0.158 | N | 0.095 | H | 0.074 | D | 0.053 | F | 0.021 | E | | 1 |
| 2 | 139 | S | 0.446 | Y | 0.388 | F | 0.101 | V | 0.043 | L | 0.014 | T | 0.007 | N | | 1 |
| 3 | 140 | V | 0.307 | E | 0.243 | A | 0.207 | M | 0.093 | D | 0.064 | I | 0.043 | V | | |
| 4 | 140 | L | 0.971 | V | 0.029 | | | | | | | | | L | | |
| 5 | 141 | T | 0.915 | A | 0.021 | S | 0.021 | I | 0.014 | K | 0.007 | L | 0.007 | T | | |
| 6 | 140 | Q | 0.993 | E | 0.007 | | | | | | | | | Q | | |
| 7 | 139 | P | 0.906 | D | 0.029 | S | 0.029 | A | 0.022 | E | 0.014 | | | S | | 1 |
| 8 | 139 | P | 0.741 | A | 0.137 | H | 0.072 | R | 0.029 | L | 0.007 | S | 0.007 | P | | |
| 9 | 139 | S | 0.964 | A | 0.014 | V | 0.014 | R | 0.007 | | | | | A | | 1 |
| 10 | 0 | - | 1.000 | | | | | | | | | | | I | | 1 |
| 11 | 138 | V | 0.790 | A | 0.138 | L | 0.058 | M | 0.014 | | | | | M | | 1 |
| 12 | 139 | S | 0.978 | F | 0.007 | T | 0.007 | E | 0.004 | Q | 0.004 | | | S | | |
| 13 | 138 | V | 0.406 | G | 0.348 | A | 0.138 | E | 0.087 | L | 0.014 | D | 0.007 | A | | |
| 14 | 135 | S | 0.630 | A | 0.230 | T | 0.111 | D | 0.007 | F | 0.007 | G | 0.007 | S | | |
| 15 | 135 | P | 0.881 | L | 0.089 | A | 0.022 | S | 0.007 | | | | | P | | |
| 16 | 134 | G | 0.978 | E | 0.015 | L | 0.007 | | | | | | | G | | |

| Position (Light Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 17 | 133 | Q | 0.811 | K | 0.098 | A | 0.045 | E | 0.024 | G | 0.015 | H | 0.008 | E | | 1 |
| 18 | 133 | T | 0.504 | S | 0.263 | R | 0.135 | K | 0.068 | E | 0.008 | G | 0.008 | K | | 1 |
| 19 | 130 | V | 0.454 | A | 0.385 | I | 0.146 | G | 0.008 | L | 0.008 | | | V | | |
| 20 | 128 | T | 0.531 | R | 0.188 | S | 0.148 | K | 0.047 | I | 0.031 | M | 0.016 | T | | |
| 21 | 121 | I | 0.901 | V | 0.050 | L | 0.017 | A | 0.008 | F | 0.008 | M | 0.008 | I | | |
| 22 | 120 | S | 0.492 | T | 0.475 | A | 0.008 | G | 0.008 | I | 0.008 | N | 0.008 | T | | |
| 23 | 117 | C | 1.000 | | | | | | | | | | | C | | |
| 24 | 112 | S | 0.536 | T | 0.259 | G | 0.089 | A | 0.045 | Q | 0.033 | I | 0.018 | S | L1 | |
| 25 | 108 | G | 0.870 | L | 0.056 | R | 0.028 | A | 0.019 | I | 0.009 | P | 0.009 | A | L1 | |
| 26 | 108 | D | 0.339 | S | 0.250 | T | 0.213 | N | 0.087 | E | 0.037 | G | 0.037 | S | L1 | |
| 27 | 104 | S | 0.415 | N | 0.118 | K | 0.113 | A | 0.104 | T | 0.066 | G | 0.047 | S | L1 | |
| 28 | 104 | L | 0.346 | S | 0.346 | I | 0.115 | G | 0.067 | A | 0.058 | D | 0.019 | S | L1 | |
| 29 | 100 | G | 0.243 | N | 0.239 | D | 0.159 | S | 0.078 | P | 0.068 | H | 0.058 | V | L1 | |
| 30 | 103 | I | 0.291 | V | 0.165 | D | 0.136 | N | 0.107 | E | 0.058 | S | 0.049 | S | L1 | |
| 31 | 101 | G | 0.356 | K | 0.168 | A | 0.099 | E | 0.084 | Q | 0.084 | D | 0.069 | Y | L1 | |
| 31a | 54 | - | 0.438 | S | 0.167 | G | 0.104 | N | 0.083 | Y | 0.063 | D | 0.052 | M | L1 | |
| 31b | 49 | - | 0.495 | N | 0.227 | Y | 0.155 | S | 0.041 | G | 0.021 | H | 0.021 | H | L1 | |
| 31c | 23 | - | 0.760 | N | 0.134 | S | 0.031 | K | 0.021 | D | 0.012 | E | 0.010 | | L1 | |
| 31d | 0 | - | 1.000 | | | | | | | | | | | | L1 | |
| 31e | 0 | - | 1.000 | | | | | | | | | | | | L1 | |
| 31f | 0 | - | 1.000 | | | | | | | | | | | | L1 | |
| 32 | 94 | Y | 0.515 | S | 0.134 | F | 0.093 | A | 0.072 | T | 0.052 | H | 0.041 | | L1 | |
| 33 | 97 | V | 0.680 | A | 0.186 | I | 0.082 | Y | 0.021 | F | 0.010 | P | 0.010 | | L1 | |
| 34 | 92 | S | 0.380 | H | 0.120 | A | 0.109 | Y | 0.098 | N | 0.076 | Q | 0.076 | | L1 | |
| 35 | 98 | W | 0.990 | Y | 0.010 | | | | | | | | | W | | |
| 36 | 96 | Y | 0.844 | F | 0.073 | H | 0.073 | W | 0.010 | | | | | F | | 1 |
| 37 | 95 | Q | 0.916 | R | 0.042 | E | 0.011 | H | 0.011 | K | 0.011 | Y | 0.011 | Q | | |
| 38 | 94 | Q | 0.862 | H | 0.053 | L | 0.053 | E | 0.011 | K | 0.011 | V | 0.011 | Q | | |
| 39 | 93 | K | 0.333 | L | 0.172 | R | 0.161 | H | 0.151 | Q | 0.086 | V | 0.043 | K | | |
| 40 | 93 | P | 0.946 | S | 0.022 | A | 0.011 | L | 0.011 | R | 0.011 | | | P | | |
| 41 | 93 | G | 0.871 | H | 0.065 | D | 0.022 | R | 0.022 | P | 0.011 | V | 0.011 | G | | |
| 42 | 92 | Q | 0.424 | T | 0.217 | K | 0.163 | R | 0.087 | S | 0.054 | G | 0.022 | T | | |
| 43 | 92 | A | 0.717 | S | 0.174 | G | 0.065 | T | 0.022 | L | 0.011 | V | 0.011 | S | | |
| 44 | 93 | P | 0.978 | A | 0.011 | M | 0.011 | | | | | | | P | | |
| 45 | 92 | K | 0.391 | V | 0.315 | R | 0.109 | L | 0.065 | T | 0.065 | A | 0.033 | K | | |
| 46 | 92 | L | 0.728 | V | 0.076 | F | 0.065 | T | 0.043 | A | 0.022 | M | 0.022 | L | | |
| 47 | 91 | V | 0.484 | L | 0.374 | I | 0.077 | M | 0.055 | N | 0.011 | | | W | | 1 |
| 48 | 91 | I | 0.791 | V | 0.110 | M | 0.077 | L | 0.011 | S | 0.011 | | | I | | |
| 49 | 91 | Y | 0.769 | F | 0.110 | R | 0.066 | H | 0.022 | D | 0.011 | I | 0.011 | Y | | |
| 50 | 89 | D | 0.303 | E | 0.210 | Q | 0.093 | V | 0.067 | G | 0.056 | K | 0.056 | S | L2 | |
| 51 | 88 | D | 0.364 | N | 0.205 | V | 0.159 | H | 0.068 | T | 0.068 | G | 0.034 | T | L2 | |

| Position (Light Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 52 | 89 | N | 0.393 | T | 0.213 | S | 0.202 | D | 0.101 | A | 0.022 | F | 0.011 | S | L2 | |
| 53 | 88 | K | 0.307 | D | 0.193 | Q | 0.182 | N | 0.080 | E | 0.057 | S | 0.057 | N | L2 | |
| 54 | 88 | R | 0.875 | X | 0.068 | K | 0.034 | L | 0.011 | W | 0.011 | | | L | L2 | |
| 55 | 86 | P | 0.851 | G | 0.080 | S | 0.023 | A | 0.011 | H | 0.011 | R | 0.011 | A | L2 | |
| 56 | 85 | S | 0.837 | D | 0.081 | P | 0.023 | A | 0.012 | L | 0.012 | T | 0.012 | S | L2 | |
| 57 | 86 | G | 0.920 | E | 0.034 | S | 0.011 | T | 0.011 | W | 0.011 | - | 0.011 | G | | |
| 58 | 84 | I | 0.600 | V | 0.353 | A | 0.012 | G | 0.012 | T | 0.012 | - | 0.012 | V | | |
| 59 | 84 | P | 0.847 | S | 0.106 | A | 0.012 | L | 0.012 | V | 0.012 | - | 0.012 | P | | |
| 60 | 85 | D | 0.488 | E | 0.325 | N | 0.047 | A | 0.035 | H | 0.023 | L | 0.023 | A | | 1 |
| 61 | 87 | R | 0.977 | D | 0.011 | - | 0.011 | | | | | | | R | | |
| 62 | 88 | F | 0.943 | I | 0.034 | L | 0.011 | R | 0.011 | | | | | F | | |
| 63 | 87 | S | 0.989 | F | 0.011 | | | | | | | | | S | | |
| 64 | 87 | G | 0.885 | A | 0.069 | S | 0.023 | V | 0.023 | | | | | G | | |
| 65 | 87 | S | 0.977 | G | 0.011 | Y | 0.011 | | | | | | | S | | |
| 66 | 86 | K | 0.430 | N | 0.186 | S | 0.186 | T | 0.081 | X | 0.070 | R | 0.035 | G | | 1 |
| 67 | 85 | S | 0.953 | T | 0.024 | K | 0.012 | L | 0.012 | | | | | S | | |
| 68 | 85 | G | 0.859 | S | 0.071 | A | 0.035 | D | 0.024 | Q | 0.012 | | | G | | |
| 69 | 85 | N | 0.434 | T | 0.318 | A | 0.129 | D | 0.036 | G | 0.024 | K | 0.024 | T | | |
| 70 | 85 | T | 0.529 | S | 0.341 | E | 0.082 | A | 0.024 | K | 0.024 | | | S | | |
| 71 | 85 | A | 0.847 | R | 0.082 | V | 0.059 | S | 0.012 | | | | | Y | | 1 |
| 72 | 85 | T | 0.447 | S | 0.424 | Y | 0.082 | A | 0.035 | I | 0.012 | | | S | | |
| 73 | 85 | L | 0.988 | S | 0.012 | | | | | | | | | L | | |
| 74 | 85 | T | 0.706 | A | 0.165 | G | 0.106 | I | 0.012 | L | 0.012 | | | T | | |
| 75 | 85 | I | 0.929 | V | 0.047 | A | 0.012 | L | 0.012 | | | | | I | | |
| 76 | 85 | S | 0.718 | T | 0.200 | N | 0.035 | I | 0.024 | G | 0.012 | R | 0.012 | S | | |
| 77 | 85 | G | 0.765 | R | 0.129 | S | 0.094 | E | 0.012 | | | | | R | | |
| 78 | 85 | L | 0.588 | V | 0.224 | T | 0.106 | A | 0.071 | G | 0.012 | | | M | | 1 |
| 79 | 85 | Q | 0.659 | E | 0.153 | R | 0.071 | K | 0.047 | L | 0.024 | A | 0.012 | E | | |
| 80 | 85 | A | 0.459 | S | 0.235 | T | 0.200 | V | 0.047 | P | 0.035 | N | 0.012 | A | | |
| 81 | 85 | E | 0.541 | G | 0.235 | M | 0.071 | D | 0.047 | L | 0.024 | N | 0.024 | E | | |
| 82 | 85 | D | 0.964 | N | 0.024 | E | 0.012 | | | | | | | D | | |
| 83 | 85 | E | 0.976 | D | 0.012 | T | 0.012 | | | | | | | A | | 1 |
| 84 | 85 | A | 0.941 | T | 0.035 | E | 0.012 | S | 0.012 | | | | | A | | |
| 85 | 85 | D | 0.859 | E | 0.082 | H | 0.024 | A | 0.012 | I | 0.012 | M | 0.012 | T | | 1 |
| 86 | 85 | Y | 0.976 | F | 0.012 | H | 0.012 | | | | | | | Y | | |
| 87 | 85 | Y | 0.894 | F | 0.106 | | | | | | | | | Y | | |
| 88 | 85 | C | 0.988 | H | 0.012 | | | | | | | | | C | | |
| 89 | 85 | Q | 0.482 | A | 0.153 | S | 0.141 | G | 0.094 | C | 0.059 | N | 0.035 | Q | L3 | |
| 90 | 85 | S | 0.388 | T | 0.271 | A | 0.212 | V | 0.118 | L | 0.012 | | | Q | L3 | |
| 91 | 85 | W | 0.576 | Y | 0.247 | A | 0.059 | F | 0.035 | R | 0.035 | D | 0.012 | R | L3 | |
| 92 | 84 | D | 0.606 | G | 0.095 | A | 0.071 | N | 0.061 | T | 0.048 | E | 0.024 | S | L3 | |

| Position (Light Chain) | Number of Observations | Observed Frequencies of 5 Most Abundant Amino Acids in Alignment of Human Sequences | | | | | | | | | | | | CAB1 Sequence | CDR | Mutated Residues |
|---|---|---|---|---|---|---|---|---|---|---|---|---|---|---|---|---|
| 93 | 84 | S | 0.405 | D | 0.179 | G | 0.107 | N | 0.095 | P | 0.071 | T | 0.060 | S | L3 | |
| 94 | 84 | S | 0.536 | G | 0.155 | N | 0.073 | R | 0.060 | D | 0.058 | T | 0.048 | Y | L3 | |
| 95 | 82 | S | 0.265 | L | 0.253 | G | 0.108 | N | 0.096 | T | 0.084 | A | 0.036 | P | L3 | |
| 95a | 60 | - | 0.268 | S | 0.183 | D | 0.159 | N | 0.110 | T | 0.073 | Q | 0.049 | L | L3 | |
| 95b | 40 | - | 0.512 | A | 0.098 | G | 0.098 | H | 0.085 | E | 0.049 | R | 0.037 | T | L3 | |
| 95c | 5 | - | 0.939 | P | 0.037 | A | 0.012 | G | 0.012 | | | | | | L3 | |
| 95d | 1 | - | 0.988 | G | 0.012 | | | | | | | | | | L3 | |
| 95e | 0 | - | 1.000 | | | | | | | | | | | | L3 | |
| 95f | 0 | - | 1.000 | | | | | | | | | | | | L3 | |
| 96 | 80 | V | 0.305 | G | 0.098 | P | 0.098 | W | 0.098 | A | 0.073 | N | 0.073 | | L3 | |
| 97 | 85 | V | 0.788 | I | 0.118 | L | 0.047 | M | 0.035 | G | 0.012 | | | | L3 | |
| 98 | 86 | F | 0.988 | V | 0.012 | | | | | | | | | F | | |
| 99 | 89 | G | 0.989 | F | 0.011 | | | | | | | | | G | | |
| 100 | 89 | G | 0.831 | T | 0.124 | A | 0.022 | S | 0.022 | | | | | A | | 1 |
| 101 | 89 | G | 1.000 | | | | | | | | | | | G | | |
| 102 | 89 | T | 0.989 | G | 0.011 | | | | | | | | | T | | |
| 103 | 88 | K | 0.739 | N | 0.091 | R | 0.068 | Q | 0.034 | T | 0.034 | E | 0.011 | K | | |
| 104 | 87 | L | 0.667 | V | 0.322 | Q | 0.011 | | | | | | | L | | |
| 105 | 87 | T | 0.954 | S | 0.023 | I | 0.011 | L | 0.011 | | | | | E | | 1 |
| 106 | 85 | V | 0.988 | T | 0.012 | | | | | | | | | L | | 1 |
| 106a | 84 | L | 0.952 | V | 0.024 | P | 0.012 | Q | 0.012 | | | | | K | | 1 |
| 107 | 78 | G | 0.782 | S | 0.103 | R | 0.090 | C | 0.013 | L | 0.013 | | | R | | 1 |
| 108 | 46 | Q | 0.957 | P | 0.022 | R | 0.022 | | | | | | | A | | 1 |
| 109 | 46 | P | 0.957 | K | 0.022 | Q | 0.022 | | | | | | | A | | 1 |

These frequencies were compared with the actual amino acid sequence of CAB1. Based on these comparisons, 33 positions that fulfilled the following criteria were identified: 1) the position is not part of a CDR as defined by the Kabat nomenclature; 2) the amino acid found in CAB1-scFv is observed in the homologous position in less than 10% of human antibodies; and 3) the position is not one of the last 6 amino acids in the light chain of scFv. These 33 positions were then used in the combinatorial mutagenesis methods of the present invention.

Mutagenic oligonucleotides were synthesized for each of the 33 positions such that the targeted position would be changed from the amino acid in CAB1-scFv to the most abundant amino acid in the homologous position of a human antibody. Figure 10 provides the sequence of CAB1-scFv, the CDRs, and the mutations that were chosen for combinatorial mutagenesis.

**C. Construction of Library NA05**

Table 6 provides the sequences of 33 mutagenic oligonucleotides that were used to generate the combinatorial library designated as "NA05."

**Table 6. Mutagenic Primers Used to Generate NA05**

| pos. (pME27) | CAB1 | Consensus aa (VH) | Primer Name | QuikChange® Oligonucleotide Primer Sequence | SEQ ID NO: |
|---|---|---|---|---|---|
| 3 | K | Q | nsa147.1fp | CGGCCATGGCCCAGGTGCAGCTGCAGCAGTCTGGGGC | 54 |
| 13 | R | K | nsa147.2fp | CTGGGGCAGAACTTGTGAAATCAGGGACCTCAGTCAA | 55 |
| 14 | S | P | nsa147.3fp | GGGCAGAACTTGTGAGGCCGGGGACCTCAGTCAAGTT | 56 |
| 16 | T | G | nsa147.4fp | AACTTGTGAGGTCAGGGGGCTCAGTCAAGTTGTCCTG | 57 |
| 28 | N | T | nsa147.5fp | GCACAGCTTCTGGCTTCACCATTAAAGACTCCTATAT | 58 |
| 29 | I | F | nsa147.6fp | CAGCTTCTGGCTTCAACTTTAAAGACTCCTATATGCA | 59 |
| 30 | K | S | nsa147.7fp | CTTCTGGCTTCAACATTAGCGACTCCTATATGCACTG | 60 |
| 37 | L | V | nsa147.8fp | ACTCCTATATGCACTGGGTGAGGCAGGGGCCTGAACA | 61 |
| 40 | G | A | nsa147.9fp | TGCACTGGTTGAGGCAGGCGCCTGAACAGGGCCTGGA | 62 |
| 42 | E | G | nsa147.10fp | GGTTGAGGCAGGGGCCTGGCCAGGGCCTGGAGTGGAT | 63 |
| 67 | K | R | nsa147.11fp | CCCCGAAGTTCCAGGGCCGTGCCACTTTTACTACAGA | 64 |
| 68 | A | F | nsa147.12fp | CGAAGTTCCAGGGCAAGTTCACTTTTACTACAGACAC | 65 |
| 70 | F | I | nsa147.13fp | TCCAGGGCAAGGCCACTATTACTACAGACACATCCTC | 66 |
| 72 | T | R | nsa147.14fp | GCAAGGCCACTTTTACTCGCGACACATCCTCCAACAC | 67 |
| 76 | S | K | nsa147.15fp | TTACTACAGACACATCCAAAAACACAGCCTACCTGCA | 68 |
| 97 | N | A | nsa147.16fp | CTGCCGTCTATTATTGTGCGGAGGGGACTCCGACTGG | 69 |
| 98 | E | R | nsa147.17fp | CCGTCTATTATTGTAATCGCGGGACTCCGACTGGGCC | 70 |

| pos. (pME27) | CAB1 | Consensus aa (VH) | Primer Name | QuikChange® Oligonucleotide Primer Sequence | SEQ ID NO: |
|---|---|---|---|---|---|
| 136 | E | Q | nsa147.18fp | CTGGCGGTGGCGGATCACAGAATGTGCTCACCCAGTC | 71 |
| 137 | N | S | nsa147.19fp | GCGGTGGCGGATCAGAAAGCGTGCTCACCCAGTCTCC | 72 |
| 142 | S | P | nsa147.20fp | GAAAATGTGCTCACCCAGCCGCCAGCAATCATGTCTGC | 73 |
| 144 | A | S | nsa147.21fp | TGCTCACCCAGTCTCCAAGCATCATGTCTGCATCTCC | 74 |
| 146 | M | V | nsa147.22fp | CCCAGTCTCCAGCAATCGTGTCTGCATCTCCAGGGGA | 75 |
| 152 | E | Q | nsa147.23fp | TGTCTGCATCTCCAGGGCAGAAGGTCACCATAACCTG | 76 |
| 153 | K | T | nsa147.24fp | CTGCATCTCCAGGGGAGACCGTCACCATAACCTGCAG | 77 |
| 170 | F | Y | nsa147.25fp | TAAGTTACATGCACTGGTACCAGCAGAAGCCAGGCAC | 78 |
| 181 | W | V | nsa147.26fp | GCACTTCTCCCAAACTCGTGATTTATAGCACATCCAA | 79 |
| 194 | A | D | nsa147.27fp | TGGCTTCTGGAGTCCCTGATCGCTTCAGTGGCAGTGG | 80 |
| 200 | G | K | nsa147.28fp | CTCGCTTCAGTGGCAGTAAATCTGGGACCTCTTACTC | 81 |
| 205 | Y | A | nsa147.29fp | GTGGATCTGGGACCTCTGCGTCTCTCACAATCAGCCG | 82 |
| 212 | M | L | nsa147.30fp | CTCTCACAATCAGCCGACTGGAGGCTGAAGATGCTGC | 83 |
| 217 | A | E | nsa147.31fp | GAATGGAGGCTGAAGATGAAGCCACTTATTACTGCCA | 84 |
| 219 | T | D | nsa147.32fp | AGGCTGAAGATGCTGCCGATTATTACTGCCAGCAAAG | 85 |
| 234 | A | G | nsa147.33fp | ACCCACTCACGTTCGGTGGCGGCACCAAGCTGGAGCT | 86 |

The QuikChange® multi site-directed mutagenesis kit (QCMS; Stratagene Catalog # 200514) was used to construct the combinatorial library NA05 using the above 33 mutagenic primers. The primers were designed so that they had 17 bases flanking each side of the codon of interest based on the template plasmid pME27.1. The codon of interest was changed to encode the appropriate consensus amino acid using an *E. coli* codon usage table (indicated in the above Table by underlining). All primers were designed to anneal to the same strand of the template DNA (*i.e.*, all were forward primers). The QCMS reaction was carried out as described in the QCMS manual with the exception of the primer concentration used, as approximately 3 ng of each primer were used in the experiments described herein, while the QCMS manual recommends using 50ng of each primer in the reaction. However, it is not intended that the present invention be limited to any particular primer concentration as other primer concentrations find use in the present invention.

In particular, the reaction used in the present Example contained 50-100 ng template plasmid (pME27.1; 5178bp), 1 μl of primer mix (10 μM stock of all primers combined containing 0.3 μM each primer), 1 μl dNTPs (QCMS kit), 2.5 μl 10x QCMS reaction buffer, 18.5 μl deoinized water, and 1 μl enzyme blend (QCMS kit), for a total volume of 25 μl. The thermocycling program was set for 1 cycle at 95° for 1 min., followed by 30 cycles of 95°C for 1 min., 55°C for 1 min., and 65°C for 10 minutes. *Dpn*I digestion was performed by adding 1 μl *Dpn*I (provided in the QCMS kit), incubation at 37°C for 2 hours, addition of another 1 μl *Dpn*I, and incubation at 37°C for an additional 2 hours. Then, 1 μl of the reaction was transformed into 50 μl of TOP10 electrocompetent cells from Invitrogen. Then, 250 μl of SOC was added after electroporation, followed by a 1 hr incubation with shaking at 37°C. Thereafter, 10-50 μl of the transformation mix was plated on LA plates with 5ppm chloramphenicol (CMP) or LA plates with 5ppm CMP and 0.1ppm of cefotaxime (CTX) for selection of active BLA clones. The active BLA clones from the CMP + CTX plates were used for screening, whereas the random library clones from the CMP plates were sequenced to assess the quality of the library.

Sixteen randomly chosen clones were sequenced. The clones contained different combinations of 1 to 7 mutations.

### D. Screen for Improved Expression

It was observed that when TOP10/pME27.1 is cultured in LB medium at 37°C, the concentration of intact fusion protein peaks after one day and most of the fusion protein is degraded by host proteases after 3 days of culture. Degradation appears to occur mainly in the scFv portion of the CAB 1 fusion protein, as the cultures contain significant amounts of free BLA after 3 days, which can be detected by Western blotting, or nitrocefin (Oxoid) activity assay. Thus, library NA05 was screened to detect variants of CAB1-scFv that would resist degradation by host proteases over 3 days of culture at 37°C.

To conduct the screen, library NA05 was plated onto agar plates with LA medium containing 5 mg/l chloramphenicol and 0.1 mg/l cefotaxime (Sigma). Then, 910 colonies were transferred into a total of 10 96-well plates containing 100 ul/well of LA medium containing 5 mg/l chloramphenicol and 0.1 mg/l cefotaxime. Four wells in each plate were inoculated with TOP10/pME27.1 as control and one well per plate was left as a blank. The plates were grown overnight at 37°C. The next day, the cultures were used to inoculate fresh plates (production plates) containing 100 ul of the same medium using a transfer stamping tool and glycerol was added to the master plates which were stored at -70°C, as known in the art. The production plates were incubated in a humidified shaker at 37°C for 3 days. Then, 100 ul/well of B-PER (Pierce) were added to the production plate to release protein from the cells.

Samples from the production plate were diluted 100-fold in PBST (PBS containing 0.125% Tween®-20) and BLA activity was measured by transferring 20 ul diluted lysate into 180 ul of nitrocephin assay buffer (0.1 mg/ml nitrocephin in 50 mM PBS buffer containing 0.125% octylglucopyranoside (Sigma)), and the BLA activity was determined at 490 nm using a Spectramax plus plate reader (Molecular Devices).

Binding to CEA (carcinoembryonic antigen; Biodesign) was measured using the following procedure: 96-well plates were coated with 100 ul per well of 5 ug/ml of CEA in 50 mM carbonate buffer pH 9.6 and incubated overnight at 4°C. The plates were washed with PBST and blocked for 1-2 hours with 300 ul of casein (Pierce) at 25°C. Then, 100 ul of sample from the production plate diluted 100-1000 fold was added to the CEA coated plate and the plates were incubated for 2 h at room temperature. Subsequently, the plates were washed four times with PBST, 200 ul nitrocefin assay buffer were added, and the BLA activity was measured as described above.

The BLA activity determined by the CEA-binding assay and the total BLA activity found in the lysate plates were compared in order to identify variants that showed high levels of total BLA activity and high levels of CEA-binding activities.

The "winners" (*i.e.*, variants with the highest total BLA activity and CEA-binding activity) were confirmed by testing 4 replicates in a similar protocol. The variants were cultured in 2 ml of LB containing 5 mg/l chloramphenicol and 0.1 mg/l cefotaxime for 3 days. Protein was released from the cells using B-PER reagent. The binding assay was performed as described above, but different dilutions of culture lysate were tested for each variant. Thus, a binding curve which provides a measure of the binding affinity of the variant for the target CEA was produced. The binding curve obtained is shown in Figure 11. The culture supernatants were also analyzed by SDS-PAGE. Variant NA05.6 was found to contain a pronounced band at an approximate molecular weight of 65 kD that was significantly weaker for the parent molecule and for most of the other tested isolates. Table 7 provides a list of 6 variants with the largest improvement in stability.

**Table 7. Sequence of Six Variants**

| Clone | Mutations |
|---|---|
| NA05.6 | R13K, T16G, W181V |
| NA05.8 | R13K, F170Y, A234G |
| NA05.9 | K3Q, S14P, L37V, E42G, E136Q, M146V, W181V, A234G |
| NA05.10 | K3Q, L37V, P170Y, W181V |
| NA05.12 | K3Q, S14P, L37V, M146V |
| NA05.15 | M146V, F170Y, A194D |

### E. Construction of Library NA06

Clone NA05.6 was chosen as the best variant and was used as the template for a second round of combinatorial mutagenesis. A subset of the same mutagenic primers that had been used to generate library NA05 were used to generate combinatorial variants with the following mutations: K3Q, L37V, E42G, E136Q, M146V, F170Y, A194D, A234G, which had been identified in other winners from library NA05. The primer encoding mutation S 14P was not used, as its sequence overlapped with mutations R13K and T16G that are present in NA05.6. A combinatorial library (designated "NA06") was constructed using QCMS method as described above. The template used was pNA05.6 and 1 μl of primer mix (10 μM stock of all primers combined containing 1.25 μM each primer) were used.

**F. Screening of Library NA06**

The screen was performed as described above with the following modifications described below. In these experiments, 291 variants were screened using three 96-well plates. For each well, a 10 μl sample from the lysate plates was added to 180 μl of 10 μg/ml thermolysin (Sigma) in 50 mM imidazole buffer pH 7.0 containing 0.005% Tween®-20 and 10 mM calcium chloride. This mixture was incubated for 1 h at 37°C, to hydrolyze unstable variants of NA05.6. This protease-treated sample was used to perform the CEA-binding assay as described above. Promising variants were cultured in 2 ml medium as described above and binding curves were obtained for samples after thermolysin treatments. Figure 12 provides binding curves for selected clones. As indicated in the Figure, a number of variants retain much more binding activity after thermolysin incubation than the parent NA05.6. Table 8 provides 6 variants that are significantly more resistant to protease than NA05.6. All 6 of these variants have the mutation L37V which was rare in randomly chosen clones from the same library. Further testing showed that variant NA06.6 had the highest level of total BLA activity and the highest protease resistance of all the tested variants.

**Table 8. Six Variants More Protease Resistant than NA05.6**

| Clone | Mutations |
|---|---|
| NAO6.2 | R13K, T16G, W181V, L37V, E42G, A194D |
| NA06.4 | R13K, T16G, W181V, L37V, M146V |
| NA06.6 | R13K, T16G, W181V, L37V, M146V, K3Q |
| NA06.10 | R13K, T16G, W181V, L37V, M146V, A194D |
| NA06.11 | R13K, T16G, W181V, L37V, K3Q, A194D |
| NA06.12 | R13K, T16G, W181V, L37V, E136Q |

## Claims

1. A method for combinatorial consensus mutagenesis comprising the steps:

   a) identifying a starting gene of interest;
   b) identifying at least two homologs of said starting gene of interest;
   c) generating a multiple sequence alignment of said at least two homologs of said starting gene of interest and said starting gene of interest;
   d) using said multiple sequence alignment to identify consensus mutations and produce a combinatorial consensus library containing multiple combinations of consensus mutations;
   e) screening said combinatorial consensus library to identify initial hits;
   f) sequencing said initial hits to provide sequenced initial hits;
   g) identifying improving mutations in said sequenced initial hits, wherein said improving mutations are more common among said initial hits as compared to said combinatorial consensus library;
   h) using said sequenced initial hits to generate an enhanced combinatorial consensus library; and
   i) screening said enhanced combinatorial consensus library to identify at least one improved hit,

   wherein said screening e) and i) comprises determining the stability of said initial hit in at least one assay selected from the group consisting of protease resistance assays, thermostability assays, denaturation assays, and functional assays.

2. The method of any one of the preceding claims, further comprising the step of sequencing said improved hits.

3. The method of any one of the preceding claims, wherein said improved hits are stabilized variants of said starting gene.

4. The method of any one of the preceding claims, wherein said improved hits comprise performance enhancing mutations.

5. The method of any one of the preceding claims, further comprising the step of analyzing the correlation between sequence and stability of at least two of said initial hits.

6. The method of any one of the preceding claims, further comprising the step of analyzing the correlation between sequence and stability of at least two of said sequenced improved hits.

7. The method of any one of the preceding claims, wherein said multiple sequence alignment identifies amino acids that occur frequently in said homologs but are not part of a consensus sequence.

8. The method of any one of the preceding claims, wherein said steps are repeated at least once.

9. A stabilized variant of beta-lactamase, wherein said stabilized variant comprises at least one amino acid change selected from the group consisting of V11I, V251I, R91K, Q95E, A153S, N232R, S247T, V293L, V294I, T342K, I262V, and V284I.

10. A stabilized variant of carcinoembryonic antigen binder, wherein said stabilized variant comprises at least one amino acid change selected from the group consisting of K3Q, L37V, E42G, E136Q, M146V, F170Y, A194D, and A234G.

11. A stabilized single chain fragment variable region (scFV), wherein said stabilized scFV variant comprises at least one amino acid change selected from the group consisting of K3Q, L37V, E42G, E136Q, M146V, F170Y, A194D, and A234G.

**Patentansprüche**

1. Verfahren zur kombinatorischen Konsensus-Mutagenese, das die folgenden Schritte umfasst:

   a) Identifizieren eines Ausgangsgens von Interesse;
   b) Identifizieren von zumindest zwei Homologen des Ausgangsgens von Interesse;
   c) Erstellen eines Mehrfach-Sequenzabgleichs der zumindest zwei Homologen des Ausgangsgens von Interesse sowie des Ausgangsgens von Interesse;
   d) Verwenden des Mehrfach-Sequenzabgleichs zur Identifizierung von Konsensus-Mutationen und zur Erstellung von kombinatorischen Konsensus-Bibliotheken, die Mehrfach-Kombinationen von Konsensus-Mutationen enthalten;
   e) Screenen der kombinatorischen Konsensus-Bibliothek, um erste Treffer zu identifizieren;
   f) Sequenzieren der ersten Treffer, um sequenzierte erste Treffer bereitzustellen;
   g) Identifizieren von sich verbessernden Mutationen in den sequenzierten ersten Treffern, worin die sich verbessernden Mutationen unter den ersten Treffern im Vergleich mit der kombinatorischen Konsensus-Bibliothek häufiger vorkommen;
   h) Verwenden der sequenzierten ersten Treffer, um eine verbesserte kombinatorische Konsensus-Bibliothek zu erstellen; und
   i) Screenen der verbesserten kombinatorischen Konsensus-Bibliothek, um zumindest einen verbesserten Treffer zu identifizieren,

   worin das Screenen e) und i) das Bestimmen der Stabilität des ersten Treffers in zumindest einem Test umfasst, der aus der aus Protease-Resistenztests, Thermostabilitätstest, Denaturierungstests und funktionellen Tests bestehenden Gruppe ausgewählt ist.

2. Verfahren nach einem der vorangegangenen Ansprüche, das weiters den Schritt des Sequenzierens der verbesserten Treffer umfasst.

3. Verfahren nach einem der vorangegangenen Ansprüche, worin die verbesserten Treffer stabilisierte Varianten des Ausgangsgens sind.

4. Verfahren nach einem der vorangegangenen Ansprüche, worin die verbesserten Treffer leistungsverbessernde Mutationen umfassen.

5. Verfahren nach einem der vorangegangenen Ansprüche, das weiters den Schritt des Analysierens der Korrelation zwischen Sequenz und Stabilität von zumindest zwei der ersten Treffer umfasst.

6. Verfahren nach einem der vorangegangenen Ansprüche, das weiters den Schritt des Analysierens der Korrelation zwischen Sequenz und Stabilität von zumindest zwei der sequenzierten verbesserten Treffer umfasst.

7. Verfahren nach einem der vorangegangenen Ansprüche, worin der Mehrfach-Sequenzabgleich Aminosäuren identifiziert, die häufig in den Homologen auftreten, jedoch nicht Teil einer Konsensus-Sequenz sind.

8. Verfahren nach einem der vorangegangenen Ansprüche, worin die Schritte zumindest einmal wiederholt werden.

9. Stabilisierte Variante von β-Lactamase, worin die stabilisierte Variante zumindest eine Aminosäureänderung umfasst, die aus der aus V11I, V251I, R91K, Q95E, A153S, N232R, S247T, V293L, V2941, T342K, I262V und V2841 bestehenden Gruppe ausgewählt ist.

10. Stabilisierte Variante eines Carcinoembryo-Antigen-Binders, worin die stabilisierte Variante zumindest eine Aminosäureänderung umfasst, die aus der aus K3Q, L37V, E42G, E136Q, M146V, F170Y, A194D und A234G bestehenden Gruppe ausgewählt ist.

11. Stabilisierte variable Region eines einkettigen Fragments (scFV), worin die stabilisierte scFV-Variante zumindest eine Aminosäureänderung umfasst, die aus der aus K3Q, L37V, E42G, E136Q, M146V, F170Y, A194D und A234G bestehenden Gruppe ausgewählt ist.

**Revendications**

1. Méthode pour une mutagénèse de consensus combinatoire comprenant les étapes:

   a) identifier un gène de début d'intérêt;
   b) identifier au moins deux homologues dudit gène de début d'intérêt;
   c) produire un alignement de séquences multiples desdits au moins deux homologues dudit gène de début d'intérêt et dudit gène de début d'intérêt;
   d) utiliser ledit alignement de séquences multiples pour identifier des mutations de consensus et produire une banque de consensus combinatoires contenant des combinaisons multiples de mutations de consensus;
   e) cribler ladite banque de consensus combinatoires pour identifier des têtes initiales;
   f) séquencer lesdites têtes initiales pour réaliser des têtes initiales séquencées;
   g) identifier l'amélioration des mutations dans lesdites têtes initiales séquencées, où lesdites mutations améliorées sont plus communes parmi lesdites têtes initiales en comparaison avec ladite banque de consensus combinatoires;
   h) utiliser lesdites têtes initiales séquencées pour produire une banque de consensus combinatoires augmentée; et
   i) cribler ladite banque de consensus combinatoires augmentée pour identifier au moins une tête améliorée,

   où ledit criblage e) et i) comprend la détermination de la stabilité de ladite tête initiale dans au moins un dosage sélectionné dans le groupe consistant en dosages de la résistance à la protéase, dosages de thermostabilité, dosages de dénaturation et dosages fonctionnels.

2. Méthode selon l'une quelconque des revendications précédentes, comprenant en outre l'étape consistant à séquencer lesdites têtes améliorées.

3. Méthode selon l'une quelconque des revendications précédentes, dans laquelle lesdites têtes améliorées sont des variantes stabilisées dudit gène de début.

4. Méthode selon l'une quelconque des revendications précédentes, dans laquelle lesdites têtes améliorées comprennent des mutations augmentant la performance.

5. Méthode selon l'une quelconque des revendications précédentes, comprenant en outre l'étape consistant à analyser la corrélation entre la séquence et la stabilité d'au moins deux desdites têtes initiales.

6. Méthode selon l'une quelconque des revendications précédentes, comprenant en outre l'étape consistant à analyser la corrélation entre la séquence et la stabilité d'au moins deux desdites têtes séquencées améliorées.

7. Méthode selon l'une quelconque des revendications précédentes, dans laquelle l'alignement de séquences multiples identifie des acides aminés qui se produisent fréquemment dans lesdits homologues mais qui ne font pas partie d'une séquence de consensus.

8. Méthode selon l'une quelconque des revendications précédentes, dans laquelle lesdites étapes sont répétées au moins une fois.

9. Variante stabilisée de la béta-lactamase, où ladite variante stabilisée comprend au moins un changement d'acide aminé sélectionné dans le groupe consistant en V11I, V251I, R91K, Q95E, A153S, N232R, S247T, V293L, V294I, T342K, I262V et V284I.

10. Variante stabilisée d'un liant d'antigène carcinoembryonnaire, où ladite variante stabilisée comprend au moins un changement d'acide aminé sélectionné dans le groupe consistant en K3Q, L37V, E42Q, E136Q, M146V, F170Y, A194D et A234G.

11. Région variable de fragment à chaîne unique stabilisée (scFV), dans laquelle ladite variante scFV stabilisée comprend au moins un changement d'acide aminé sélectionné dans le groupe consistant en K3Q, L37V, E42G, E136Q, M146V, F170Y, A194D et A234G.

FIGURE 1.

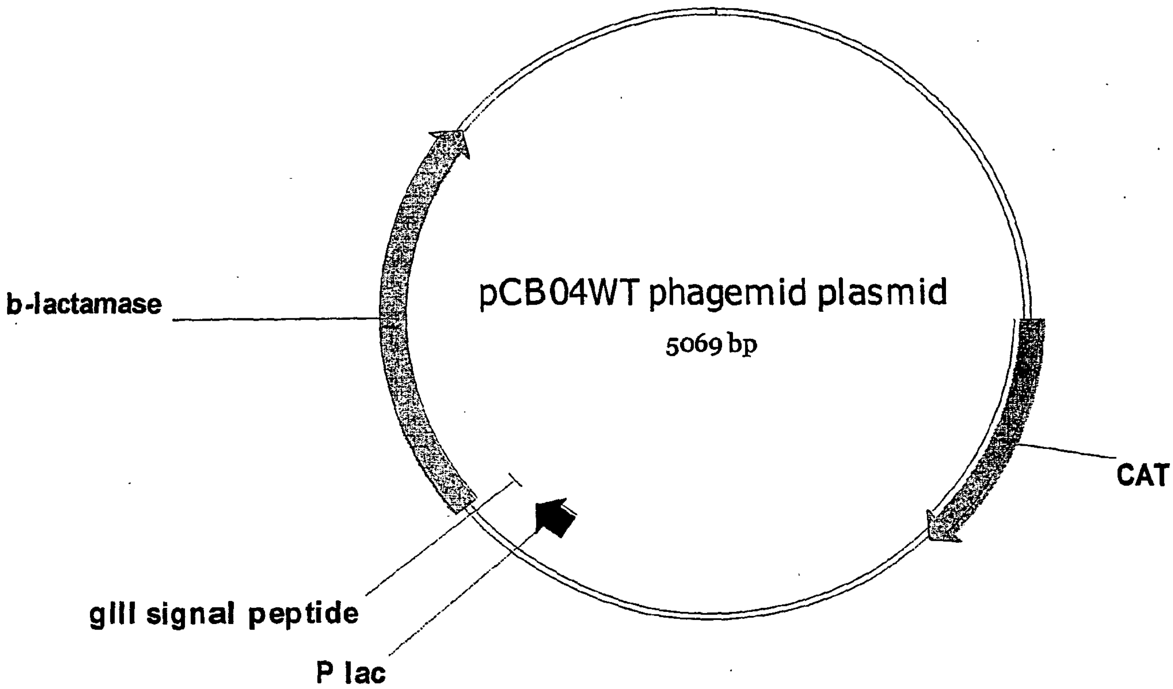
pCB04WT phagemid plasmid
5069 bp
b-lactamase
CAT
gIII signal peptide
P lac

## FIGURE 2

gagtcgtattacaattcactggccgtcgttttacaacgtcgtgactgggaaaaccctggcgttacccaacttaatcgccttgcagcacatccccctttcgccagctggc
gtaatagcgaagaggcccgcaccgatcgcccttcccaacagttgcgcagcctgaatggcgaatggacgcgccctgtagcggcgcattaagcgcggcgggtgtg
gtggttacgcgcagcgtgaccgctacacttgccagcgccctagcgcccgctcctttcgctttcttcccttcctttctcgccacgttcgccggctttccccgtcaagctct
aaatcgggggctccctttagggttccgatttagtgctttacggcacctcgaccccaaaaaacttgattagggtgatggttcacgtagtgggccatcgccctgatagac
ggtttttcgccctttgacgttggagtccacgttctttaatagtggactcttgttccaaactggaacaacactcaaccctatctcggtctattcttttgatttataagggattttg
ccgatttcggcctattggttaaaaaatgagctgatttaacaaaaatttaacgcgaattttaacaaaatattaacgcttacaatttcctgatgcggtattttctccttacgcatc
tgtgcggtatttcacaccgcatatggtgcactctcagtacaatctgctctgatgccgcatagttaagccagccccgacacccgccaacacccgctgacgcgccctga
cgggcttgtctgctcccggcatccgcttacagacaagctgtgaccgtctccgggagctgcatgtgtcagaggttttcaccgtcatcaccgaaacgcgcgagacgaa
agggcctcgtgatacgcctatttttataggttaatgtcatgataataatggtttcttagacgtcaggtggcacttttcggggaaatgtgcgcggaacccctatttgtttattt
ttctaaatacattcaaatatgtatccgctcatgagacaataaccctgtggcagcatcacccgacgcactttgcgccgaataaatacctgtgacggaagatcacttcgca
gaataaataaatcctggtgtccctgttgataccgggaagccctgggccaacttttggcgaaaatgagacgttgatcggcacgtaagaggttccaactttcaccataat
gaaataagatcactaccgggcgtattttttgagttatcgagattttcaggagctaaggaagctaaaatggagaaaaaaatcactggatataccaccgttgatatatccc
aatggcatcgtaaagaacattttgaggcatttcagtcagttgctcaatgtacctataaccagaccgttcagctggatattacggcctttttaaagaccgtaaagaaaaat
aagcacaagttttatccggcctttattcacattcttgcccgcctgatgaatgctcatccggaattccgtatggcaatgaaagacggtgagctggtgatatgggatagtgt
tcacccttgttacaccgttttccatgagcaaactgaaacgttttcatcgctctggagtgaataccacgacgatttccggcagtttctacacatatattcgcaagatgtggc
gtgttacggtgaaaacctggcctatttccctaaagggtttattgagaatatgtttttcgtctcagccaatccctgggtgagtttcaccagttttgatttaaacgtggccaata
tggacaacttcttcgcccccgttttcaccatgggcaaatattatacgcaaggcgacaaggtgctgatgccgctggcgattcaggttcatcatgccgtctgtgatggctt
ccatgtcggcagaatgcttaatgaattacaacagtactgcgatgagtggcagggcggggcgtaaagacagatcgctgagataggtgcctcactgattaagcattgg
taactgtcagaccaagtttactcatatatactttagattgatttaaaacttcatttttaatttaaaaggatctaggtgaagatcctttttgataatctcatgaccaaaatccctta
acgtgagttttcgttccactgagcgtcagaccccgtagaaaagatcaaaggatcttcttgagatcctttttttctgcgcgtaatctgctgcttgcaaacaaaaaaaccac
cgctaccagcggtggtttgtttgccggatcaagagctaccaactctttttccgaaggtaactggcttcagcagagcgcagataccaaatactgttcttctagtgtagcc
gtagttaggccaccacttcaagaactctgtagcaccgcctacatacctcgctctgctaatcctgttaccagtggctgctgccagtggcgataagtcgtgtcttaccgg
gttggactcaagacgatagttaccggataaggcgcagcggtcgggctgaacggggggttcgtgcacacagcccagcttggagcgaacgacctacaccgaactg
agatacctacagcgtgagctatgagaaagcgccacgcttcccgaagggagaaaggcggacaggtatccggtaagcggcagggtcggaacaggagagcgcac
gagggagcttccagggggaaacgcctggtatctttatagtcctgtcgggtttcgccacctctgacttgagcgtcgatttttgtgatgctcgtcaggggggcggagcct
atggaaaaacgccagcaacgcggcctttttacggttcctggccttttgctggccttttgctcacatgttctttcctgcgttatcccctgattctgtggataaccgtattacc
gcctttgagtgagctgataccgctcgccgcagccgaacgaccgagcgcagcgagtcagtgagcgaggaagcggaagagcgcccaatacgcaaaccgcctctc
cccgcgcgttggccgattcattaatgcagctggcacgacaggtttcccgactggaaagcgggcagtgagcgcaacgcaattaatgtgagttagctcactcattagg
caccccaggctttacactttatgcttccggctcgtatgttgtgtggaattgtgagcggataacaatttcacacaggaaacagctatgaccatgattacgccaagctattt
aggtgacactatagaatactcaagcttaagaaggagatatacatatgaaaaagttactattcgcaattccactagtcgttcctttctattctcactctacgccagtgtcag
aaaaacagctggcggaggtggtcgcgaatacgattaccccgctgatgaaagcacagagtgttccaggcatggcggtggccgttatttatcagggaaaaccgcact
attacacatttggcaaggccgatatcgcggcgaataaacccgttacgcctcagaccctgttcgagctgggttctataagtaaaaccttcaccggcgttttaggtgggg
atgccattgctcgcggtgaaatttcgctggacgatgcggtgaccagatactggccacagctgacgggcaagcagtggcagggtattcgtatgctggatctcgccac
ctacaccgctggcggcctgccgctacaggtaccggatgaggtcacggataacgcctccctgctgcgcttttatcaaaactggcagccgcagtggaagcctggcac
aacgcgtctttacgccaacgccagcatcggtctttttggtgcgctggcggtcaaaccttctggcatgccctatgagcaggccatgacgacgcgggtccttaagccg
ctcaagctggaccatacctggattaacgtgccgaaagcggaagaggcgcattacgcctggggctatcgtgacggtaaagcggtgcgcgtttcgccgggtatgctg
gatgcacaagcctatggcgtgaaaaccaacgtgcaggatatggcgaactgggtcatggccaacatggccccggagaacgttgctgatgcctcacttaagcaggg
catcgcgctggcgcagtcgcgctactggcgtatcgggtcaatgtatcagggtctcggctgggagatgctcaactggcccgtggaggcaaacacggtggtcgagg
gcagcgacagtaaggtagcgctagcgccgttgcccgtggcagaagtgaatccaccggctcccccggtcaaagcgtcctgggtccataaaactggctctactggc
gggtttggatcctacgtggcctttattcctgaaaagcagatcggtattgtgatgctcgcgaatacaagctatccgaacccggctcgagttgaggcggcataccatatc
ctagaggcgctacagggtggcggatcggccgaaactgttgaacaccaccatcatcaccattagcccggggctgctcactacactctaggtggaggttcaccattc
gtttgtgaatatcaaggccaatcgtctgacctgcctcaacctcctgtcaatgctggcggcggctctggtggtggttctggtggcggctctgagggtggtggctctgag
ggtggcggttctgagggtggcggctctgagggaggcggttccggtggtggctctggttccggtgattttgattatgaaaagatggcaaacgctaataagggggcta
tgaccgaaaatgccgatgaaaacgcgctacagtctgacgctaaaggcaaacttgattctgtcgctactgattacggtgctgctatcgatggtttcattggtgacgtttc
cggccttgctaatggtaatggtgctactggtgattttgctggctctaattcccaaatggctcaagtcggtgacggtgataattcacctttaatgaataatttccgtcaatatt
taccttccctccctcaatcggttgaatgtcgcccttttgtctttagcgctggtaaaccatatgaattttctattgattgtgacaaaataaacttattccgtggtgtctttgcgttt
cttttatatgttgccacctttatgtatgtattttctacgtttgctaacatactgcgtaataaggagtcttaataagaattcgccctatagt (SEQ ID NO:1)

FIGURE 3.

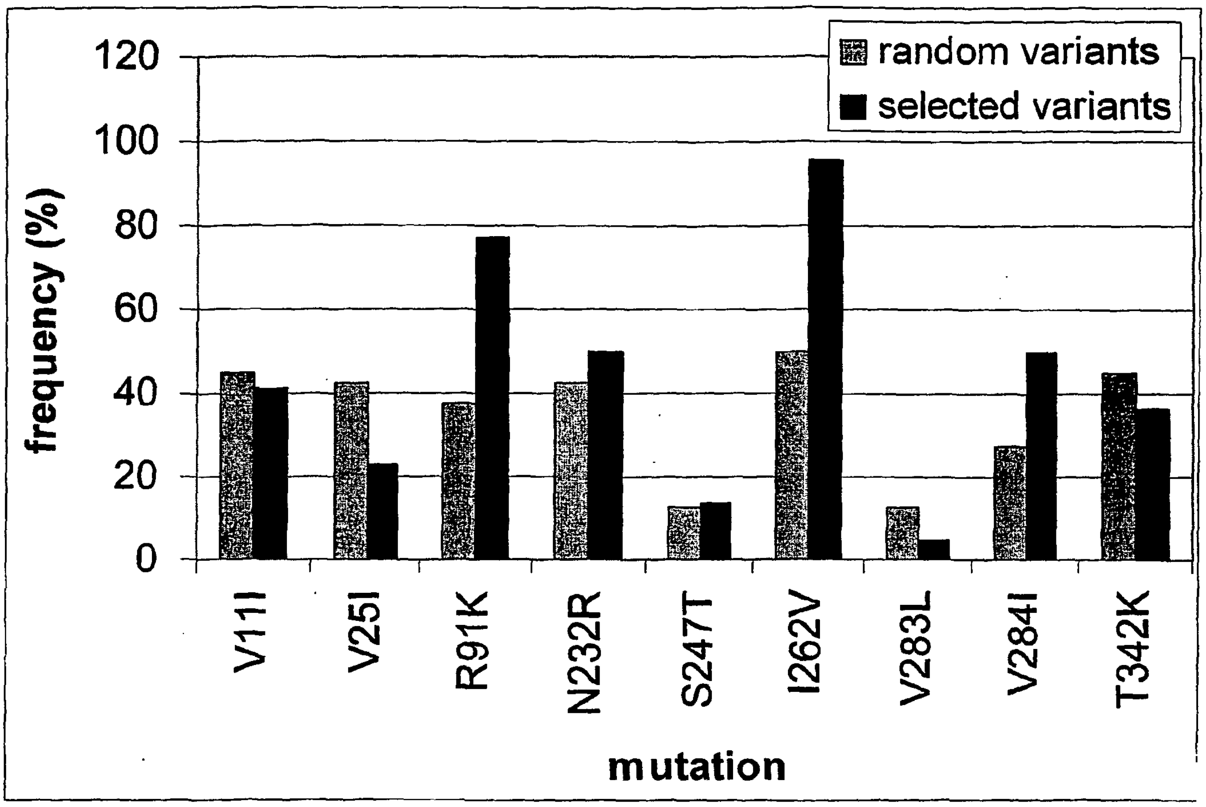
random variants
selected variants
120
100
80
60
40
20
0
frequency (%)
V11I
V25I
R91K
N232R
S247T
I262V
V283L
V284I
T342K
mutation

FIGURE 4.

| aa change | T1A | S4T | V11I | A13D | V25I | Y41F | F43Y | I65V | R91K | Q95E | R105T | M106L | S130A | A153S | Y170F | A194S | A208P | Q219E | T225S | N226T | V227A | N232R | S247T | I262V | V283L | V284I | P295A | I334L | T342K | const. C |
|---|---|---|---|---|---|---|---|---|---|---|---|---|---|---|---|---|---|---|---|---|---|---|---|---|---|---|---|---|---|---|
| parameter | 0.100 | 0.050 | 0.375 | 0.036 | 0.194 | -0.232 | -0.092 | -0.219 | 0.367 | 0.756 | -0.223 | -0.175 | -0.102 | 0.669 | -0.256 | 0.141 | -0.021 | -0.446 | 0.172 | -0.198 | -0.311 | 0.442 | 0.177 | 0.294 | 0.334 | 0.378 | -0.299 | -0.062 | 0.107 | 0.471 |
| # occur. | 0 | 4 | 4 | 24 | 2 | 7 | 5 | 20 | 2 | 9 | 2 | 3 | 14 | 10 | 4 | 4 | 8 | 8 | 3 | 8 | 6 | 6 | 4 | 15 | 3 | 19 | 5 | 6 | 9 | |
| rank | | 14 | 5 | 15 | 9 | 24 | 18 | 22 | 6 | 1 | 23 | 20 | 19 | 2 | 25 | 12 | 16 | 28 | 11 | 21 | 27 | 3 | 10 | 8 | 7 | 4 | 26 | 17 | 13 | |

FIGURE 5.

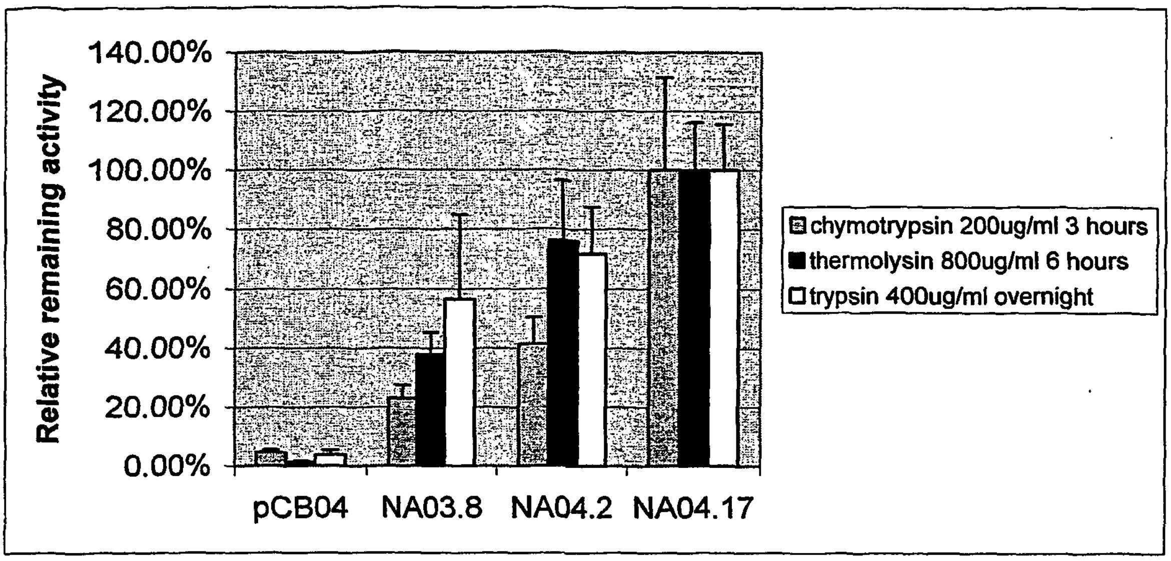

Relative remaining activity
140.00%
120.00%
100.00%
80.00%
60.00%
40.00%
20.00%
0.00%
pCB04
NA03.8
NA04.2
NA04.17
chymotrypsin 200ug/ml 3 hours
thermolysin 800ug/ml 6 hours
trypsin 400ug/ml overnight

FIGURE 6.

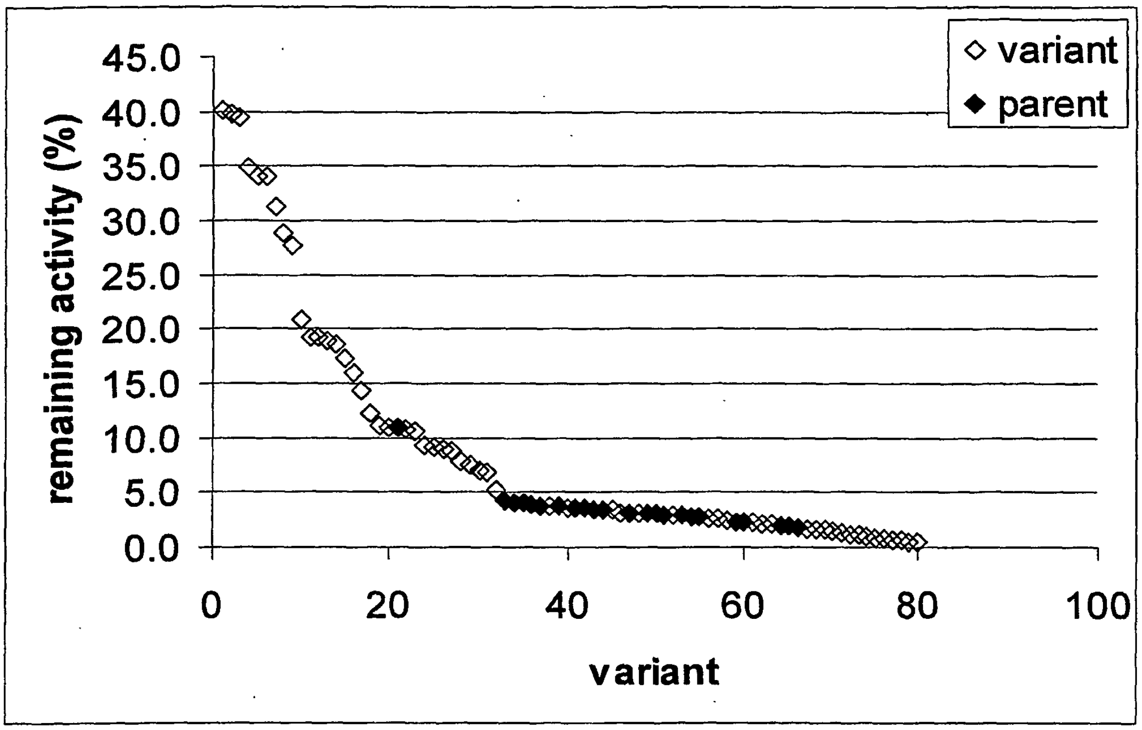
45.0
40.0
35.0
30.0
25.0
20.0
15.0
10.0
5.0
0.0
remaining activity (%)
0
20
40
60
80
100
variant
◇ variant
◆ parent

**FIGURE 7.**

heavy chain:
Qvklqqsgaelvrsgtsvklsctasgfnikdsymhwlrqgpeqglewigwidpengdteyapkfqgkatfttdtssntaylqlssltsedtavyycnegtptgpyyfdywgqgttvt
vss (SEQ ID NO:2)

linker:
Ggggsggggsggggs (SEQ ID NO:3)

light chain:
Envltqspaimsaspgekvtitcsasssvsymhwfqqkpgtspklwiystsnlasgvparfsgsgsgtsysltisrmeaedaatyycqqrssypltfgagtklelkraat (SEQ
ID NO:4)

BLA:
Lvsekqlaevvantitplmkaqsvpgmavaviyqgkphyytfgkadiaankpvtpqtlfelgsisktftgvlggdaiargeislddavtrywpqltgkqwqgirmldlatytagglpl
qvpdevtdnasllrfyqnwqpqwkpgttrlyanasiglfgalavkpsgmpyeqamttrvlkplkldhtwinvpkaeeahyawgyrdgkavrvspgmldaqaygvktnvqdm
anwvmanmapenvadaslkqgialaqsrywrigsmyqglgwemlnwpveantvvetsfgnvalaplpvaevnppappvkaswvhktgstggfgsyvafipekqigivml
antsypnparveaayhilealq (SEQ ID NO:5)

FIGURE 8.

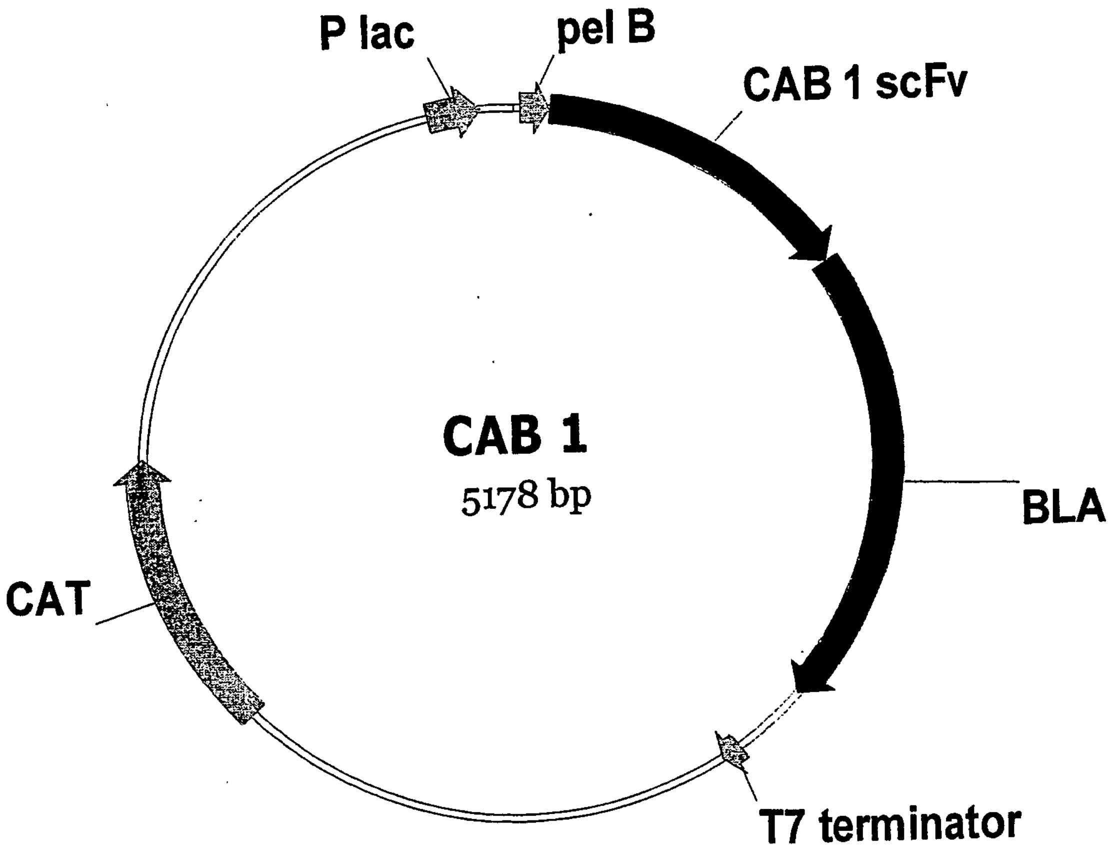

**FIGURE 9.**

aggaattatcatatgaaatacctgctgccgaccgctgctgctggtctgctgctcctcgctgcccagccggccatggcccaggtgaaactgcagcagtctggggcag
aacttgtgaggtcagggacctcagtcaagttgtcctgcacagcttctggcttcaacattaaagactcctatatgcactggttgaggcaggggcctgaacagggcctg
gagtggattggatggattgatcctgagaatggtgatactgaatatgccccgaagttccagggcaaggccacttttactacagacacatcctccaacacagcctacct
gcagctcagcagcctgacatctgaggacactgccgtctattattgtaatgaggggactccgactgggccgtactactttgactactggggccaagggaccacggtc
accgtctcctcaggtggaggcggttcaggcggaggtggctctggcggtggcggatcagaaaatgtgctcacccagtctccagcaatcatgtctgcatctccaggg
gagaaggtcaccataacctgcagtgccagctcaagtgtaagttacatgcactggttccagcagaagccaggcacttctcccaaactctggatttatagcacatccaa
cctggcttctggagtccctgctcgcttcagtggcagtggatctgggacctcttactctctcacaatcagccgaatggaggctgaagatgctgccacttattactgccag
caaagatctagttacccactcacgttcggtgctggcaccaagctggagctgaaacgggcggccacaccggtgtcagaaaaacagctggcggaggtggtcgcga
atacgattaccccgctgatgaaagcccagtctgttccaggcatggcggtggccgttatttatcagggaaaaccgcactattacacatttggcaaggccgatatcgcg
gcgaataaacccgttacgcctcagaccctgttcgagctgggttctataagtaaaaccttcaccggcgttttaggtggggatgccattgctcgcggtgaaatttcgctg
gacgatgcggtgaccagatactggccacagctgacgggcaagcagtggcagggtattcgtatgctggatctcgccacctacaccgctggcggcctgccgctaca
ggtaccggatgaggtcacggataacgcctccctgctgcgcttttatcaaaactggcagccgcagtggaagcctggcacaacgcgtctttacgccaacgccagcat
cggtctttttggtgcgctggcggtcaaaccttctggcatgccctatgagcaggccatgacgacgcgggtccttaagccgctcaagctggaccatacctggattaacg
tgccgaaagcggaagaggcgcattacgcctggggctatcgtgacggtaaagcggtgcgcgtttcgccgggtatgctggatgcacaagcctatggcgtgaaaacc
aacgtgcaggatatggcgaactgggtcatggcaaacatggcgccggagaacgttgctgatgcctcacttaagcagggcatcgcgctggcgcagtcgcgctactg
gcgtatcgggtcaatgtatcagggtctgggctgggagatgctcaactggcccgtggaggccaacacggtggtcgagacgagttttggtaatgtagcactggcgcc
gttgcccgtggcagaagtgaatccaccggctcccccggtcaaagcgtcctgggtccataaaacgggctctactggcgggtttggcagctacgtggcctttattcctg
aaaagcagatcggtattgtgatgctcgcgaatacaagctatccgaacccggcacgcgttgaggcggcataccatatcctcgaggcgctacagtaggaattcgagc
tccgtcgacaagcttgcggccgcactcgagatcaaacgggctagccagccagaactcgccccggaagaccccgaggatgtcgagcaccaccaccaccaccac
tgagatccggctgctaacaaagcccgaaaggaagctgagttggctgctgccaccgctgagcaataactagcataaccccttggggcctctaaacgggtcttgagg
ggttttttgctgaaaggaggaactatatccggattggcgaatgggacgcgccctgtagcggcgcattaagcgcggcgggtgtggtggttacgcgcagcgtgaccg
ctacacttgccagcgccctagcgcccgctcctttcgctttcttcccttcctttctcgccacgttcgccggctttccccgtcaagctctaaatcgggggctccctttagggt
tccgatttagtgctttacggcacctcgaccccaaaaaacttgattagggtgatggttcacgtagtgggccatcgccctgatagacggtttttcgccctttgacgttggag
tccacgttctttaatagtggactcttgttccaaactggaacaacactcaaccctatctcggtctattcttttgatttataagggattttgccgatttcggcctattggttaaaaa
atgagctgatttaacaaaaatttaacgcgaattttaacaaaatattaacgcttacaatttcctgatgcggtattttctccttacgcatctgtgcggtatttcacaccgcatatg
gtgcactctcagtacaatctgctctgatgccgcatagttaagccagccccgacacccgccaacacccgctgacgcgccctgacgggcttgtctgctcccggcatcc
gcttacagacaagctgtgaccgtctccgggagctgcatgtgtcagaggttttcaccgtcatcaccgaaacgcgcgagacgaaagggcctcgtgatacgcctatttt
ataggttaatgtcatgataataatggtttcttagacgtcaggtggcacttttcggggaaatgtgcgcggaacccctatttgtttatttttctaaatacattcaaatatgtatcc
gctcatgagacaataaccctgtggcagcatcacccgacgcactttgcgccgaataaatacctgtgacggaagatcacttcgcagaataaataaatcctggtgtccct
gttgataccgggaagccctgggccaacttttggcgaaaatgagacgttgatcggcacgtaagaggttccaactttcaccataatgaaataagatcactaccgggcgt
atttttgagttatcgagatttcaggagctaaggaagctaaaatggagaaaaaaatcactggatataccaccgttgatatatcccaatggcatcgtaaagaacattttg
aggcatttcagtcagttgctcaatgtacctataaccagaccgttcagctggatattacggcctttttaaagaccgtaaagaaaaataagcacaagttttatccggccttta
ttcacattcttgcccgcctgatgaatgctcatccggaattccgtatggcaatgaaagacggtgagctggtgatatgggatagtgttcacccttgttacaccgttttccatg
agcaaactgaaacgttttcatcgctctggagtgaataccacgacgatttccggcagtttctacacatatattcgcaagatgtggcgtgttacggtgaaaacctggccta
tttccctaaagggtttattgagaatatgtttttcgtctcagccaatccctgggtgagtttcaccagttttgatttaaacgtggccaatatggacaacttcttcgcccccgtttt
cacgatgggcaaatattatacgcaaggcgacaaggtgctgatgccgctggcgattcaggttcatcatgccgtctgtgatggcttccatgtcggcagaatgcttaatg
aattacaacagtactgcgatgagtggcagggcggggcgtaaagacagatcgctgagataggtgcctcactgattaagcattggtaactgtcagaccaagtttactc
atatatactttagattgatttaaaacttcatttttaatttaaaaggatctaggtgaagatcctttttgataatctcatgaccaaaatcccttaacgtgagttttcgttccactgag
cgtcagaccccgtagaaaagatcaaaggatcttcttgagatcctttttttctgcgcgtaatctgctgcttgcaaacaaaaaaaccaccgctaccagcggtggtttgtttg
ccggatcaagagctaccaactctttttccgaaggtaactggcttcagcagagcgcagataccaaatactgttcttctagtgtagccgtagttaggccaccacttcaag
aactctgtagcaccgcctacatacctcgctctgctaatcctgttaccagtggctgctgccagtggcgataagtcgtgtcttaccgggttggactcaagacgatagttac
cggataaggcgcagcggtcgggctgaacggggggttcgtgcacacagcccagcttggagcgaacgacctacaccgaactgagatacctacagcgtgagctat
gagaaagcgccacgcttcccgaagggagaaaggcggacaggtatccggtaagcggcagggtcggaacaggagagcgcacgagggagcttccagggggaa
acgcctggtatctttatagtcctgtcgggtttcgccacctctgacttgagcgtcgatttttgtgatgctcgtcaggggggcggagcctatggaaaaacgccagcaacg
cggcctttttacggttcctggccttttgctggccttttgctcacatgttctttcctgcgttatcccctgattctgtggataaccgtattaccgcctttgagtgagctgataccg
ctcgccgcagccgaacgaccgagcgcagcgagtcagtgagcgaggaagcggaagagcgcccaatacgcaaaccgcctctccccgcgcgttggccgattcatt
aatgcagctggcacgacaggtttcccgactggaaagcgggcagtgagcgcaacgcaattaatgtgagttagctcactcattaggcaccccaggctttacactttatg
cttccggctcgtatgttgtgtggaattgtgagcggataacaatttcacacaggaaacagctatgaccatgattacgccaagctatttaggtgacactatagaatactca
agctttctagattaagg (SEQ ID NO:6)

**FIGURE 10.**

```
                          1                                                                             80
Translation of CAB1   (1) QVKLQQSGAELVRSGTSVKLSCTASGFNIKDSYMHWLRQGPEQGLEWIGWIDPENGDTEYAPKFQGKATFTTDTSSNTAY (SEQ ID NO:7)
     human consensus  (1)   Q         KP G           TFS      V  A G                        RF  I R   K

                          81                                                                           160
Translation of CAB1  (81) LQLSSLTSEDTAVYYCNEGTPTGPYYFDYWGQGTTVTVSSGGGGSGGGGSGGGGSENVLTQSPAIMSASPGEKVTITCSA (SEQ ID NO:8)
     human consensus (81)                 AR                                     QS     P S V    QT

                          161                                                                          240
Translation of CAB1 (161) SSSVSYMHWFQQKPGTSPKLWIYSTSNLASGVPARFSGSGSGTSYSLTISRMEAEDAATYYCQQRSSYPLTFGAGTKLELKRAA (SEQ ID NO:9)
     human consensus(161)          Y           V            D     K    A      L    E D             G
```

FIGURE 11.

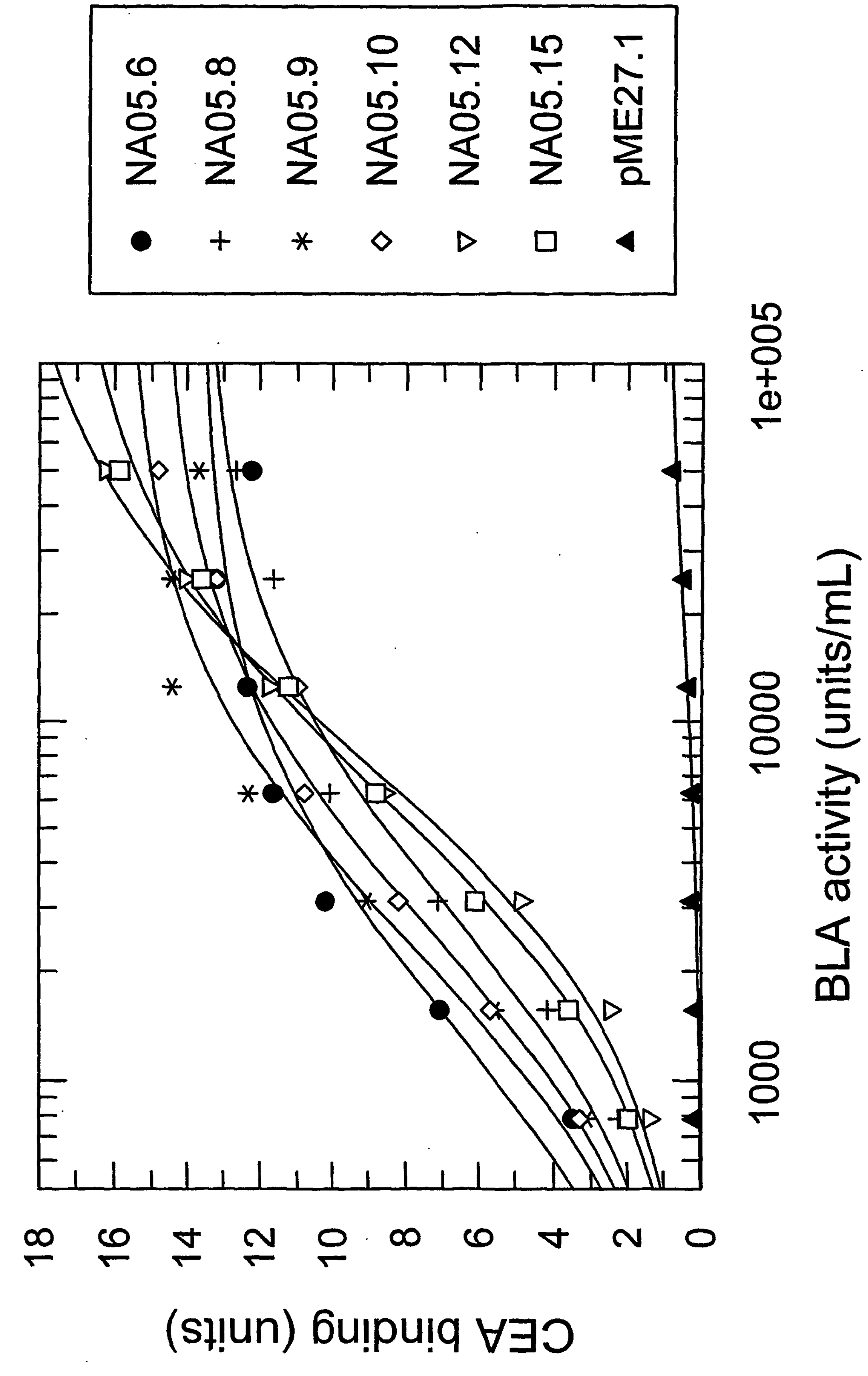
CEA binding (units)
0
2
4
6
8
10
12
14
16
18
1000
10000
1e+005
BLA activity (units/mL)
NA05.6
NA05.8
NA05.9
NA05.10
NA05.12
NA05.15
pME27.1

FIGURE 12.

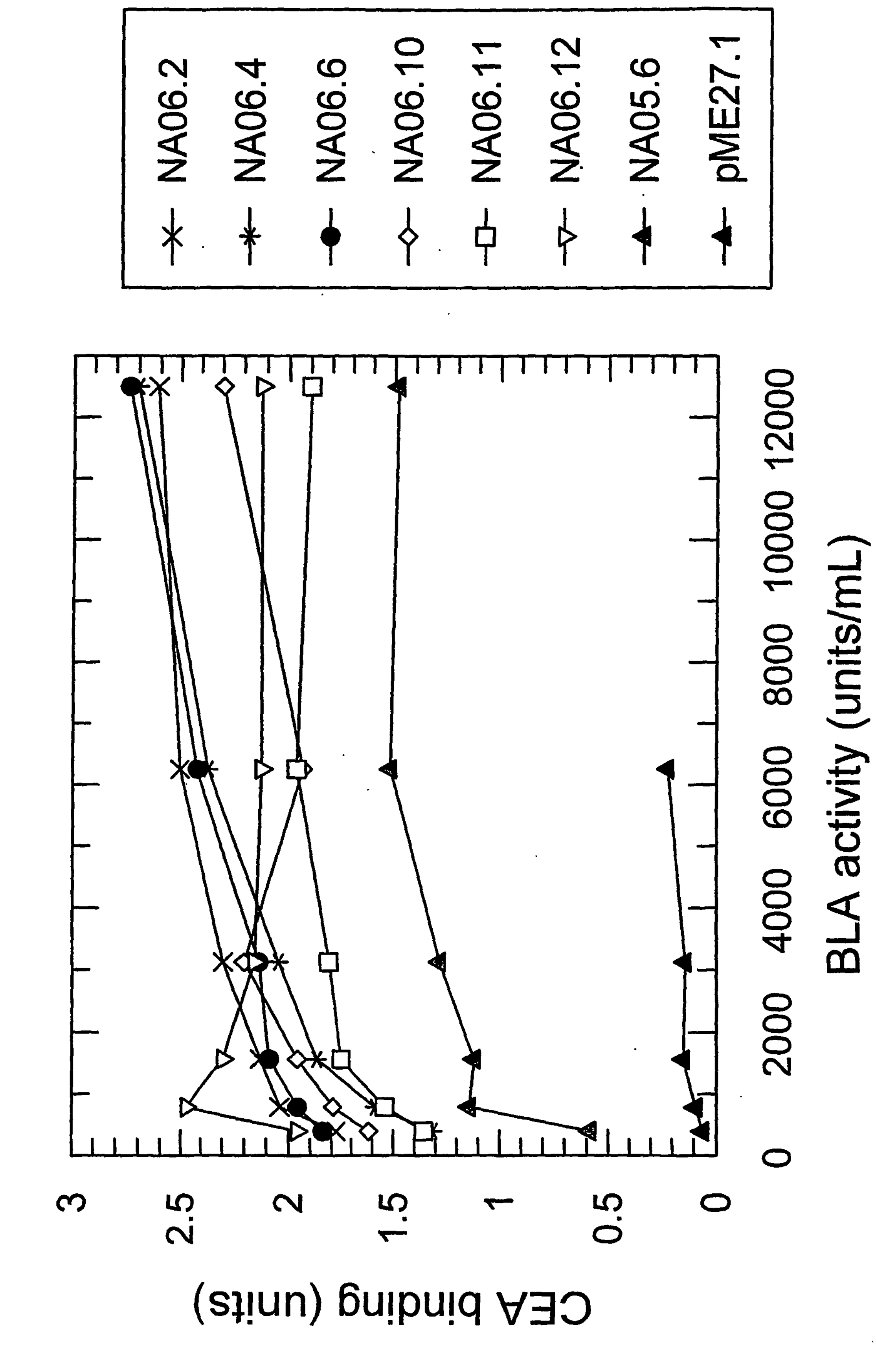
CEA binding (units)
0
0.5
1
1.5
2
2.5
3
BLA activity (units/mL)
0
2000
4000
6000
8000
10000
12000
NA06.2
NA06.4
NA06.6
NA06.10
NA06.11
NA06.12
NA05.6
pME27.1

**FIGURE 13.**

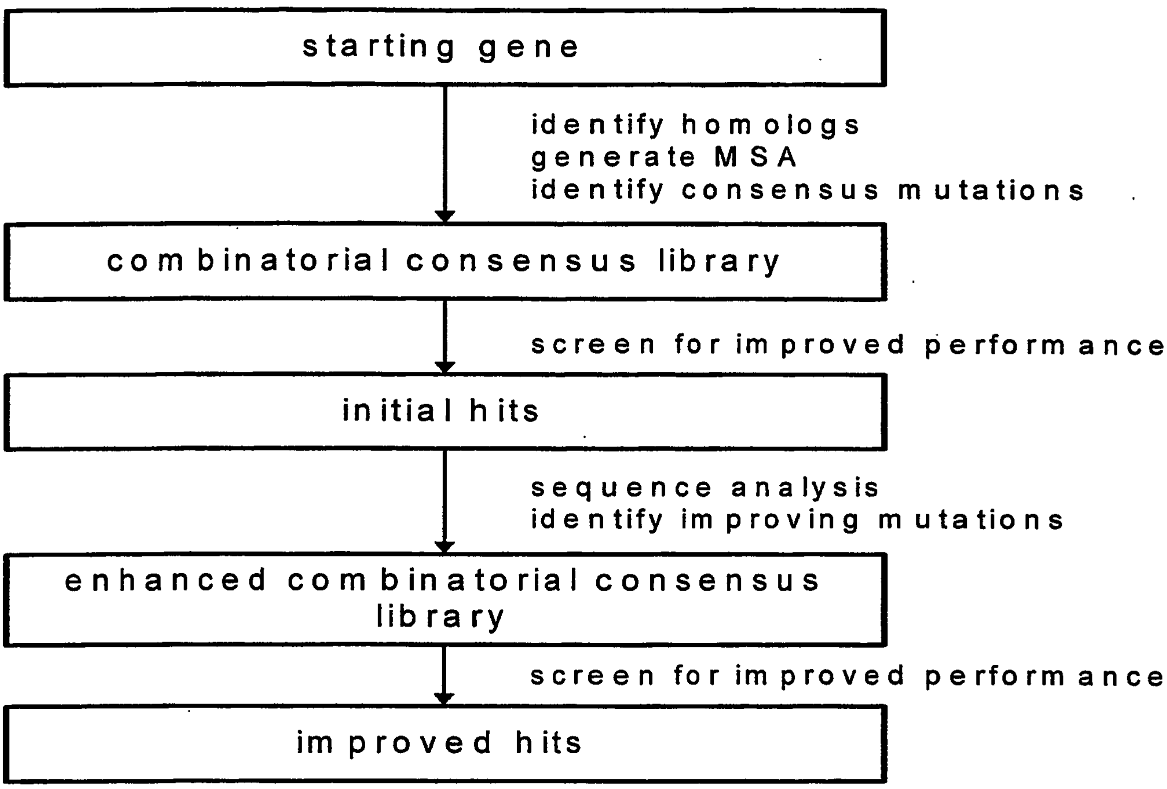
starting gene
identify homologs
generate MSA
identify consensus mutations
combinatorial consensus library
screen for improved performance
initial hits
sequence analysis
identify improving mutations
enhanced combinatorial consensus library
screen for improved performance
improved hits

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5605793 A
- US 5965408 A
- WO 9842728 A
- US 6582914 B
- EP 04788755 A
- US 2004030085 W
- US 10688255 B


### Non-patent literature cited in the description


- **Meyerhans ; Wain-Hobson.** *Nucleic Acids Res.,* 1990, vol. 18, 1687-1891
- **Stemmer et al.** *Biotechn.,* 1995, vol. 18, 194-196
- **Osuna et al.** *Gene,* 1991, vol. 106, 7-12
- **Lehmann et al.** *Protein Engineering,* 2002, vol. 15, 403-411
- **Tu et al.** *Biotechn.,* 1996, vol. 20, 352-353
- **Merino et al.** *Biotechn.,* 1992, vol. 12, 508-509
- **Ostermeier.** *Trends Biotechnol.,* 2003, vol. 21, 244-7
- **Singleton et al.** DICTIONARY OF MICROBIOLOGY AND MOLECULAR BIOLOGY. John Wiley and Sons, 1994
- **Hale ; Marham.** THE HARPER COLLINS DICTIONARY OF BIOLOGY. Harper Perennial, 1991
- **Steipe et al.** *J. Mol. Biol.,* 1994, vol. 240, 188-92
- **Lehmann et al.** *Protein Eng.,* 2000, vol. 13, 49-57
- **Lehmann et al.** *Protein Eng.,* 2002, vol. 15, 403-11
- **Free ; Wilson.** *J. Med. Chem.,* 1964, vol. 7, 395-399
- **Pozsgay et al.** *Eur. J. Biochem.,* 1981, vol. 115, 491-495
- **Arnold ; Ulbrich-Hofmann.** *Biochem.,* 1997, vol. 36, 2166-2172
- **Boehm et al.** *Biochem. J.,* 2000, vol. 346, 519-28
- Sequenzdatenanalyse. **Steipe.** Bioanalytik. Spektrum Akademischer Verlag, 1998, 233-241